# EUROPEAN PATENT APPLICATION

(11) **EP 1 426 442 A1**
(43) Date of publication of application: **09.06.2004**
(21) Application number: 02026772.0
(22) Date of filing: 02.12.2002
(51) Int. Cl.: C12N 15/12, C07K 14/435, C12N 15/11, C12N 15/63, C07K 16/18, A01K 67/00, G01N 33/50, A61K 31/7088, A61K 39/395, A61K 38/17

(54) **Marker molecules associated with colorectal lesions**

(71) Applicant: MTM Laboratories AG, 69120 Heidelberg (DE)
(72) Inventor: Hipfel, Rainer, 69126 Heidelberg (DE)

(57) **Abstract**

**Summary of the invention:**

The present invention relates to colorectal lesion associated (CLA) nucleic acids and polypeptides useful for the detection and treatment of disorders characterized by abnormal cell proliferation. The invention is more specifically related to a nucleic acid and the polypeptides transcribed thereof, the expression of which is significantly altered in association with adenomas and adenocarcinomas of the colon and other cancers. Another aspect of the nucleic acids disclosed herein are transcripts of the gene disclosed herein, that are differentially produced in various tumours, such as e.g. those of the gastrointestinal tract, the respiratory tract or the anogenital tract. Furthermore the present invention provides methods for early diagnosis, prognosis and monitoring of the disease course and for therapy and vaccination of tumours associated with the expression of the inventive compounds.

SEQUENCE LISTING

<110>  MTM Laboratories AG

<120>  Marker molecules associated with colorectal lesions

<130>  colma2/P/EP

<160>  161

<170>  PatentIn version 3.1

<210>  1
<211>  35586
<212>  DNA
<213>  Homo sapiens

<220>
<221>  gene
<222>  (1)..(35586)
<223>  genomic Sequence of CLA2


<220>
<221>  promoter
<222>  (1)..(19867)
<223>


<220>
<221>  TATA_signal
<222>  (19489)..(19498)
<223>


<220>
<221>  misc_feature
<222>  (19868)..(20103)
<223>  Exon 1


<220>
<221>  Intron
<222>  (20104)..(23118)
<223>


<220>
<221>  misc_feature
<222>  (23119)..(23279)
<223>  Exon 2


<220>
<221>  Intron
<222>  (23280)..(23670)
<223>

<220>
<221>  misc_feature
<222>  (23071)..(23742)
<223>  Exon 3

<220>
<221>  Intron
<222>  (23743)..(25222)
<223>

<220>
<221>  misc_feature
<222>  (25223)..(25385)
<223>  Exon 4

<220>
<221>  Intron
<222>  (25386)..(32509)
<223>

<220>
<221>  misc_feature
<222>  (32510)..(33412)
<223>  Exon 5

<220>
<221>  misc_feature
<222>  (33413)..(35586)
<223>  Extended 3'UTR of CLA2 transcript

<400>  1
acacattatg gtaatccact gtgtcccagg tacctaacaa ggtgatggta ataaagtgat      60

gaaataaaaa aaaacacaca cacacaaaca cttactgtct acccctagaa gcttacattt     120

tcctggggga ggtatcataa agcaaataaa tagaaaagaa caaagaatta ccatattgaa     180

ggtagtaaca taagatcaat gaggccataa ggttgtgggg cagcatcagt gctctgaatt     240

taagttcggt aagtaagctg ggtttcagat gtttttgttt tttaaatttg cactgatatt     300

attttataaa atatttgaaa ggcatattag aagaaacaat gttttcaata aaaattcact     360

ttcataccaa aacataattt ctgaaaatgt ttataatata tagtaagctt tttaaaatat     420

atattatcat cttctttgaa ttcaatagat tcaagtataa gcagaatata atttttatac     480

aataattcaa taggtaccac aattagaaac tacttgtgaa attagataca ctaatctact     540

gagaggaatt aatttatcag atcatagagg aattcacttt ttgcgtataa agaaacaaaa 600
gactgtttca ttcttacctg agtagagcca tcagaatgta ttatagtcaa attattaatc 660
tcttacattt ataaatattt atatgttgaa taaacatttt gacatatgaa ttggcaaata 720
aataatcaga aaaaaattgt ctttggaaaa gataaaactt ttttttctcc aacagggaaa 780
atatgtatcc taaaaagttt tgcagcaaat ctgtccattt tcattttttc acctcatttt 840
catcttatca agtcacaatt gtaaaacaca aatttcagta ttcatctaaa aagacttggc 900
taatatgtca gacctaaata tctaagagtt ctgatttatt ttccttgtgc cggccagctt 960
tctcttttga aagcatttgt gggaattatt taaaaagaca aaatttcata tttgtgtcat 1020
ttcaaatatc catcagtata aaatatttaa ctagcattct gaaaaatatt aaaatgatgt 1080
aattattatt gttttatttg atgaagcaga aaaatgaaga agattaagga aacattctat 1140
aaattaattg aaactatcat aaaggagaaa catgtatgct taatcaaaca ttttaagtag 1200
tttggacgca ggtaacagag atgtttaagc aaaagctgaa gaaaaatatt ccataaaaat 1260
tatagaacat tcaatatatc tctattacca ataaaatatt ttaattttta aaatatataa 1320
ttattcaaat aacacgatat ttggacattc actatttatt aaattccaaa ctaccatttt 1380
aaaaagcata ctctgagact gaactattta tttctttgga aaatatgaaa atgtgaaaag 1440
caagaacgtt ccaaaatgag ttccaaagta ttttaaactg gtttagacaa aaaaaattta 1500
aaaagcaata ttgtgatgta aatatagaaa cacagttgac acacaagtga aatcagattt 1560
cacattgttt cattctcaat aacagcactg ccactcacaa ttttcagcac agaattattc 1620
tctgccaaaa cgtttatttg cttttatgga cagcctaaat gtgatcatgt tcacattacc 1680
ccaaaacaac ttctgctaag agagatagct gaaatgtttt cctggtcaga gatggaattt 1740
ttagaccact agtaataaca gatatcagag aagcaaacct ctaacttcat gttttgtagg 1800
taagattaat tttgaaaaat aggctgcttt aacttgtgtt attcttatat cagacaagtc 1860
agtgtatagc tcaatagaat atggcaaaga tatttgcatt catttaaaaa cagttatttt 1920
gtatcttcaa atagcttgta caaaaccaaa agtgcttcaa cacatctaaa tgaggacata 1980
tagaaaaatt tagcaatcac tgagagcaat tgtttctttt tatttgtgtg tgtgtacaaa 2040
tggaagaaat gaggtatttt tgcatgggaa tctattttct attaaaattt actagattta 2100
ttatatcact gttcctgcag tagaatcata tcattttatc tgaatcagct catatgccat 2160
tgtacaaccg tatatgataa tcaatttgat ttttggtgtt ggggggacaa gaacatagca 2220
attcaatgca gattgcacat gataaattta gcaatctgac aatttgtgca ccaggcacag 2280

ggcctgacaa atagtttggc caattttcta ggctatgtcc aatttgcaat tggtatacag 2340
acatgatata aagtttattg actgcatcct tctcttacgg ctttgcatgt aagcagagtg 2400
agatgctgtg cctatggaga gacgtgagaa ggtttgctgt gtcatttcta atgagaacat 2460
aattgtagca atggttcatc gtcatgacaa caacctgatg cttccatcca agaatcaata 2520
ctaattcctt gagtctattt cttttctact ggagttttac agtagtgtag ggaaaggtca 2580
tatggcacca agtgatacaa tgttatttct attgcaacta ctgaaagttc ttgtctaata 2640
tttctccttt ttacacgact aatagctaat gtgacaaata tgatagaaac actagaggat 2700
tgtgataaaa tccagatatt atcaatatga acctatcagt gccatgagaa acattaggca 2760
actagctata gaattatact ctcggactca agctaggatg acgtagactc cttccaaggt 2820
ttattttctc tgaagctcat tagatatcag tttgtcattt tttgcgtgcc tctgaaaaag 2880
aggtgcttac tagcaaataa cattgttatg tcttttgaca aaattaaaag agatttgtat 2940
tgtgtgacgg atttttccaa aattgtgaaa taatcaacta tgaattgctt attttatctc 3000
tagttatgaa cataatttta aatatcataa aaatgatcta gactaatgta ggcacatatt 3060
ttcctttcct gaacacttac tctctctaga gtaaaagtct acagaaataa tataattttt 3120
ttcataccat catacataga ttgtagttga aaacatcagt tcatatatga tattacaaaa 3180
aatatgaaaa ataaaatctt gacatcacag gtgataagac cagagacaac aggcattaga 3240
aattttagtg ttgtttttaa ttacatagaa atgttaaaac tgatacaaag ataacttcaa 3300
tgtaaaatca atcaagtttt ttcaacctaa tgcattgatg tctgaatata acaagaaaaa 3360
ttaatccttt taaactctaa tctctacaat aaaactgtaa aatagctttt acttttgtga 3420
ttgtttatat cctaaaaaga catttgtttg tttcagattt atttctttgg gaaggaaacg 3480
gtaagagaga gtaacgaatt aaaaaagtat attggaaagc aacactgaag gaaataggca 3540
agacacagga atgtccccta atgcagactt gacggtttct gccagttccc taaaaattct 3600
gaagtgaagt ctttcctttg gtaaaatgat tagacccttg aacatgtgac ttgtgcagtc 3660
attgtctgag ggccactcaa gaatgctgaa caatcgttaa cactattaac agttacagtg 3720
tcattggtca aggtctgtcc ctagaaaata tatcaactag ccaaacttct tacagctatc 3780
cagcaagtat gttttggaaa gaagttctgt ctctatatgg gttattttat ttcattatat 3840
tttattttgg gtcacaacca attccaagcc ctaatcacat tttcttgttt ctctgtttca 3900
ttttacttga tttcatttga aggaaaaact ggacacttgc tcttaatgtc actttgtctt 3960

ttcaagcaaa gcctaccttg ttaaatcatc caacaaaaat atcttccatg atgaagaaaa 4020
ttaaaagcta cttcacaaag gttgtaccgt tgaacattaa ataatgtgta aattatgatt 4080
gttttattct atttctttct gcatgcattt ttaataattg atatattatt tagagaaaga 4140
cagatataga aactctacat ttaaactcaa aatttagaag tttaatattt tggaaaattt 4200
acctgaacaa gtaatacttt atcagaaaac ccactagttc taggtattta gagatggaat 4260
ttaagataca aaactacaca agatttatgg ctttttatta gcttttttga ttaaaatctg 4320
aatcctgatt ttttagttca actaagggcc ttctcaaaaa acaatcaaag aaaagaggta 4380
cgtcaatgta gacaatgatt atggtaatct cagaaagaag agggatacta ttacagcatt 4440
tttaatgtct tctcatactg aaaataacca aaagaagaca ggacatttaa gttttgtcaa 4500
ataatatgtt gaaagtaatc gacaacttaa aaaaaactct ttacttgtgt tattccgcgt 4560
tgaaatagat aactttaaaa atatttaatt catttaaaca gattatttat agcatttata 4620
tttgtttcta agttagagaa tattttctaa tatctatgac tcccaaagtc cattttggtt 4680
ctatgtatcc aagtaaaaca gtatttcctc aactaataga taatttccag catgacagaa 4740
atttatattt taccatgcta aatccttagt gctttgaata gcatccatgt tataatagac 4800
atttaataaa catttaagtt tattaataca tttatgaatt tagctttata ttttaacaca 4860
atgctatttt tcttagaatg attctaggga ctggctaaca actcagaata aataaaaaat 4920
agtatggaaa gctatataag ttggattaaa atatttagtc tttatcactt aataaatgaa 4980
cagtgagact atgctaagtt ataacaaagt attcccaagt gtcagtgcct taacctagta 5040
aaggtttatt tcttgcttgt tatattctaa tacaggacaa ataattcctc tccaattaac 5100
gatatcaggt ccatgctcct tattgtgtta ccattattgt caccacttgg actccaagtt 5160
acctgtcaca tgaatgacaa tagagaatgt atgtgaatgc tatttagtga ccaagtctgc 5220
aaaagtcaag tatccatctg cccatatccc tttggctaaa attcattgat gttgttctat 5280
ctaactgcaa gaaagatgga aaatctaatt aaagtgtgtg ttgaaaaaaa ggagaaaaaa 5340
aaactttttg aagattagca gtcattgcca cagagatatg tttctcctgg atattagaga 5400
gttgttactg tttaaattat gaattaatgt actttgttta ttatcttttc aatatcactg 5460
gcaaacttaa ctagaaaaat caaatactaa cccagaagtt tgaattactt ttttattttg 5520
ttgtgtttct ttttcttaac aagagtcatt atcctaacag acaaaaattg taatagccac 5580
atccatggaa ttcagtagaa tacaattaca tttctttatt ttgaaggatt taagaaacag 5640
tggttgcctc tggaagacag gaattgctag aggggaacat aaagggaact ttctagggtg 5700

atgaaaatgc taaaaataaa aaggcaaaag gaaaaaagaa agaaatcata cccttaaatg 5760
tttacaaaag tgaataaaat aattggttaa aaacaacaaa accttcattt ctgactacta 5820
taaattagat ttagaagaca aatatttccg ctgaggacaa ctatgaaagc tgaatacagt 5880
tttaaaaata aacaaatttt ttttaaatta ggcattgaag agacaccata gcaaccaaag 5940
cttgagacac taagaaccca gggtaaaaaa aaaattgaga ggagagaagg ctatatatca 6000
cacttaaagc agcagctgag acactacagt cctaagcaga aatactggca gactcactat 6060
gctgatgaca tgaaatttga aagttgggtg aaggcaaaga ggagatgccc tgataaacac 6120
tgggtttcag ttgagtcttc taaaatggta tatcctgaga gtgaagcaaa gaggaaatag 6180
atcagtcctc acaaagttga aactcagact cgaaatatca caaattctaa ctggatatag 6240
attacttgca cctaatcaac tacttctcct ctatagtttt ttctttctta atattttagt 6300
ccatttttgt gttgctctaa agaatacctg aagatgggta atttacaaag caaagaggct 6360
tatttggctc acagttcagc aggctgtacc agaagcatgg cacaggcatc tactcagctt 6420
ttgatgatgg cctcctgctg cttctactca tggcagaagg tgatggggag ccagcttgta 6480
aacatgacac ggcaagagcg aggaatagag gaggaggtgt caagctcttt ccaacaaaca 6540
gttctcctag gaactaaaaa tgagaactca ctctcaaaaa tgccactaag atattcatga 6600
agcatctccc cccagactca aacgtttcct tgaaggaccc acctccaaca ctgggaataa 6660
aatatcaaca tgagacttga cggtaccaaa caaaccatct tcaaaccaca gcatttaaca 6720
taaagtccag tttaaaaata aaataaaatg tcagtaaggt gggacacaaa gacaaaacaa 6780
acacaaaaga cacctacaaa attaattgac aacataaaca gagtataaat gaatcccttt 6840
tttggagttg aaggcagaca agctacaata gctcattaat aggtaaaaga aaatagaaga 6900
taaaaaattt tgccaaaaaa attgaatata taaaaaagta tcaaaattct agaaaagggg 6960
caacattgaa tcttaaaaca ctaaaacagt tttaatagct gaatagttac ctaaaagttg 7020
cattgattat gtggaaatta aatatccata ggaattagtc aaatagaagt agagagagaa 7080
aagacacatg aactgcaagt ggattacatg tgaactacac atgaagcaca tgtgaacaac 7140
ataagaaata tgaaacgtgg tgtattgttc tcatatatgt atacctggaa tcacagaagg 7200
aaagcagaga gaggagagag gtgaaacaat atttgatcag agttataata atacaaaact 7260
caagtgggaa aacacacata acgccacacc tttttacata agctatcaca caagaccaga 7320
ttgtgaaata atatgtgaat tagagatttt acatataatt gagtataagc attggtcaga 7380

tacctccttc ccatgttgga tgtgaacttt ctactcagaa taatacagga tgtctgttag 7440
tgcagcttgt gggatcacct catctaatgc agaggacatt tatttatgta ataaagcatc 7500
tatattttaa tcaccagaat tttttttagc ttatgtgata cgacgaagga aaccatgtct 7560
catagatcct ggatttaaat gattcatttt atatccttga ggctctctta aggtcattca 7620
ttgtctattt atctaaaaat acagttgatt gtgtgtgtgt gtgtgtgtgt gtgtgtgtgt 7680
gtgtgtgtgt atgtatgtat gtatgtaccc tgattgttat tttagggttc tgctttatta 7740
ttctttttta ctcaaatgtc tgttttgtta ataagtttta aaattatttt aagtacacat 7800
tttgaaaaat tatgctctga tattgataat gttaaaacca ataaaatatt gaaagatgat 7860
atactttatt atttcttata tggaagcaat atataaataa atacagaggt tgattgacaa 7920
attgatcaat agacaaataa atgaccaagt tacaactttt taattaaatg taacactgag 7980
gagttgaaat gttttcatga tttcaaagaa accgtaactt tttttaaaat aatgcaaaat 8040
ttgtcctttt ttggtcagtg tttactagta aattatgggt tagttcatta cataaagttt 8100
aattatttta cttctgaaat aaactttatt ttataaaact atacaattga attttttact 8160
gaaaattaca tatattttgc ctgggatatt gatctgtaat tttcacttgt gtgtgtgtgc 8220
ctgtgtgctc tgtgtatgta ccaaccttta gttcagttga cactactctc tctttaaaac 8280
aaattcctct gtaacttcca tttattggag gtttaagata attcacttgc taaactctgg 8340
tttttgaatt tttaaaaatt tgactatgat gatattattt tttcaataca ctattttact 8400
catattatct accctttttt ccataagtta atttgaaaat ttccaccaca agagaacaat 8460
aagattttaa gatgtattgg catataatag ggcataatga tcttttacaa tgccttaatt 8520
tttgaaagct tttttttgga cagtaagatt tttgaaatat gcatataact aaaaattgtg 8580
cttataaaca caattcagaa aaaatctcct ttcaggtttt ctttctgacc tataatatga 8640
attatttgct gaaacaaata tatgccattc ttaaaattta gaaacaggta aaatataaaa 8700
atgatatata atgatatgta tacatacatc attgtgtgtg tgtgtgtgtg tgtgtgtgtg 8760
tgtgtgtgtg tgtgtaaaat acctggccag gtagcacaat atctcaagaa aaaagaattt 8820
aattaccaag tggaagaaaa aaattgtcat ttatagattg atgatatagt tttcccccaa 8880
accccaaaac agattagtac ttgaaatgtc ataatgttgc tagagatata taaaaatcat 8940
gttttatatt tactaaacaa ccaattagaa attgcaattt tattcataag ttttaataga 9000
gtaataaaaa gtgtaaggaa catataaata tatctaatac ttattataaa agtcatttgt 9060
taaaaactat aaaatttatt aaggataaat aacaattata tgagagataa agtgtttatg 9120

taaattttaa cataccataa cagtttcagt acacctaatt atatctatgt attcaataca 9180
attttaataa atgcctttaa agtattttta ctcttcaaac ttacaagctg attgaaacct 9240
ttatacagaa tattaaagga gctgccatta tcaagaaata ttgacagaat acggtagatg 9300
aggacttaat ctataagatt tatgatttag ctttacgata aagctagaat aattaaaata 9360
atgtgatatt ggcacagtga tagacctgcc aattaatcat aactgagagt ctatgggatc 9420
acacatttac aaaaactttg taatagcaag taaccgaaat agcagaacaa atcaatgagg 9480
acaggaaggc tttgtaaaag taagttctgt cataactatc tgcatattgt agattcaaca 9540
cttctcagag caggggattc catggaaagc atttttggtg tgtccaaaat ttggcatgac 9600
acatctaata aggcttatca ttactttgta caacatgctt ttgtacatct aactctgagt 9660
ttatctgttt ataatcagag cttttcactg actgatagca aggcaatgct tgtgcccgaa 9720
gcatatcatc cttttttgta atgtgtcctc aagctttata tacaatgttt caacattatt 9780
tgagtaatat atttctttca gggagagatc agagagaaaa gtgtagctgt ttgaagtgtg 9840
atacttgact ttctctcatt caactttata agcctttttg tactgtggct tgctattaca 9900
ctttgtgaaa tatttatata tttcaaagct tgtcaacaga gcagcaagag ttggctttca 9960
gagagctaac acgatatagt agaattatta ttatataatt ataattattt tgaagttaac 10020
acaaatctcg ttttaaattt tagctctcct tattttatga tttaggcatg ccacttgatg 10080
tttttcagtc tcagcttctt gattagtaaa atgcaataat aatattttat cagagggaac 10140
tatttcaaag agacaaattt aaaataaatg gcaaacgtag accttcacaa taaattacct 10200
tctttaaatt cttcatgata ctagcaagaa aagaacactg tttggtataa aggtaaatgc 10260
tgtcattttg gtttatctat ccattcattc tctaacataa tgtttgactt tttcaatcaa 10320
tagctaatga taaccaaaga acaaccaacc ataattaaca accagttaca ttatctatac 10380
caatattttt taagttaata ttctcatttt tcttattaat cttccaaaat aactagtata 10440
gactctcact actttcagag gatttgatac agagaagtga agccagctgg gattctgggt 10500
caggtgggga cttggaaaac ttttctgtct agctagagga ttgtaaatgc accaatcagc 10560
attctgaaaa aatggaccaa tcaacactct gtaaaatggg ccaatcagca ctctgtaaaa 10620
tggaccaatc agcaggacat gggtggggtc aaataaggga ataaaagctg gcaaccctag 10680
ccagcagcag caacgtggga gaggttggtt ctttcgatct tgacaataaa tcttgcagct 10740
gctcactctg tgggtacatg ccacctttaa gagctgtaac acttgcctgc aaaggtctgc 10800

ggcttcattc ttgaaaaaag cgagaccgag aagccaccag aaggaagcaa ctccggacac 10860
agtacttcaa aatattacag aaatttgttt tgaaatacgt gacaaatagt attgataata 10920
catgaagcag ttcgtagata ctatcagtgc cataagacaa ttttatacat ttaatttctc 10980
aaaagataga gctgtaagga gggtccctga gtagaaaaaa aaaaaaaggt tttcctttgt 11040
gctcctatca taataattaa cacagaagac ttctgtgacc aaacgtgggt ttttttccca 11100
ccacatatca agcaagcagt taattctgca gaggaaacca gttggatgtc ctctacttca 11160
attcaatttg acactaactg gagataacat gagatcatac aggctgaggg ctcagttcca 11220
caagactgaa tgccacctac agtaacaatt gcatgcccag gttatttttc ttgtgcttct 11280
ggctgactgg ttgcattggg gttcctacaa cccttttttt atttaatttg taagagcagt 11340
tcacagaact caggcaaaca tgtactttat ttactaccta ttacaaaggc tgttttaaaa 11400
gatgcagttg agatgcgttt tgcatctggg attcaggatg gagggaagga gcacagagcc 11460
tccttgcact cttcggcgtg caccatcctc caggcacctg cagaagttca gctgtccaga 11520
agctctccat accccaccct ctgagcctct atggagactt cactgatagg cacgattgaa 11580
gtacggtaat actagcagac tcagtgagga aacccagaaa ggcctgtctg ctcggattct 11640
ccttggcttc tctgctcagc attccttcct ccagaaagga ctagatgcct tatgtaacag 11700
gggtctcatg acccacaatc agacacggta ggttagagaa tttctttata actggctcca 11760
agacagaaag gcagttgaag attaggggat attttcagtt tctatgggct gctttgagga 11820
gaaaaaggaa caggtgaaag aagggcagga gaaggtcaga gagagatgct gttttatgag 11880
gcccgcttcc gaggcttaca gcacattata agaaaagatt gccttttact tttatcgttc 11940
tgaagctatt caggaaccaa ggaaaaaagg ccaaatactt tgacaaaata tatgcttatt 12000
gttttactca cttaggaaat aataaggact atgggagtta tgagccaaga actgtggatg 12060
aaaagatatg tgtatatatg taaaataata tactctccgt tagatttgat ttgacatcat 12120
aggacagaaa gagttgacaa aacatcatat atttaaagac ataattacta gaatctacaa 12180
atactgtcaa ttaatcttga gtttcacatt gttactggat acttgtttta gaggaaacta 12240
ttcatgactt tttcctctct gtctcatttt attattataa agtcattgat gtactgcaat 12300
gtgatttaaa gtatatgtgc caaagttttt ttctgttcca aaaaaagaaa ctttttttga 12360
atattgtatt attgctaggc ttaaaatctt tttctcaaag aagttaaatg tgtaaaattc 12420
agatacaaat taagttcatt aaaatagtca tttatcaatt actatgcaac tatgtctcag 12480
aattcaacaa gaagtttaag agactataaa aataaaatta aaatttcaca tcatttgata 12540

aagtgaaaat attcaattgt tggaaaagta attgtggttt cagactgtgc attttatatc 12600
attataacta ggctgaaaca catctttatt aatcaaaata ggaaccatta caatcaaaac 12660
atttctgcca acaagaagta agtttgttta ttcctgtagc gtaaaaatct gtgcttcata 12720
attgaatgaa ctcttggaaa gcattttcta cattctgctg gttgtggaag catttgccct 12780
gcaaaacatt gtcaatatgc ttgaaaaagt ggtagtcggt ttgcaagacg tcaggtgaat 12840
attgtggatg gggcaatttg ttgagcctag tagcccaatt tcttgagctt ttgaagtgtt 12900
ggttttgcaa tctgtggtca agcattgtgg tgaagaattg ggccctttct gttggccaat 12960
tcggctgcag gtggtgcggt tttcagctca tctcatcaat ttcctgagca tacttctcag 13020
atgtaatggt tcaatcaaaa agctgtagtg gatcagacga gcagcagacc accaaacagc 13080
aaccattatc tttttttggt ggaagtttgg cttttggtag tgctttggag cttcttctca 13140
cactgagctc gtcttcagta gttgctgtaa aaaatccact tttgtcccac atcacaatcc 13200
aatagagaaa tggttcattg tttttgggta gaataagaga agatgacact tcaaattgaa 13260
gatttttttg attttcgctc agctcatgag gcacccactt actgagcttt ttcacctttc 13320
acaattgctt caaatgccaa acgactgtag aatggttgac gttgagctct tcggcaactt 13380
ctcgtatagt tgtaggagga ttgggtttga taattgctct caatttgtcg ttttcaactt 13440
ctgatggcca gccactagct cttcatcttc aaggctctca tctcatttgc aaaagttctt 13500
gaatcaccac tgactgtatg tttgtaagca gttcctgggc caaatgcgtt gttgttgttg 13560
cgagttttct ccactggttt gtgactcagt ttaaacgcga ataagaaagt cgcttgaatt 13620
tgctttttgt ctgacatcaa ttccatagtc taaaataaac ataaaataaa cagcaagtaa 13680
taagtcatta gcaaaaaaag aaatgaagtg agaaattccc cttaaaatga tgtataacat 13740
aactacattt atttaagaac gtattccaat atcaaatgac aaatttcaac aatgcaaaaa 13800
ccacaattac ttttgtacca acctgaaaat agaaagagct ttcctaaata aacaactaat 13860
tttaataaat aatatatgca gttaagtgaa aaaggctggg cattcacaga aaaaaaacaa 13920
acaaacaaga gatactattt ggttattaca cataattcta aaaaaagaac aaatctagac 13980
attttaaaac ttatggaaaa aagaattaat aaagacacaa accataaata ttttgattct 14040
gcaaatgtgt gagcactgca caatttcaca ttgacttaga aataagaata ctcatagata 14100
tgtaaaagtc aagagccaaa agagtagtat atataaatac aaagtctcct aaaaattata 14160
gttcttacat aatttgcaac ttacataatg tgttcattat ttgttctttc ttcactcgcc 14220

atatattttt aacataaaat tatgtaacta tataaataga gggtgttttg acccctatgt 14280
ctacattata ccccaatggg tcaatctatc aatctactga ataacaatag acctccaatt 14340
tttgttaata tctaactacc agaaatatca atcccttgaa attgaagtgg ttgcagagct 14400
tactctgtga gttgagattg agtattttca ttttttcttc ctgtcctctt taatttattt 14460
tcataagcct cctgatttca gttttaccaa tttttttcct tactttctca ttgcctatag 14520
ataaacaaaa atgttttatt ttttctgggg ataattttat tatagccaaa ttttttttca 14580
gcagtttcct tgccttagtt ccaaacctaa tgcctctgtc atttttctct acactcttcc 14640
ttttatatta ttgtccatga gattttgttt atttactctc accccttgat acctttctat 14700
ttgttatttt tattctttaa aaatgctgac tttactaaaa tggaaagagg ttaaactgtg 14760
taaataataa tgtaagagtc aagtctcgtc atattcacaa tttggcaatt aggcttgagg 14820
ttcctcaaac aatttcttat gttaggaaga aattgtttat agataccagt gtttttcaaa 14880
ctatgcaatc cttgacaatc aaattgagtg tccttttcat taagatttaa caagagtaat 14940
gtttttcctt gcaagctaaa gtaaataatt tcataagcca cttatttttc acttccatct 15000
ctcctacttc taccttggca actatttctc aaaaatcaag tctaaaagta tttctttgtt 15060
tttccctata ataattctat gacgttgagt tgggtttcta acatttggta catgagtgtt 15120
ttaaaaacat attgtaacta agtgtcttct aatactgctc tatcaaatta acatattcag 15180
gaagtgttaa gtgagacagt ccatcacaaa tcaaaattaa aatcaatttt gatgtctcca 15240
aacaatgaga gaggcacttg catcttccga atttcgtcaa agcatttaaa ttatgattgc 15300
ttaacatatt caaataagct attgaatact cttttccaga tagatcacat ttttagtgtt 15360
ttcatgtgat atacatgaat ttgactctcc tgttactttg ttgcaaggac gctgtagtga 15420
atgtcttgaa ggcatagaca atttaagttg aatcccttct tataaaaaaa gaagctgtga 15480
atggacaatt ctcaatgtca tttcagagtc actagcaatg aaagggaacc tatttaagga 15540
ttttctctgc ctttcagtag tgaaaaaagc ttctggttgg tttacaccca cactatttta 15600
tttggaattt aattagaatg aatgtcactg gaggcaaata ctgtgtgcag catttctaaa 15660
ttcatttata aagatgccta ctagataatg cagattcagt ttcaggagtt tagttcctat 15720
agaattgtag gtaggctaga gagaattata gtcccttcta atcattaaaa cgtatgcatg 15780
aagtacttca agccagctta ttcatcactc ctgaaatgct gacaaaatgc ttaagggcct 15840
acttcttctg aaataaaatg agaaaatccc ccattcttta aggttcagga gaatattgtg 15900
cacgggaaga tctattttta aatgacactt cttataatgt gcagacaaaa ccacggatta 15960

gttactgctg ttcctcagca tgttactttg tacataaact tgaagctata tcagggagag 16020
aaacccactg ctaaaaggtt ttaaggcatg agttagaatg cttttaatat tcactttttc 16080
aaacatcatc atcctgaaga ttgtatctct cactgaaaat tctattcatg tgtgaggcta 16140
ccaattatta atgtaagaga cattcaaatt tgcagaataa tttgtatatg tttaaatatt 16200
tttatttaac ttagtaaaat aatggattta attataatgg atgcatatta tattgagatg 16260
ggatagtttg cccaataatg taatttcttg tagtagcata taatgatatc atatataata 16320
gatataatga aaccatttgg tggcaccatt ttaatagaat cagaatttat ctgaacttag 16380
ctctgaattt ttcaaattat aatcagtgga gcttcactaa tttccctgaa ataaattgta 16440
aaaagacgga aaaaataaga ccattacaaa aatattttta aatatatttt tatattttaa 16500
atatcatgtt atgaatattt gaagttaaat aatattaaat taatgtactt atcagaaaac 16560
ttaaattttc tgaattagaa ttgaagctac agtgactcta gcacttccaa atgtttttat 16620
atcattccac actaaatctt ggatcacctt taaatgaagt gcatataaaa aacgcttaag 16680
tgctcttagt aattttcaag caatatgtac aacattgccc agaatttgaa ctcttccaga 16740
tttcgtttcc aataaattta aattgctttg tgtagtgaag agtaatttaa aattatcaaa 16800
gtgctctaaa ttccacagta gagtacccat aaaatgtaaa gcctttcaat gattttaaat 16860
cactatacac actttagagt aatttcagca catcaaacgc tttgattttt agttttcatt 16920
gccttaattt gaaagaatga aaaaatgcca aaccaaaata gcatttatta tcagtgagag 16980
ttgtaactaa catgattttc aaattgactc agactttacc atgtgaaaca taccgttgtt 17040
actaatgagt caaaaaaaac tgtagccacc attatagcaa atatttataa tattaatgaa 17100
aatttatttg tcagacaaga tagaatgtat tattctagta tatatttaca gtggacccat 17160
agtatagttt tctattttac acatcccatg tgagtgtgaa agttggctac ttgcaacttc 17220
tcagagatat ttacatataa tttataaaaa tctgaattga ttcaaagtta agaaaacaga 17280
atcactcaat tacattggaa aatgttcaaa gaataaccag taaaggcaat aaaaatgctt 17340
caaaatttaa agtaacaaaa aatttatatt tttattctaa gaaaacacaa taaaatttgt 17400
tgacaattac taaaaatatc agaatatctc atatatgatt aagtaaaact cagttgattg 17460
atagtatact caattaaatt aagagcaata aaatgctggt gtaccaaggg ggttgataaa 17520
taacaccact cttgtatgcc caagttctta atcaaatgag aaactggata agaaaccagt 17580
aaagttaaac cagattctac atgattagtg ctttcttgtg gggaagggag cttataatgc 17640

tagattccag aattgttcaa gggtgttctg tctaaacaga ttcttcccca ggaataagct 17700
gttttactat gggccagagg aaacagcagt gcatatggcc tttgagaatc tcttagttcc 17760
aaagaaaaat aaaacagaga gttgtattct ttataaagaa tttgtcttcc tcaacttatc 17820
aattaacatt aactcatcta cacatcatga atagaatgaa attaggagtg gataggtaac 17880
aaagtgttac agtgaggaag catcagtaat agagatagag cattctctct tcatgtgaga 17940
cagtgcatca atgaggagaa atctattgta ttaatattac ctccaaaaat gttaagataa 18000
tgtcattaat tatttcaatg gatgctacaa agcccagtat aatatgtaaa gataaaattg 18060
tatgaaatgt ctcaattttt attaacttga tcatgaaatt aaataaaagc aactaagttg 18120
taaaaaattg gaaagcctga tacaaaataa tttaaatgac ataattctat gtcttcgaaa 18180
aaaattaaag tttaagaaat cagttaagta taagtattta tacaaagttt ataacctgtc 18240
cttataccag caaagtactt agagagtaaa tgatagataa caatgtttca ttattgcaac 18300
aaaatggaag gaagaaaaca ataaatagaa agggttctat aagacaaata cacgatcaaa 18360
tggaacaaaa ttaaagagct ttattttaat tatatattta tatgtgtata ataggacaag 18420
aagactaaat aaaatctata tttgttttct ttatatgaac actaaataac aaatattcta 18480
aacctgtcta ttaaaattaa tttattgttt ggataaaatt cttttttata gtaagtggaa 18540
tatcagaaag cagacttaat aaagttattt taaagttaat ctcaaaaaat aaaaacaaaa 18600
gcaaaaatac aaccctgaaa ttctggttca tctttggcag tgaaagctcc ccatagtcca 18660
tccatcctac taattgtaac tataaacttt gaatactttt tttttttttg agacggtgtc 18720
tcactctgtc gcccaggttg gagtgcagtg gcaagatctc ggctcacttc aacctctgct 18780
gcctgggttc aagtgattct cctgcctcag cctcctgagt agctgggatt acaggcgctt 18840
gccaccgcgc ctggctaatt tttgtagttt ttctaagtag agacggggaa ctttggatac 18900
attttttaga gtaaaaaaag taaattaaaa ataccctaat tgaaaactct gaaaataaac 18960
aagttagttt tttttaagag atgccaaaat ttgaaaaagc aactgttatt aaagtgattt 19020
gcattatttt tttttccttt gggatatttt ttcccattca cagagtggaa aagcataggt 19080
gactacaagt cttgctagac actctgctgt caatttggct aggaaaaaag caaaaacaaa 19140
aacaagtaat ggacattcag taaatattag cagagacaaa ggaatgttac aaaggaagca 19200
aatggaaatt ttttaaatga aaaataaaat atctgaaatt aaacatttat taagttcaac 19260
agcaaaaaaa gaataatagt aggaaaatat gttgaacata aaatagtatg tccaaaaagc 19320
cagatctaac ttcttattgc ctacaaggaa tcaactttga atataaagaa aatggtacaa 19380

attaatagat caatagccat tccccaatat aaagaacaca gaacaatgaa tgagactaaa 19440
gagatacttt gaaacagaaa aataaggaaa cctcaaaacg tgaaggacaa tattaaaagg 19500
tctaacgtat ttgtaatttc ggtacccaaa gttaaaattg gggcatactt ctaaataccc 19560
aaagactttc aaaatctgca gtacagtcaa tgacatacat ttacgaatga aagaagctca 19620
gcaaattcca aatagtgtaa gtgggagcaa aatgcctagg cagataaaaa ggggtcccta 19680
gagaatttct ggccttcttc acaagtgttt acattagatg cttttgtgca gatgagggaa 19740
cctgcccagg gcttgtctgg gcatgcccac agtggactgg agcctgacat gctcactggg 19800
gcaagtgggt ggagccatga ggaattcatg ccttgcaaag gggaggagcc tgccctcttg 19860
agctcttgtg gtgacctggg aatcaatgtg tgaggtggtg ctatgtagca ggaacccctc 19920
ttgctttgca aatagttttt tgtttgtttt cctttttgcc caatagagcc ctgctctact 19980
gacccttcaa tgtgcccgtg tgcctaaata ttcctggtcg tgtgaaaaga acccaggtat 20040
tagctgaact aaggagcaca attctgcaac attttggcac ccaaacacgg ggcttgagaa 20100
atggtgagta agatgcgaac aaaaacgccc ctttttttcc tttaccttct aagcctttgt 20160
gtcgtcggac tttttctgag agtaggggaa attgtacctc cctcccctcc actcctgtct 20220
cttccagcgg tcgggaatat cagccttttc ctgtttttaa gggatagaca ggtgaaccag 20280
aggctccctg ctcccctctt aagttcctcg ccctgctgga ggaacccatt tccataagaa 20340
taagattttc ttcctgcagg catctttcca actctgcact ttaaactttt ctgcctttct 20400
ctaccctgcc agcagttaac ttttaagtga gagttttgtt tttttttttt ccttttagaa 20460
gacattttac taggccagga attataagga tcactgttta tattctctgt aaagttttaa 20520
ttattaaaaa tgattcatga ggttgatttt aatctgtagc caatctagtg tgctttgcat 20580
ggctttctgt atggtcagta gcaactttgc cgcaggtctc catctagttt tacgtctttc 20640
aagtgtgacc tgtaacctca tggcaatgtt tcgtttagcc tccaccattt tacagtggca 20700
gctgggttca atcctggctt aaggaataaa ctccttctga tttgatatct ggatgacctt 20760
tgcaatttgt tgattctttt cccctcaaca gccttggatt tttctttccc tgagccttta 20820
gtaaagtttg aaagccagaa atattggctg ctcggcataa ctaaaaccag ataacaaggg 20880
atttaaaaca attttcttaa agagcacaca gcttaattaa aagtgtatat ccaagttata 20940
ggcatatttg aaaggccttt atttatttaa tttttttccc ccttggattt tgttttgctg 21000
gaaaaagggt tttttctcag ttggccgaat tctttttctc cattttgtct tgccactctt 21060

gatgcatgca cgtgaggctc taaggtaact ctggggtgca tgggactcct tgagaaaaac 21120
agaaggcacc actgacccct ttttgggaag aaatctgttt tcctcatgga accccaggaa 21180
ttaaaagcag atagatccct ctcaaaatct gtttttgctt cccaattatg cctgtttatt 21240
acgccctaga agcagcatgt tttcctagcc ctgtctctta aagggatccg cctggaggac 21300
aataatccta ttaggagact ggcaaatgaa aaaccttgtg gctgctgagt tttcttctgc 21360
ctggctgtgt agttatatat gtgttgtatt tgtgatgttt ataaaaacag ctctaattaa 21420
ttggcctaaa gaaaaataag cacttggatc aaatattttt taatggaaag ataaaagctg 21480
tggtagcttt tagttctcat gactttaatc tttgagaaat aaaaacagcc ttaaagatta 21540
tttggtgaaa tgcatatgtc atcaaaattt aaataggtgg actaaattat gcacgtcaga 21600
tactaggttt gctaaatgtt ttaaggctat aaaatgcttt ttgagaatga ttcaatttgc 21660
tggcttcaca actggtaagg cccggagaca tatgttacta accacgccct tagttgtgct 21720
ggaaggagtc aaaccttggc tgcccctagc acataattaa aacaacttac caggttttac 21780
tttaaaggta aaaattgcta ggagttacca ttataacatg taattgagac cactaaaaat 21840
atatttacat ttgagttatg tgagaacagt aaaatatgtt tttagtaaaa gattataaga 21900
aggcatagaa atgtaaaatt ctgcctaagg ttaaaagatt gttttgaatt aaataagata 21960
acagtaaaag ttcaaacaag ttgtggaagg attgcaaaaa ttaaccttgc aaaggaaatc 22020
ccatgtgtga acatattgac taaattcaaa gaatattata tggtttttct gtaaattcag 22080
cattgaaata aaagcataac aaggcactct taaagcagta agctactctt cagcaaaatt 22140
tgtaaagtgt tataacagat ttttgctttt agaatttctg agtcatcatt ttagcaaagt 22200
aaataacatg gtaatctgga atcctatttc ataacatcaa gtattttaaa tccctaacat 22260
acttaacagc cttcccaaaa tcaaacttca gtttcaaaat tgtctttctt gataccagac 22320
ttttggatgc tacagaggac acctgcagca tcaaaaaaag taaggtaaat ggattatctg 22380
gtatgtttta ggtacatggg attgacaaaa cggtgttcaa tcctttttcg gttgtatttt 22440
tgtgaataat actaatagta ctaataataa tactaatgta tagttccaaa cttttatggg 22500
atttaaaaaa ttctaatgtt tgagcatata ctatcaatca taattatagc tattatgtta 22560
aattattgta aaccatggag ataaccaaat ttatttgtca atcatgtttc tgactgcaac 22620
taccctggac attttgttac tcacaaacca ttgttgtctt gttttgatcc tatcctcttc 22680
acatgatgtt tataatcagc tatggaactt tgacaggtgt tctcaaatgc aggcttctga 22740
taactttgga gattgtgaca ttggaataaa ggaaaaacat acaggctcca tgaaaattga 22800

aatgttcatg gatagcaagc aaaacaagag ttaactgaat ggactgaact aatagaaaac 22860
tgaagtaatc gttttagctt ttgcttggaa tattgctgat cattattttg tttttcagag 22920
tccaggaaac ttattttgaa ctatttatga cctttaataa ttgagtaagg tgtactcctg 22980
tgaacaaaat ttggagcatt atttgtctct ctctgcctgg cttctccaga atttggaaac 23040
taatattgag tattcttaac ttatggcagt atagtcattt gcatcagtgc aataagaatc 23100
ctttttcttt tgcaacagaa tgcaattgga gaaactggtt gttttaccag gcgttgattg 23160
gaaatgtgtg cttcccttta agcagtcaag ctcaacttgc agaactgatg ggaacccctt 23220
gggaaaactg gcctcaaatg tttgtctaca cagtccacat acagggttct taacctgcgg 23280
tgagtaaaga atgtcacttt ccaacaggcc cagatatcat atgctcttgg gacctcaaga 23340
agagaggagt ttacccaatt cataggtgtg tgaggataaa aacctagggc tgggctcagc 23400
tttaaatggt cttatctgag attctttgtg aaacagattt tcatgaaagc caatctagag 23460
ggcctacgta gaaataattg tttttgctgc actttatgca cataatcaag ccaattataa 23520
gactaaagtc tatattgcaa gcaacacact cctattatga tttgttttta acagaaatga 23580
ggacgagaca gagagaaatt atgtttcaaa acttatcatc attagtttct agacttatta 23640
tttcttttta agtttttacc tacattttag actgaccctg cttgtgcctg tgaaccaacc 23700
aacaatctct ggctgcagct cagaaaggac aaaagagaat gggtaatgta aaaacctgga 23760
tcagtatttt agttctgaac aattatcctg caaatcctgc cagctgatgg gaataaatag 23820
gatgcccatg atccggaggt ttactttttg ggaaagtaaa accaagggag ctaaccaaag 23880
taaaacccca tacacccaaa tctgagcaag cataactata gccaccagtt atctgggtgt 23940
gtcacaagac actcttttct ttcctttgtt ggaggagaac tcatttccac agcttcactt 24000
tagcattatg gcttatgtta aggagtctat gcaacttcca tgctgagaca cgtttttggt 24060
cccaaagtta attccaagct ttgtgtcaaa ggcctaagaa aactggatct tagggatcca 24120
gaagccagac aataatgaaa gtcaaaaggc acagcacagg tgagcatgac taattcccgc 24180
tgattaagcc aagcttcctg tttcatggat aaaggtcatg ctagtatcca tggcataaat 24240
gaggtctaga gaactaaaag gctactgcca gcaggggaga tagggaggac ataagacagg 24300
gcagctattc ccatccccta gaccccctgt taacatggat gaaagccact ttgacaccca 24360
tgggtggcac cctgtcacag ttggtgggac tcagcaatac agggacagag gaaagaaaga 24420
ggaacaactc actttctctc cctcacacac cctgagtatt tgctagaaaa agaaaggaac 24480

caggcacaca tgctccccta tttctagatg agtagccatt catctttagt ctgcaccctt 24540
ttagaacatg tcctgaaccc ctgggactct tctgaaaaaa atgccttctt ttttcctttt 24600
tcttcctctg tcctctattc actggtaggt aggtaatggt gtcccatacc acagaacact 24660
tccctcagat gcatccttct aactgggagg agttaatttc tcaaaccgta gactaattgg 24720
cttaggatta ggttcagggg aagggaacac agaaacatga catgccagca aaagggtaaa 24780
aggttttttt tccccgttgg gcttttggcc tctctctccc tgtgcaaact ggtaaaaggc 24840
gtctggattt tctagttgtc cttacccact gcttgtttcg ttttgataca tattttctaa 24900
taacctgttt gtctcttctc acttttaggc catcaaactc cagtctgcac ctggagcctt 24960
ggatgatggc cttttttact gggaaccctg aaataggcct ctgagggaaa tctgattgcc 25020
attttcccaa aacagcttcc ctgacaatag gaagcaatta agattggtct tcatccttat 25080
ctttatcctt attctaatgg cagttagagg tacttcttta gaggggggaa tgagacagcc 25140
aaatgcctag gcagataaaa aggtgtacct ggaaaatttc caacccgtca cacaagtgtt 25200
tacatcagat gcttttgtgt agatgaggga aacttcccag ggcttgtctg ggtatgccca 25260
cagtggactg gagcccaaaa tgcacactgg aggaagtgga tggagccacg tggatgtcat 25320
gccttatgca ggggaggagc ctgctctctt cagctcctgt ggtaatgtgg gaatcgatct 25380
gtgaggtgag gacctgttag caggaacccc tcttgctttg ctgaaagttt tatttttctt 25440
tttgccctat aaaaccctgt tctactaacc cttcaatatg tctgcattcc taaattttcc 25500
tggtcatgtg acgagaacct aggttttagc tgaactaagg agcaaaattc tgcaacataa 25560
agtcaaagaa aactatgcct atatccatta aatcaaatgt taaaaagcaa atataaagaa 25620
aaagtcttca cagccctaga gaaaagtgaa acattgtaca taaagaagta ataatttgaa 25680
tggattgtca gaaaccatga ctagaagaca gaggagcaat attttcaaat ttcatagaga 25740
aaggaaaaaa agaaaagaag aaaaaaaaga agacataata aaacacctgt cattttagta 25800
ttctgtaccc attgaacata accttcaata gaatgtgaag tagacatatt caggtgaata 25860
aaaaatcaag agaataatag ttatgttcta catgaaatta taaaggaaat ttctcagact 25920
aggtggaaat tatactagtg agaaacagat ttgaaggact aaaggaagag atgatagact 25980
gacaaagtca atttttaaaa gccagatcta acttcttatt ggctacaagg aatccacttt 26040
taatatacaa aaataggtaa taattaaatt gctagaaaat acatttcaaa taaagaatca 26100
gcacatgtag cttggagaag atatattaac ataaaaagga cttcaataaa aggagtatta 26160
tgagaagaca tttatgcagc caaaaaacac atgaaaaaat gctcaccatc actggccatc 26220

agagaaatgc aaatcaaaac cacaatgaga taccatctca caccagttag aatggcaatc 26280
attaaaaagt caggaaacaa caggtgctgg agaggatgtg gagaaatagg aacactttta 26340
cactgttggt gggactgtaa actagttcaa ccattgtgga agtcagtgtg gtgattcctc 26400
agggatctag aactggaaat accatttgac ccagccatcc cattactggg tgtataccca 26460
aaggactata aatcatgctt ctataaagac acatgcacag gtatgtttat tgcggcacta 26520
ttcacaatag caaagacctg gaaccaaccc aaatgtccaa caatgataga ctggattaag 26580
aaaatgtggc acatatacac catggaatac tatgcagcca taaaaaatga tgagttcatg 26640
tcctttgtag ggacatggat gaaattggaa atcatcattc tcagtaaact atcacaagga 26700
caaaaaacca aacaccacat attctcactc ataggtggga attgaacaat gagatcacat 26760
ggacacggga aagggaacat cacactctgg ggactgttgt ggggtggggg gagaggggag 26820
ggatagcatt aggagatata cctaatgcta aatgatgagt taatgagtgc agcacaccag 26880
catggcacat gtatacatat gtaactaacc tttacgttgt gcacatgtac cctaaaactt 26940
aaagtataat aataataaaa taaaataaaa taaaaattta tcagaaaaac aatatcaatt 27000
ataatgtata tacctaataa gagagctcaa aatatttgaa acaaatctga cagaattaaa 27060
aagaagaatg gataaattca caattataga aaaagattat acaccagttt cttaataatt 27120
gatacaacca ctggagaaat aataagaaag caaaaataaa gacatagatc caaataacac 27180
tttgtactaa tcgatatttc tagaaaacgg cagtcaacat tgttttcccg tgttcatgta 27240
tattcaccat ggtagaccct ttactgtata taaacctaat gttgataagt ttaaaaagat 27300
taacaattta tagtgtatta tccagttaca atgaaaccta attaaaaatt agtgattgta 27360
atctaggaaa gcgaagctct tttaaatcat ccattactca aagaggaaat ctctaaaatg 27420
taaattctgt gaaaagggag tttagtttta tacactgttt tgttcccagc atctagacga 27480
gtgactggca attagtaaat ggttaacaac tattattata agaaatgccc caggggacag 27540
agaaagaaat gataaggaaa tatgcaacga tttttcatga gaaataaatg atgaacaggt 27600
ttttcatgaa agatgtaaca gaggcccagc atggggcggg atggcaatac acattggtct 27660
gtctcctagg aaacacacag attaatggct aaaaacacat gcctacaaaa gacaatactt 27720
agagtttaca atcaagactc tattacgaac tctataaact agagcaatat tctaaaatta 27780
ttgattcttt agtttcctaa tagaaaaaac ttaagaaata atgaagcaaa gtttttttgt 27840
tgttgttttt tggtcaagct taattgaagt gatttgtgtt aacgtgctca gtacagtaaa 27900

tggttcgaaa caaagaaatc aatgaataat actattatca caattatatg ccgttaatgc 27960
cagaaagact aagcatctga ttcctaacat caaggtttgt aagagtgtgt gaactttgta 28020
acctcactta atctcttaga tatatatttc ctttaaggaa aacatgggag aaaagagcat 28080
ttgtgtgcta tggagcaatt tagaacacat ttcttggggg aaaatggcta atataatatt 28140
aatattagta ttattgtgat taaatggatt aggaggttct acaaagaaaa gtaggttaat 28200
ttagagaata aatgtaaact acaaatacaa taactcattt tatcaaacta ttttggtgtt 28260
atggattttt acacagtgaa caaaacacac ttgattttct ttaggaaatg atatactatt 28320
aagaaatgag tatatagagg cttagctcta tttttgaaat gactctctaa ggtcttacaa 28380
attgaaaaag caaaactcaa gacaaaaaaa aaattaattt gcaaaagtca gtattatcta 28440
caggaattaa caaagaaaaa gactcaaatc tctgaatatt taatgtgtgg gtttatgagt 28500
tttttttttt ttttgctttg ttttgctttt ttgagatgga gtctcactct gtcacccagg 28560
ctggagtgca gtggcgtgat cttggctcac tgcaacctct gcctcccagg ttcaagtgat 28620
tctcctgcct cagcctcctg agtagctggg actataggtg cccactacca tgcacggcta 28680
atttttgtac atttagtaga gacggtctca ccatattggc caggctggtc ttgcactact 28740
gaccttgtgg tccacccacc ttggcctccc aaagtgctgg gattacagtt gtgagccacc 28800
gtgcccggcc aagtttaata tgttgttcga tttgttactt tcctttgtct ccgcttcccc 28860
tctctgaatt aaaatcaatg ccttgatttg tcaaacaaat atgtagtgag tacccactat 28920
gtataaatct ttgcatcatt tgccacaaaa aaaggctgat atcaattata taaagcatta 28980
gaatgtacta gaaacaataa ggaaaaaagt gacagataaa tacaacagaa atatatctaa 29040
tattgcttta aagttataat ttgtaatttt aattcaacct gtagcagtct ggttagtttt 29100
aagtatttat tgaaattcta tggactgtat ttgtaaactg agaattaaaa caaatagcct 29160
gcagattttc tgtattgact tattagaatg gtatatataa attgcctgcc tgatataaag 29220
taagtcactt catatatagt agttactgtt actatatttg gggaaaacca tgaaagtttc 29280
atgaaaaaaa gtgtcctttg agcttttaca aaccagctta gtaggtagag tctgaaagtt 29340
aaatgtgtgt gtgtgcacgt gtgtgtgcca tggcccactt ggacgatctg ttcagtttta 29400
caaactaccc atcaaaatat gtgtttaaat gcataaatta catagaaata catagtatta 29460
caataaacat gaaatatatt gacaattatt acagtatttt ataaattgta acatatatat 29520
gcttctcata acacaataaa taagaaagca tttaaatcta ggcaagttaa gttttatgtt 29580
tcaagaagga ttagtgtaat gaatgtaatg gctgaacatt aaactctagg ctggttgtta 29640

actgcctttt acatattgat cagcaaatga ttgtgaatgg gcaatgatgg aaagtaactt 29700
cttgtctggg tcagaattgg agaactctga atttgagtgt tttgtggcct aatgactatc 29760
gccaggccca atacagctca atagatggcc agttttatgg catagccatc tgtagagagg 29820
aagatggtaa tcactaatgt tttgttgatc aaaataatta tgattttgca ataaaggtaa 29880
ctataaatgg ctttttgatg tatctgcaaa agatatatcc aatgaaaata atctgaaatt 29940
tcaaagatgt gttgtttcca tgccggatct caaaatccct gaatttcaca cacagattcc 30000
atgaactcca agttaagaac acatgatctg gatgtttgac aaaatacctt cagcaggaat 30060
aatgaaagcc ctgacatttc tgtgtgcata aaaactggaa aactttaaaa gtaataaatc 30120
accaagtctt tataaaaatc gatgagttag gtgtgtatta gcttatatcg cagatgagga 30180
aactgtaaca tggaatggat aaataatttg ctaaagtcac atagattgtg agtaggaaac 30240
tggaaattga acaacagctg tattgcttca tggtcagtgg ttaatgcttc aataactaca 30300
ctaaaaggct tatttaaaag gctttttcac agatgcattt attctcttct acataattag 30360
taatgaaatt ttagtaatat ctgtagcaaa aagaaccagg tttagattgt tgtacaaaat 30420
ggcaaatgca tagagggtca gttataccat gaaaagtttt atgaatactc aaactcacct 30480
actaatggca acagtgagag aatgtggagg tcatcctgaa gagagatcat cacccaggcc 30540
tatcaaatct aaccactcct atgttacttt ctcattttta atttagcatt tttcatatgc 30600
tgccacgtat atgccatttt tcactatatg atttgggaag attttcttta aacaataaaa 30660
caaaattgtt gaccacaccc tcaacactag gtgatcatat attttaggct aaatcattta 30720
tccttatata aaaggaataa tattatccct gaaaatatag aagactaatt ggtatatata 30780
tgtgtataca tatatatata tatgattaaa tcaatatgta tattcaacct gagaatgact 30840
ttttgatatc acaataccct tgagtatcag gtttttgaaa ggatctcaga cttctagaag 30900
tccagtctga aaatgtaatc tttggaaata caggtatcaa aaggttttca aaatatttga 30960
gtgtaaaaca gaattattta ctaataagtt attaatattt atataaagca tatagccgtt 31020
tgggaatatt aaatattttt atgttatctt ttgtggcact atgaaagatt ggagtctttc 31080
atgtctatca cattgtcatt ttaaaagaca ctaaataaat ctaaaaatag taattataat 31140
aattagtcat cagaagatct aggctttcag atgttaaaag ttatgactta gtcaccttca 31200
tggaagcatt taattgagca agaaactcaa tgtgaaattc atatgtcgct accaaaaact 31260
gtcataaatc aatgtgcttc attacattac tgccataaat aagtggctta ggtggaaaaa 31320

gaaatatgtt ttgtgatatt attcagtaac cgattttaac attggaatct ttaaaaaaat 31380
caaatccagc tggtcatgta tatctcatag attgcaggtc ttttccattg tagactgact 31440
gcaatttgga aatgtggatt tgtcaaccag gaaatgtatg agatatgaag atgctgtctg 31500
agatagttgg tccttcagaa atctaataga gcaacagtct agatatactg aggaataata 31560
gctatatgtt aataaattcc ataattgttc tcataggctc agtgtgattc agcataggaa 31620
actcataaaa cattgctatg gcctttagaa gcctgaaatg caagtctcct tgagcaacag 31680
gctactgaag agatctcaga ctcttacaag tcccgtccaa tagtgtaaca catttatgta 31740
tatcctatat ccaataatgt aactaaactt aaactacccg ataactaaaa ttatttggta 31800
aagctcctat atttcatcca ccttagataa aataatgctg gttaaatttt cattttgaag 31860
aattgtttgg ttattggcta aattttaatg agccatgatc ttagtttagt aaatttttaa 31920
cataaaatta tgctaaacaa gatatgatat ataaacaaaa agactttaat ttcttaggct 31980
tctttttatg gtgcactttg ccatttaaaa attaaatttt ttatgctata aattcagtaa 32040
ggtcatacat acgtaaaact ttctataata gtctcgattt tgagatggct tgagggtcag 32100
atgtaaggag attttacata ggttagactg tgattagtag actggatttt tttttttttt 32160
tttttttttt tttttttttt gagttggagt tttgctcttg ttgcccagac tggagtgcaa 32220
tggcattatc tcagctcact gcaacatccg cctcccgtgt tcaagcaatt ctcctgcttc 32280
agtctccgaa gtagctggga ttacaataga ctggatttta tcaaggaatg tatttcttct 32340
agaaaataga tacactttca aagatacgtt aatttacatg agagcttgcc tgtgtgcaca 32400
atagaaagcc aaaaataaag aggttatcat ctaatggcac aaagttttct tatttcataa 32460
gcttgcattt tcttttctta ttaaacaccg tctcaccttt ctttttcagg aaacactttc 32520
tgaaaatgtc agaactcttg aaaacatgga tgaggaaaag cataagtact gctttatgct 32580
tttcttatga attttaaaag atgtaattta aaggtacttt aaagtgatat ttgtgccctt 32640
gtaactgatt caaagtagac tgctaaggac aataagtaat aagagtctac aaatatcagt 32700
aaattatgag aaaaaacggt ctaatggtac ttaaaattgc attgtgattt attaaataac 32760
attgaacagc tccacctgta acattaagat tccatttgag catatagaaa atatatttat 32820
cgcaacctga agtgatttgg aaagtatgct gggaagaaaa tatatttaca aattagtctc 32880
agagggtagg tatcctgtag ccagatggaa taagaagaga gggaagggag attttgttca 32940
aagggggagc ataagctaag gaataaagcc acaaacaatg attaaattag gtaaagtgca 33000
attagctgaa tattgcataa gcatacatat gggaagaagt ggaaaaagga gtgacaatat 33060

gaagctaaag ggtttgaagt gttcaagata atactaggct tcctagccta ttccgagaca 33120
tcttgtttta gagtgcaata gataagaact ctaaggatga aagtaggaga catgcattct 33180
agtctacgtt ttggttaatg gactgaaagt gaaagatgga ctgaaattgg gggatggaat 33240
aaaaatgtga tagaatagaa aatagaaaaa gcaattagcc taatgtcatt gatacttaga 33300
gaaaatatgg aaaatagtaa attagtgata tatatctttt ctaattcacc agggtgagtg 33360
aacaaaggaa atcggattta aaagtattca tgacatagat gcaataaggt ttctcagtca 33420
gacatagcaa attgagggaa gggaagattt agaggattgg aaaatgtctt gcttgtagac 33480
taataggata tcaataagcc atatgagcaa taatttgaaa tagtttttgt aaagtatcat 33540
gaaggtaaac cctgtttaga acaaattaga tttgaagtac ttgtgacaaa tgcaattgac 33600
aatgccttta tgcagctgga aaattgttgc ttagcagaaa agcttgtgct ggacaaatag 33660
atgtccaaat tgaatagatg ttttcttaag ctgaactaat tgagacaaat atccttatta 33720
aatcaaaaaa ttaaaaattg aaataactta cctatgatag ggtaaataat gttattaata 33780
tacacaagaa atatgtgatg tatatgtgta tgtacatatg tatacatagt aataatacat 33840
agtgctattt ttaaatgata ttttacatag tgctttttta aatgattttt caatcatcgt 33900
tgttggtctg tgttatttta atcatcaaag ctgattgatt tctgtttttg tgtttttttt 33960
tgtttttttt gaggtggagt ctcactctgt cacccatggt ggagtacagt agtgcggtct 34020
tagctcacta caaactccac ttccctgatt caagtaattc ttgtgcctca gcctctcgag 34080
tagcttggat tgcaggcatg caccaccacg agctgctaaa ttttttgtat tttttgtaga 34140
ggggttttgc catgttggcc aagctggtct caaactcctg acctcaagtg atccgccctg 34200
gcttcccaag cccagctgat tcctgtttaa ataaaaactt acaaagatct tcccacaagt 34260
actttatctt acaatttact gaacaataga ggcagtgaag atgtttttca gaaagcaatt 34320
gtgatcctga cattttcttc ttataatcct gtgctttcct cattttttta tattaaaagt 34380
aatagttctt gtgatggcaa ctaggatttg taacaacatg taccctcatt tcctgcatga 34440
ccttttttat gtcacttctt cacatgcaaa gtatactccc tcagtgtcat tacattattt 34500
gatttttgcc ctttccctca cctttccatg tgtttatttg cctatgccca tctcctagtc 34560
acattttatc tagttaacgc caacttgact ttcaatgctg ttcaagattt acctcaatct 34620
caagtgtttt ctctttatat tccatacacg cttagcttgg ctgccaatcc tcttggctgc 34680
caaagaatcc caaattatct ctttcaaaaa tacaagaaat aaaggatgga ggataggaag 34740

gagggtggtc aggaaagaac aaagaaagga ggggagagga aaaaaagaga gaaagaaaga 34800

gaattaaagt taaggaggaa gaatttcaca ctgaattaaa attgataata ttcaggaatg 34860

attttatagt aggcatacat tgataaatat tagttaaatt aatgaattat tccaaaagat 34920

catgatttgt aaaaattacc ctgaagcact ttaggaatat tacattgaaa taggtatgca 34980

atttctaaag aaactggact gcaaaaatag ccattgtgtg tacctgtgga ccagaatatt 35040

atattttgct taagtttgaa gtgttaaagt gcccaacgtg ctcttatttt aacctagcaa 35100

attttcagca gaacttaagc tcatatagta tttgattttt tcatttctca caactgttgg 35160

aattctgttt tattgaaaat tatacaataa aatgttaaaa aatctttgtt cctgcagtta 35220

tgcaatctta tgccaaagtc aataaattat taaattaaaa tcaaacccac taagcctaac 35280

aatttgagaa aatgattatt aaaacatata ccatttagaa gggcaatgcc tttttaatat 35340

tatagtgaag acaaccctat caattattta atacaccaag aatacaagga tataaaatct 35400

ttttctctca tatatcacat tatctgatgt atttagctgt gatgacattc cagaaaacat 35460

cactttttaa aatttctaag attttattat gttgctagca aatcgatttt tttcaagtac 35520

tgtaacaaaa tccctaaatg ggctttatga gaactatttt cagtgtgata ttaatcatat 35580

tttatt 35586

<210> 2
<211> 1535
<212> RNA
<213> Homo sapiens

<220>
<221> mRNA
<222> (1)..(1535)
<223> mRNA encoding CLA2.1

<220>
<221> 5'UTR
<222> (1)..(237)
<223>

<220>
<221> CDS
<222> (238)..(399)
<223>

<220>
<221> 3'UTR
<222> (400)..(1535)

<223>

<400> 2
guggugaccu gggaaucaau gugugaggug gugcuaugua gcaggaaccc cucuugcuuu 60

gcaaauaguu uuuuguuugu uuuccuuuuu gcccaauaga gcccugcucu acugacccuu 120

caaugugccc gugugccuaa auauuccugg ucgugugaaa agaacccagg uauuagcuga 180

acuaaggagc acaauucugc aacauuuugg cacccaaaca cggggcuuga gaaauga 237

aug caa uug gag aaa cug guu guu uua cca ggc guu gau ugg aaa ugu 285
Met Gln Leu Glu Lys Leu Val Val Leu Pro Gly Val Asp Trp Lys Cys
1               5                   10                  15

gug cuu ccc uuu aag cag uca agc uca acu ugc aga acu gau ggg aac 333
Val Leu Pro Phe Lys Gln Ser Ser Ser Thr Cys Arg Thr Asp Gly Asn
            20                  25                  30

ccc uug gga aaa cug gcc uca aau guu ugu cua cac agu cca cau aca 381
Pro Leu Gly Lys Leu Ala Ser Asn Val Cys Leu His Ser Pro His Thr
        35                  40                  45

ggg uuc uua acc ugc gac ugacccugcu ugugccugug aaccaaccaa 429
Gly Phe Leu Thr Cys Asp
    50

caaucucugg cugcagcuca gaaaggacaa aagagaaugg augagggaaa cuucccaggg 489

cuugucuggg uaugcccaca guggacugga gcccaaaaug cacacuggag gaaguggaug 549

gagccacgug gaugucaugc cuuaugcagg ggaggagccu gcucucuuca gcuccugugg 609

uaauguggga aucgaucugu gaggaaacac uuucugaaaa ugucagaacu cuugaaaaca 669

uggaugagga aaagcauaag uacugcuuua ugcuuuucuu augaauuuua aaagauguaa 729

uuuaaaggua cuuuaaagug auauuugugc ccuuguaacu gauucaaagu agacugcuaa 789

ggacaauaag uaauaagagu cuacaaauau caguaaauua ugagaaaaaa cggucuaaug 849

guacuuaaaa uugcauugug auuuauuaaa uaacauugaa cagcuccacc uguaacauua 909

agauuccauu ugagcauaua gaaaauauau uuaucgcaac cugaagugau uuggaaagua 969

ugcugggaag aaaauauauu uacaaauuag ucucagaggg uagguauccu guagccagau 1029

ggaauaagaa gagagggaag ggagauuuug uucaaagggg gagcauaagc uaaggaauaa 1089

agccacaaac aaugauuaaa uuagguaaag ugcaauuagc ugaauauugc auaagcauac 1149

auaugggaag aaguggaaaa aggagugaca auaugaagcu aaagguuuug aaguguucaa 1209

gauaauacua ggcuuccuag ccuauuccga gacaucuugu uuuagagugc aauagauaag 1269

aacucuaagg augaaaguag gagacaugca uucuagucua cguuuugguu aauggacuga 1329

aagugaaaga uggacugaaa uugggggaug gaauaaaaau gugauagaau agaaaauaga 1389

aaaagcaauu agccuaaugu cauugauacu uagagaaaau auggaaaaua guaaauuagu 1449

gauauauauc uuuucuaauu caccagggug agugaacaaa ggaaaucgga uuuaaaagua 1509

uucaugacau agaugcaaua agguuu 1535

<210> 3
<211> 54
<212> PRT
<213> Homo sapiens

<400> 3

Met Gln Leu Glu Lys Leu Val Val Leu Pro Gly Val Asp Trp Lys Cys
1               5                   10                  15

Val Leu Pro Phe Lys Gln Ser Ser Ser Thr Cys Arg Thr Asp Gly Asn
            20                  25                  30

Pro Leu Gly Lys Leu Ala Ser Asn Val Cys Leu His Ser Pro His Thr
        35                  40                  45

Gly Phe Leu Thr Cys Asp
    50

<210> 4
<211> 1535
<212> RNA
<213> Homo sapiens

<220>
<221> mRNA
<222> (1)..(1535)
<223> mRNA encoding CLA2.2

<220>
<221> 5'UTR
<222> (1)..(500)
<223>

<220>
<221> CDS
<222> (501)..(710)
<223>

<220>

<221>  3'UTR
<222>  (711)..(1535)
<223>

<400>  4
guggugaccu gggaaucaau gugugaggug gugcuaugua gcaggaaccc cucuugcuuu      60

gcaaauaguu uuuuguuugu uuuccuuuuu gcccaauaga gcccugcucu acugacccuu     120

caaugugccc gugugccuaa auauuccugg ucgugugaaa agaacccagg uauuagcuga     180

acuaaggagc acaauucugc aacauuuugg cacccaaaca cggggcuuga gaaaugaaug     240

caauuggaga aacugguugu uuuaccaggc guugauugga aaugugugcu ucccuuuaag     300

cagucaagcu caacuugcag aacugauggg aaccccuugg gaaaacuggc cucaaauguu     360

ugucuacaca guccacauac aggguucuua accugcgacu gacccugcuu gugccuguga     420

accaaccaac aaucucuggc ugcagcucag aaaggacaaa agagaaugga ugagggaaac     480

uucccagggc uugucugggu aug ccc aca gug gac ugg agc cca aaa ugc aca     533
                      Met Pro Thr Val Asp Trp Ser Pro Lys Cys Thr
                      1               5                   10

cug gag gaa gug gau gga gcc acg ugg aug uca ugc cuu aug cag ggg     581
Leu Glu Glu Val Asp Gly Ala Thr Trp Met Ser Cys Leu Met Gln Gly
            15                  20                  25

agg agc cug cuc ucu uca gcu ccu gug gua aug ugg gaa ucg auc ugu     629
Arg Ser Leu Leu Ser Ser Ala Pro Val Val Met Trp Glu Ser Ile Cys
        30                  35                  40

gag gaa aca cuu ucu gaa aau guc aga acu cuu gaa aac aug gau gag     677
Glu Glu Thr Leu Ser Glu Asn Val Arg Thr Leu Glu Asn Met Asp Glu
    45                  50                  55

gaa aag cau aag uac ugc uuu aug cuu uuc uua ugaauuuuaa aagauguaau     730
Glu Lys His Lys Tyr Cys Phe Met Leu Phe Leu
60                  65                  70

uuaaagguac uuuaaaguga uauuugugcc cuuguaacug auucaaagua gacugcuaag     790

gacaauaagu aauaagaguc uacaaauauc aguaaauuau gagaaaaaac ggucuaaugg     850

uacuuaaaau ugcauuguga uuuauuaaau aacauugaac agcuccaccu guaacauuaa     910

gauuccauuu gagcauauag aaaauauauu uaucgcaacc ugaagugauu uggaaaguau     970

gcugggaaga aaauauauuu acaaauuagu cucagagggu agguauccug uagccagaug    1030

gaauaagaag agagggaagg gagauuuugu ucaaaggggg agcauaagcu aaggaauaaa    1090

gccacaaaca augauuaaau uagguaaagu gcaauuagcu gaauauugca uaagcauaca    1150

uaugggaaga aguggaaaaa ggagugacaa uaugaagcua aaggguuuga aguguucaag    1210

auaauacuag gcuuccuagc cuauuccgag acaucuuguu uuagagugca auagauaaga    1270

acucuaagga ugaaaguagg agacaugcau ucuagucuac guuuugguua auggacugaa    1330

agugaaagau ggacugaaau ugggggaugg aauaaaaaug ugauagaaua gaaaauagaa    1390

aaagcaauua gccuaauguc auugauacuu agagaaaaua uggaaaauag uaaauuagug    1450

auauauaucu uuucuaauuc accaggguga gugaacaaag gaaaucggau uuaaaaguau    1510

ucaugacaua gaugcaauaa gguuu                                          1535

<210>  5
<211>  70
<212>  PRT
<213>  Homo sapiens

<400>  5

Met Pro Thr Val Asp Trp Ser Pro Lys Cys Thr Leu Glu Glu Val Asp
1               5                   10                  15

Gly Ala Thr Trp Met Ser Cys Leu Met Gln Gly Arg Ser Leu Leu Ser
            20                  25                  30

Ser Ala Pro Val Val Met Trp Glu Ser Ile Cys Glu Glu Thr Leu Ser
        35                  40                  45

Glu Asn Val Arg Thr Leu Glu Asn Met Asp Glu Glu Lys His Lys Tyr
    50                  55                  60

Cys Phe Met Leu Phe Leu
65                  70

<210>  6
<211>  1463
<212>  RNA
<213>  Homo sapiens

<220>
<221>  mRNA
<222>  (1)..(1463)
<223>  mRNA encoding CLA2.3

<220>
<221>  5'UTR
<222>  (1)..(237)
<223>

<220>
<221> CDS
<222> (238)..(537)
<223>


<220>
<221> 3'UTR
<222> (538)..(1463)
<223>


<400> 6
   guggugaccu gggaaucaau gugugaggug gugcuaugua gcaggaaccc cucuugcuuu 60
   gcaaauaguu uuuuguuugu uuuccuuuuu gcccaauaga gcccugcucu acugacccuu 120
   caaugugccc gugugccuaa auauuccugg ucgugugaaa agaacccagg uauuagcuga 180
   acuaaggagc acaauucugc aacauuuugg cacccaaaca cggggcuuga gaaauga 237
   aug caa uug gag aaa cug guu guu uua cca ggc guu gau ugg aaa ugu 285
   Met Gln Leu Glu Lys Leu Val Val Leu Pro Gly Val Asp Trp Lys Cys
   1 5 10 15
   gug cuu ccc uuu aag cag uca agc uca acu ugc aga acu gau ggg aac 333
   Val Leu Pro Phe Lys Gln Ser Ser Ser Thr Cys Arg Thr Asp Gly Asn
   20 25 30
   ccc uug gga aaa cug gcc uca aau guu ugu cua cac agu cca cau aca 381
   Pro Leu Gly Lys Leu Ala Ser Asn Val Cys Leu His Ser Pro His Thr
   35 40 45
   ggg uuc uua acc ugc gau gag gga aac uuc cca ggg cuu guc ugg gua 429
   Gly Phe Leu Thr Cys Asp Glu Gly Asn Phe Pro Gly Leu Val Trp Val
   50 55 60
   ugc cca cag ugg acu gga gcc caa aau gca cac ugg agg aag ugg aug 477
   Cys Pro Gln Trp Thr Gly Ala Gln Asn Ala His Trp Arg Lys Trp Met
   65 70 75 80
   gag cca cgu gga ugu cau gcc uua ugc agg gga gga gcc ugc ucu cuu 525
   Glu Pro Arg Gly Cys His Ala Leu Cys Arg Gly Gly Ala Cys Ser Leu
   85 90 95
   cag cuc cug ugg uaauguggga aucgaucugu gaggaaacac uuucugaaaa 577
   Gln Leu Leu Trp
   100
   ugucagaacu cuugaaaaca uggaugagga aaagcauaag uacugcuuua ugcuuuucuu 637
   augaauuuua aaagauguaa uuuaaaggua cuuuaaagug auauuugugc ccuuguaacu 697
   gauucaaagu agacugcuaa ggacaauaag uaauaagagu cuacaaauau caguaaauua 757
   ugagaaaaaa cggucuaaug guacuuaaaa uugcauugug auuuauuaaa uaacauugaa 817
   cagcuccacc uguaacauua agauuccauu ugagcauaua gaaaauauau uuaucgcaac 877
   cugaagugau uuggaaagua ugcugggaag aaaauauauu uacaaauuag ucucagaggg 937
   uagguauccu guagccagau ggaauaagaa gagagggaag ggagauuuug uucaaagggg 997
   gagcauaagc uaaggaauaa agccacaaac aaugauuaaa uuagguaaag ugcaauuagc 1057
   ugaauauugc auaagcauac auaugggaag aaguggaaaa aggagugaca auaugaagcu 1117
   aaaggguuug aaguguucaa gauaauacua ggcuuccuag ccuauuccga gacaucuugu 1177
   uuuagagugc aauagauaag aacucuaagg augaaaguag gagacaugca uucuagucua 1237
   cguuuugguu aauggacuga aagugaaaga uggacugaaa uuggggaaug gaauaaaaau 1297
   gugauagaau agaaaauaga aaaagcaauu agccuaaugu cauugauacu uagagaaaau 1357
   auggaaaaua guaaauuagu gauauauauc uuuucuaauu caccagggug agugaacaaa 1417
   ggaaaucgga uuuaaaagua uucaugacau agaugcaaua agguuu 1463

<210> 7
<211> 100
<212> PRT
<213> Homo sapiens

<400> 7

Met Gln Leu Glu Lys Leu Val Val Leu Pro Gly Val Asp Trp Lys Cys
1               5                   10                  15

Val Leu Pro Phe Lys Gln Ser Ser Ser Thr Cys Arg Thr Asp Gly Asn
            20                  25                  30

Pro Leu Gly Lys Leu Ala Ser Asn Val Cys Leu His Ser Pro His Thr
        35                  40                  45

Gly Phe Leu Thr Cys Asp Glu Gly Asn Phe Pro Gly Leu Val Trp Val
    50                  55                  60

Cys Pro Gln Trp Thr Gly Ala Gln Asn Ala His Trp Arg Lys Trp Met
65                  70                  75                  80

Glu Pro Arg Gly Cys His Ala Leu Cys Arg Gly Gly Ala Cys Ser Leu
                85                  90                  95

Gln Leu Leu Trp
            100

<210> 8
<211> 1535
<212> RNA
<213> Homo sapiens

<220>
<221> mRNA
<222> (1)..(1535)
<223> mRNA encoding CLA2.4

<220>
<221> 5'UTR
<222> (1)..(526)
<223>

<220>
<221> CDS
<222> (527)..(628)
<223>

<220>
<221> 3'UTR
<222> (629)..(1535)
<223>

<400> 8
guggugaccu gggaaucaau gugugaggug gugcuaugua gcaggaaccc cucuugcuuu 60

gcaaauaguu uuuuguuugu uuuccuuuuu gcccaauaga gcccugcucu acugacccuu 120

caaugugccc gugugccuaa auauuccugg ucgugugaaa agaacccagg uauuagcuga 180

acuaaggagc acaauucugc aacauuuugg cacccaaaca cggggcuuga gaaaugaaug 240

caauuggaga aacugguugu uuuaccaggc guugauugga aaugugugcu ucccuuuaag 300

cagucaagcu caacuugcag aacugauggg aaccccuugg gaaaacuggc cucaaauguu 360

ugucuacaca guccacauac aggguucuua accugcgacu gacccugcuu gugccuguga 420

accaaccaac aaucucuggc ugcagcucag aaaggacaaa agagaaugga ugagggaaac 480

uucccagggc uugucugggu augcccacag uggacuggag cccaaa aug cac acu 535
Met His Thr
1

gga gga agu gga ugg agc cac gug gau guc aug ccu uau gca ggg gag 583
Gly Gly Ser Gly Trp Ser His Val Asp Val Met Pro Tyr Ala Gly Glu
    5                   10                  15

gag ccu gcu cuc uuc agc ucc ugu ggu aau gug gga auc gau cug 628

Glu Pro Ala Leu Met Ser Ser Cys Gly Asn Val Gly Ile Asp Leu
20                  25                  30

ugaggaaaca cuuucugaaa augucagaac ucuugaaaac auggaugagg aaaagcauaa    688

guacugcuuu augcuuuucu uaugaauuuu aaaagaugua auuuaaaggu acuuuaaagu    748

gauauuugug cccuuguaac ugauucaaag uagacugcua aggacaauaa guaauaagag    808

ucuacaaaua ucaguaaauu augagaaaaa acggucuaau gguacuuaaa auugcauugu    868

gauuuauuaa auaacauuga acagcuccac cuguaacauu aagauuccau uugagcauau    928

agaaaauaua uuuaucgcaa ccugaaguga uuuggaaagu augcugggaa gaaaauauau    988

uuacaaauua gucucagagg guagguaucc uguagccaga uggaauaaga agagagggaa   1048

gggagauuuu guucaaaggg ggagcauaag cuaaggaaua aagccacaaa caaugauuaa   1108

auuagguaaa gugcaauuag cugaauauug cauaagcaua cauaugggaa gaaguggaaa   1168

aaggagugac aauaugaagc uaaaggguuu gaaguguuca agauaauacu aggcuuccua   1228

gccuauuccg agacaucuug uuuuagagug caauagauaa gaacucuaag gaugaaagua   1288

ggagacaugc auucuagucu acguuuuggu uaauggacug aaagugaaag auggacugaa   1348

auugggggau ggaauaaaaa ugugauagaa uagaaaauag aaaaagcaau uagccuaaug   1408

ucauugauac uuagagaaaa uauggaaaau aguaaauuag ugauauauau cuuuucuaau   1468

ucaccagggu gagugaacaa aggaaaucgg auuuaaaagu auucaugaca uagaugcaau   1528

aagguuu                                                             1535

<210>  9
<211>  34
<212>  PRT
<213>  Homo sapiens

<400>  9

Met His Thr Gly Gly Ser Gly Trp Ser His Val Asp Val Met Pro Tyr
1               5                   10                  15

Ala Gly Glu Glu Pro Ala Leu Met Ser Ser Cys Gly Asn Val Gly Ile
            20                  25                  30

Asp Leu

<210>  10
<211>  1535

<212>  RNA
<213>  Homo sapiens

<220>
<221>  mRNA
<222>  (1)..(1535)
<223>  mRNA encoding CLA2.5


<220>
<221>  5'UTR
<222>  (1)..(668)
<223>


<220>
<221>  CDS
<222>  (669)..(710)
<223>


<220>
<221>  3'UTR
<222>  (711)..(1535)
<223>


<400>  10
guggugaccu gggaaucaau gugugaggug gugcuaugua gcaggaaccc cucuugcuuu      60

gcaaauaguu uuuuguuugu uuuccuuuuu gcccaauaga gcccugcucu acugacccuu     120

caaugugccc gugugccuaa auauuccugg ucgugugaaa agaacccagg uauuagcuga     180

acuaaggagc acaauucugc aacauuuugg cacccaaaca cggggcuuga gaaaugaaug     240

caauuggaga aacugguugu uuuaccaggc guugauugga aaugugugcu ucccuuuaag     300

cagucaagcu caacuugcag aacugauggg aacccccugg gaaaacuggc cucaaauguu     360

ugucuacaca guccacauac aggguucuua accugcgacu gacccugcuu gugccuguga     420

accaaccaac aaucucuggc ugcagcucag aaaggacaaa agagaaugga ugagggaaac     480

uucccagggc uugucugggu augcccacag uggacuggag cccaaaaugc acacuggagg     540

aaguggaugg agccacgugg augucaugcc uuaugcaggg gaggagccug cucucuucag     600

cuccuguggu aaugugggaa ucgaucugug aggaaacacu uucugaaaau gucagaacuc     660

uugaaaac aug gau gag gaa aag cau aag uac ugc uuu aug cuu uuc uua     710
         Met Asp Glu Glu Lys His Lys Tyr Cys Phe Met Leu Phe Leu
         1               5                   10

ugaauuuuaa aagauguaau uuaaagguac uuuaaaguga uauuugugcc cuuguaacug     770

auucaaagua gacugcuaag gacaauaagu aauaagaguc uacaaauauc aguaaauuau     830

gagaaaaaac ggucuaaugg uacuuaaaau ugcauuguga uuuauuaaau aacauugaac 890

agcuccaccu guaacauuaa gauuccauuu gagcauauag aaaauauauu uaucgcaacc 950

ugaagugauu uggaaaguau gcugggaaga aaauauauuu acaaauuagu cucagagggu 1010

agguauccug uagccagaug gaauaagaag agagggaagg gagauuuugu ucaaaggggg 1070

agcauaagcu aaggaauaaa gccacaaaca augauuaaau uagguaaagu gcaauuagcu 1130

gaauauugca uaagcauaca uaugggaaga aguggaaaaa ggagugacaa uaugaagcua 1190

aaggguuuga aguguucaag auaauacuag gcuuccuagc cuauuccgag acaucuuguu 1250

uuagagugca auagauaaga acucuaagga ugaaaguagg agacaugcau ucuagucuac 1310

guuuugguua auggacugaa agugaaagau ggacugaaau ugggggaugg aauaaaaaug 1370

ugauagaaua gaaaauagaa aaagcaauua gccuaauguc auugauacuu agagaaaaua 1430

uggaaaauag uaaauuagug auauauaucu uuucuaauuc accaggguga gugaacaaag 1490

gaaaucggau uuaaaaguau ucaugacaua gaugcaauaa gguuu 1535

<210> 11
<211> 14
<212> PRT
<213> Homo sapiens

<400> 11

Met Asp Glu Glu Lys His Lys Tyr Cys Phe Met Leu Phe Leu
1               5                   10

<210> 12
<211> 1535
<212> RNA
<213> Homo sapiens

<220>
<221> mRNA
<222> (1)..(1535)
<223> mRNA encoding CLA2.6

<220>
<221> 5'UTR
<222> (1)..(1181)
<223>

<220>
<221> CDS
<222> (1182)..(1211)

<223>

<220>
<221> 3'UTR
<222> (1212)..(1535)
<223>

<400> 12
guggugaccu gggaaucaau gugugaggug gugcuaugua gcaggaaccc cucuugcuuu 60

gcaaauaguu uuuuguuugu uuuccuuuuu gcccaauaga gcccugcucu acugacccuu 120

caaugugccc gugugccuaa auauuccugg ucgugugaaa agaacccagg uauuagcuga 180

acuaaggagc acaauucugc aacauuuugg cacccaaaca cggggcuuga gaaaugaaug 240

caauuggaga aacugguugu uuuaccaggc guugauugga aaugugugcu ucccuuuaag 300

cagucaagcu caacuugcag aacugauggg aaccccuugg gaaaacuggc cucaaauguu 360

ugucuacaca guccacauac aggguucuua accugcgacu gacccugcuu gugccuguga 420

accaaccaac aaucucuggc ugcagcucag aaaggacaaa agagaaugga ugagggaaac 480

uucccagggc uugucugggu augcccacag uggacuggag cccaaaaugc acacuggagg 540

aaguggaugg agccacgugg augucaugcc uuaugcaggg gaggagccug cucucuucag 600

cuccuguggu aaugugggaa ucgaucugug aggaaacacu uucugaaaau gucagaacuc 660

uugaaaacau ggaugaggaa aagcauaagu acugcuuuau gcuuuucuua ugaauuuuaa 720

aagauguaau uuaaagguac uuuaaaguga uauuugugcc cuuguaacug auucaaagua 780

gacugcuaag gacaauaagu aauaagaguc uacaaauauc aguaaauuau gagaaaaaac 840

ggucuaaugg uacuuaaaau ugcauuguga uuuauuaaau aacauugaac agcuccaccu 900

guaacauuaa gauuccauuu gagcauauag aaaauauauu uaucgcaacc ugaagugauu 960

uggaaaguau gcugggaaga aaauauauuu acaaauuagu cucagagggu agguauccug 1020

uagccagaug gaauaagaag agagggaagg gagauuuugu ucaaaggggg agcauaagcu 1080

aaggaauaaa gccacaaaca augauuaaau uagguaaagu gcaauuagcu gaauauugca 1140

uaagcauaca uaugggaaga aguggaaaaa ggagugacaa u aug aag cua aag ggu 1196
Met Lys Leu Lys Gly
1               5

uug aag ugu uca aga uaauacuagg cuuccuagcc uauuccgaga caucuuguuu 1251
Leu Lys Cys Ser Arg
                10

uagagugcaa uagauaagaa cucuaaggau gaaaguagga gacaugcauu cuagucuacg 1311

uuuugguuaa uggacugaaa gugaaagaug gacugaaauu gggggaugga auaaaaaugu 1371

gauagaauag aaaauagaaa aagcaauuag ccuaauguca uugauacuua gagaaaauau 1431

ggaaaauagu aaauuaguga uauauaucuu uucuaauuca ccagggugag ugaacaaagg 1491

aaaucggauu uaaaaguauu caugacauag augcaauaag guuu 1535

<210> 13
<211> 10
<212> PRT
<213> Homo sapiens

<400> 13

Met Lys Leu Lys Gly Leu Lys Cys Ser Arg
1               5                   10

<210> 14
<211> 1535
<212> RNA
<213> Homo sapiens

<220>
<221> mRNA
<222> (1)..(1535)
<223> mRNA encoding CLA2.7

<220>
<221> 5'UTR
<222> (1)..(1294)
<223>

<220>
<221> CDS
<222> (1295)..(1318)
<223>

<220>
<221> 3'UTR
<222> (1319)..(1535)
<223>

<400> 14
guggugaccu gggaaucaau gugugaggug gugcuaugua gcaggaaccc cucuugcuuu 60

gcaaauaguu uuuuguuugu uuuccuuuuu gcccaauaga gcccugcucu acugacccuu 120

caaugugccc gugugccuaa auauuccugg ucgugugaaa agaacccagg uauuagcuga 180

acuaaggagc acaauucugc aacauuuugg cacccaaaca cggggcuuga gaaaugaaug 240

caauuggaga aacugguugu uuuaccaggc guugauugga aaugugugcu ucccuuuaag 300

cagucaagcu caacuugcag aacugauggg aaccccuugg gaaaacuggc cucaaauguu 360

ugucuacaca guccacauac aggguucuua accugcgacu gacccugcuu gugccuguga 420

accaaccaac aaucucuggc ugcagcucag aaaggacaaa agagaaugga ugagggaaac 480

uucccagggc uugucugggu augcccacag uggacuggag cccaaaaugc acacuggagg 540

aaguggaugg agccacgugg augucaugcc uuaugcaggg gaggagccug cucucuucag 600

cuccuguggu aaugugggaa ucgaucugug aggaaacacu uucugaaaau gucagaacuc 660

uugaaaacau ggaugaggaa aagcauaagu acugcuuuau gcuuuucuua ugaauuuuaa 720

aagauguaau uuaaagguac uuuaaaguga uauuugugcc cuuguaacug auucaaagua 780

gacugcuaag gacaauaagu aauaagaguc uacaaauauc aguaaauuau gagaaaaaac 840

ggucuaaugg uacuuaaaau ugcauuguga uuuauuaaau aacauugaac agcuccaccu 900

guaacauuaa gauuccauuu gagcauauag aaaauauauu uaucgcaacc ugaagugauu 960

uggaaaguau gcugggaaga aaauauauuu acaaauuagu cucagagggu agguauccug 1020

uagccagaug gaauaagaag agagggaagg gagauuuugu ucaaaggggg agcauaagcu 1080

aaggaauaaa gccacaaaca augauuaaau uagguaaagu gcaauuagcu gaauauugca 1140

uaagcauaca uaugggaaga aguggaaaaa ggagugacaa uaugaagcua aagguuuuga 1200

aguguucaag auaauacuag gcuuccuagc cuauuccgag acaucuuguu uuagagugca 1260

auagauaaga acucuaagga ugaaaguagg agac aug cau ucu agu cua cgu uuu 1315
Met His Ser Ser Leu Arg Phe
1               5

ggu uaauggacug aaagugaaag auggacugaa auuggggaau ggaauaaaaa 1368
Gly

ugugauagaa uagaaaauag aaaaagcaau uagccuaaug ucauugauac uuagagaaaa 1428

uauggaaaau aguaaauuag ugauauauau cuuuucuaau ucaccagggu gagugaacaa 1488

aggaaaucgg auuuaaaagu auucaugaca uagaugcaau aagguuu 1535

<210> 15
<211> 8
<212> PRT
<213> Homo sapiens

<400> 15

Met His Ser Ser Leu Arg Phe Gly
1               5

<210>  16
<211>  1535
<212>  RNA
<213>  Homo sapiens

<220>
<221>  mRNA
<222>  (1)..(1535)
<223>  mRNA encoding CLA2.8

<220>
<221>  5'UTR
<222>  (1)..(1429)
<223>

<220>
<221>  CDS
<222>  (1430)..(1477)
<223>

<220>
<221>  3'UTR
<222>  (1478)..(1535)
<223>

<400>  16
guggugaccu gggaaucaau gugugaggug gugcuaugua gcaggaaccc cucuugcuuu      60

gcaaauaguu uuuuguuugu uuuccuuuuu gcccaauaga gcccugcucu acugacccuu     120

caaugugccc gugugccuaa auauuccugg ucgugugaaa agaacccagg uauuagcuga     180

acuaaggagc acaauucugc aacauuuugg cacccaaaca cggggcuuga gaaaugaaug     240

caauuggaga aacugguugu uuuaccaggc guugauugga aaugugugcu ucccuuuaag     300

cagucaagcu caacuugcag aacugauggg aaccccuugg gaaaacuggc cucaaauguu     360

ugucuacaca guccacauac aggguucuua accugcgacu gacccugcuu gugccuguga     420

accaaccaac aaucucuggc ugcagcucag aaaggacaaa agagaaugga ugagggaaac     480

uucccagggc uugucugggu augcccacag uggacuggag cccaaaaugc acacuggagg     540

aaguggaugg agccacgugg augucaugcc uuaugcaggg gaggagccug cucucuucag     600

cuccuguggu aaugugggaa ucgaucugug aggaaacacu uucugaaaau gucagaacuc     660

uugaaaacau ggaugaggaa aagcauaagu acugcuuuau gcuuuucuua ugaauuuuaa 720

aagauguaau uuaaagguac uuuaaaguga uauuugugcc cuuguaacug auucaaagua 780

gacugcuaag gacaauaagu aauaagaguc uacaaauauc aguaaauuau gagaaaaaac 840

ggucuaaugg uacuuaaaau ugcauuguga uuuauuaaau aacauugaac agcuccaccu 900

guaacauuaa gauuccauuu gagcauauag aaaauauauu uaucgcaacc ugaagugauu 960

uggaaaguau gcugggaaga aaauauauuu acaaauuagu cucagagggu agguauccug 1020

uagccagaug gaauaagaag agagggaagg gagauuuugu ucaaaggggg agcauaagcu 1080

aaggaauaaa gccacaaaca augauuaaau uagguaaagu gcaauuagcu gaauauugca 1140

uaagcauaca uaugggaaga aguggaaaaa ggagugacaa uaugaagcua aaggguuuga 1200

aguguucaag auaauacuag gcuuccuagc cuauuccgag acaucuuguu uuagagugca 1260

auagauaaga acucuaagga ugaaaguagg agacaugcau ucuagucuac guuuugguua 1320

auggacugaa agugaaagau ggacugaaau ugggggaugg aauaaaaaug ugauagaaua 1380

gaaaauagaa aaagcaauua gccuaauguc auugauacuu agagaaaau aug gaa aau 1438
Met Glu Asn
1

agu aaa uua gug aua uau auc uuu ucu aau uca cca ggg ugagugaaca 1487
Ser Lys Leu Val Ile Tyr Ile Phe Ser Asn Ser Pro Gly
    5                   10                  15

aaggaaaucg gauuuaaaag uauucaugac auagaugcaa uaagguuu 1535

<210> 17
<211> 16
<212> PRT
<213> Homo sapiens

<400> 17

Met Glu Asn Ser Lys Leu Val Ile Tyr Ile Phe Ser Asn Ser Pro Gly
1               5                   10                  15

<210> 18
<211> 22
<212> PRT
<213> Artificial Sequence

<220>
<223> Description of the artificial Sequence: Immunogenic Peptide

<400> 18

Glu Glu Thr Leu Ser Glu Asn Val Arg Thr Leu Glu Asn Met Asp Glu

1               5                   10                  15

Glu Lys His Lys Tyr Cys
            20

<210>  19
<211>  17
<212>  PRT
<213>  Artificial Sequence

<220>
<223>  Description of the artificial Sequence: Immunogenic Peptide

<400>  19

Val Asp Trp Ser Pro Lys Cys Thr Leu Glu Glu Val Asp Gly Ala Thr
1               5                   10                  15

Trp

<210>  20
<211>  20
<212>  PRT
<213>  Artificial Sequence

<220>
<223>  Description of the artificial Sequence: Immunogenic Peptide

<400>  20

Leu Met Gln Gly Arg Ser Leu Leu Ser Ser Ala Pro Val Val Met Trp
1               5                   10                  15

Glu Ser Ile Cys
            20

<210>  21
<211>  18
<212>  PRT
<213>  Artificial Sequence

<220>
<223>  Description of the artificial Sequence: Immunogenic Peptide

<400>  21

Ser Ile Cys Glu Glu Thr Leu Ser Glu Asn Val Arg Thr Leu Glu Asn
1               5                   10                  15

Met Asp

<210> 22
<211> 12
<212> PRT
<213> Artificial Sequence

<220>
<223> Description of the artificial Sequence: Immunogenic Peptide

<400> 22

Glu Glu Lys His Lys Tyr Cys Phe Met Leu Phe Leu
1               5                   10

<210> 23
<211> 15
<212> PRT
<213> Artificial Sequence

<220>
<223> Description of the artificial Sequence: Immunogenic Peptide

<400> 23

Arg Thr Asp Gly Asn Pro Leu Gly Lys Leu Ala Ser Asn Val Cys
1               5                   10                  15

<210> 24
<211> 16
<212> PRT
<213> Artificial Sequence

<220>
<223> Description of the artificial Sequence: Immunogenic Peptide

<400> 24

Met Gln Leu Glu Lys Leu Val Val Leu Pro Gly Val Asp Trp Lys Cys
1               5                   10                  15

<210> 25
<211> 15
<212> PRT
<213> Artificial Sequence

<220>
<223> Description of the artificial Sequence: Immunogenic Peptide

<400> 25

Val Leu Pro Phe Lys Gln Ser Ser Ser Thr Cys Arg Thr Asp Gly

1               5                   10                  15


<210>  26
<211>  18
<212>  PRT
<213>  Artificial Sequence

<220>
<223>  Description of the artificial Sequence: Immunogenic Peptide

<400>  26

Asn Pro Leu Gly Lys Leu Ala Ser Asn Val Cys Leu His Ser Pro His
1               5                   10                  15


Thr Gly




<210>  27
<211>  15
<212>  PRT
<213>  Artificial Sequence

<220>
<223>  Description of the artificial Sequence: Immunogenic Peptide

<400>  27

Phe Leu Thr Cys Asp Glu Gly Asn Phe Pro Gly Leu Val Trp Val
1               5                   10                  15


<210>  28
<211>  16
<212>  PRT
<213>  Artificial Sequence

<220>
<223>  Description of the artificial Sequence: Immunogenic Peptide

<400>  28

Cys His Ala Leu Cys Arg Gly Gly Ala Cys Ser Leu Gln Leu Leu Trp
1               5                   10                  15


<210>  29
<211>  15
<212>  PRT
<213>  Artificial Sequence

<220>
<223>  Description of the artificial Sequence: Immunogenic Peptide

<400>  29

Ala Gln Asn Ala His Trp Arg Lys Trp Met Glu Pro Arg Gly Cys
1               5                   10                  15


<210>  30
<211>  20
<212>  DNA
<213>  Artificial Sequence

<220>
<223>  Description of the artificial Sequence: Primer

<400>  30
ccacaggagc tgaagagagc                                                   20


<210>  31
<211>  19
<212>  DNA
<213>  Artificial Sequence

<220>
<223>  Description of the artificial Sequence: Primer

<400>  31
agggcttgtc tgggtatgc                                                    19


<210>  32
<211>  29
<212>  DNA
<213>  Artificial Sequence

<220>
<223>  Description of the artificial Sequence: Primer

<400>  32
gaggccagtt ttcccaaggg gttcccatc                                         29


<210>  33
<211>  28
<212>  DNA
<213>  Artificial Sequence

<220>
<223>  Description of the artificial Sequence: Primer

<400>  33
cacgggcaca ttgaagggtc agtagagc                                          28


<210>  34
<211>  19
<212>  DNA

<213>  Artificial Sequence

<220>
<223>  Description of the artificial Sequence: Primer

<400>  34
ccatccccca atttcagtc                                                    19


<210>  35
<211>  22
<212>  DNA
<213>  Artificial Sequence

<220>
<223>  Description of the artificial Sequence: Primer

<400>  35
gtgtgaggtg gtgctatgta gc                                                22

<210>  36
<211>  19
<212>  DNA
<213>  Artificial Sequence

<220>
<223>  Description of the artificial Sequence: Primer

<400>  36
ccatccccca atttcagtc                                                    19


<210>  37
<211>  22
<212>  DNA
<213>  Artificial Sequence

<220>
<223>  Description of the artificial Sequence: Primer

<400>  37
gtgtgaggtg gtgctatgta gc                                                22


<210>  38
<211>  24
<212>  DNA
<213>  Artificial Sequence

<220>
<223>  Description of the artificial Sequence: Primer

<400>  38
atgcaattgg agaaactggt tgtt                                              24

<210>  39
<211>  24
<212>  DNA
<213>  Artificial Sequence

<220>
<223>  Description of the artificial Sequence: Primer

<400>  39
gtcgcaggtt aagaaccctg tatg                                              24


<210>  40
<211>  25
<212>  DNA
<213>  Artificial Sequence

<220>
<223>  Description of the artificial Sequence: Primer

<400>  40
atgcccacag tggactggag cccaa                                             25


<210>  41
<211>  28
<212>  DNA
<213>  Artificial Sequence

<220>
<223>  Description of the artificial Sequence: Primer

<400>  41
taagaaaagc ataaagcagt acttatgc                                          28


<210>  42
<211>  24
<212>  DNA
<213>  Artificial Sequence

<220>
<223>  Description of the artificial Sequence: Primer

<400>  42
atgcaattgg agaaactggt tgtt                                              24


<210>  43
<211>  21
<212>  DNA
<213>  Artificial Sequence

<220>
<223>  Description of the artificial Sequence: Primer

<400>  43

ccacaggagc tgaagagagc a                                                21

<210>  44
<211>  21
<212>  DNA
<213>  Artificial Sequence

<220>
<223>  Description of the artificial Sequence: Primer

<400>  44
ggcactcgac cggaattatc g                                                21

<210>  45
<211>  10
<212>  PRT
<213>  Artificial Sequence

<220>
<223>  Description of the artificial Sequence: tryptic peptide

<400>  45

Leu Val Val Leu Pro Gly Val Asp Trp Lys
1               5                   10

<210>  46
<211>  8
<212>  PRT
<213>  Artificial Sequence

<220>
<223>  Description of the artificial Sequence: tryptic peptide

<400>  46

Thr Asp Gly Asn Pro Leu Gly Lys
1               5

<210>  47
<211>  18
<212>  PRT
<213>  Artificial Sequence

<220>
<223>  Description of the artificial Sequence: tryptic peptide

<400>  47

Leu Ala Ser Asn Val Cys Leu His Ser Pro His Thr Gly Phe Leu Thr
1               5                   10                  15

Cys Asp

<210> 48
<211> 8
<212> PRT
<213> Artificial Sequence

<220>
<223> Description of the artificial Sequence: chymotryptic peptide

<400> 48

Val Val Leu Pro Gly Val Asp Trp
1               5

<210> 49
<211> 14
<212> PRT
<213> Artificial Sequence

<220>
<223> Description of the artificial Sequence: chymotryptic peptide

<400> 49

Lys Gln Ser Ser Ser Thr Cys Arg Thr Asp Gly Asn Pro Leu
1               5                   10

<210> 50
<211> 17
<212> PRT
<213> Artificial Sequence

<220>
<223> Description of the artificial Sequence: elastatic peptide

<400> 50

Leu Pro Phe Lys Gln Ser Ser Ser Thr Cys Arg Thr Asp Gly Asn Pro
1               5                   10                  15

Leu

<210> 51
<211> 8
<212> PRT
<213> Artificial Sequence

<220>
<223> Description of the artificial Sequence: elastatic peptide

<400>  51

His Ser Pro His Thr Gly Phe Leu
1               5


<210>  52
<211>  7
<212>  PRT
<213>  Artificial Sequence

<220>
<223>  Description of the artificial Sequence: peptide resulting from co
       mbined tryptic/chymotryptic and elastatic digestion

<400>  52

Gln Ser Ser Ser Thr Cys Arg
1               5


<210>  53
<211>  7
<212>  PRT
<213>  Artificial Sequence

<220>
<223>  Description of the artificial Sequence: peptide resulting from co
       mbined tryptic/chymotryptic and elastatic digestion

<400>  53

His Ser Pro His Thr Gly Phe
1               5


<210>  54
<211>  9
<212>  PRT
<213>  Artificial Sequence

<220>
<223>  Description of the artificial Sequence: tryptic peptide

<400>  54

Met Pro Thr Val Asp Trp Ser Pro Lys
1               5


<210>  55
<211>  19
<212>  PRT
<213>  Artificial Sequence

<220>

<223>  Description of the artificial Sequence: tryptic peptide

<400>  55

Cys Thr Leu Glu Glu Val Asp Gly Ala Thr Trp Met Ser Cys Leu Met
1               5                   10                  15

Gln Gly Arg

<210>  56
<211>  24
<212>  PRT
<213>  Artificial Sequence

<220>
<223>  Description of the artificial Sequence: tryptic peptide

<400>  56

Ser Leu Leu Ser Ser Ala Pro Val Val Met Trp Glu Ser Ile Cys Glu
1               5                   10                  15

Glu Thr Leu Ser Glu Asn Val Arg
            20

<210>  57
<211>  9
<212>  PRT
<213>  Artificial Sequence

<220>
<223>  Description of the artificial Sequence: tryptic peptide

<400>  57

Thr Leu Glu Asn Met Asp Glu Glu Lys
1               5

<210>  58
<211>  7
<212>  PRT
<213>  Artificial Sequence

<220>
<223>  Description of the artificial Sequence: tryptic peptide

<400>  58

Tyr Cys Phe Met Leu Phe Leu
1               5

<210> 59
<211> 8
<212> PRT
<213> Artificial Sequence

<220>
<223> Description of the artificial Sequence: chymotryptic peptide

<400> 59

Glu Glu Val Asp Gly Ala Thr Trp
1               5


<210> 60
<211> 8
<212> PRT
<213> Artificial Sequence

<220>
<223> Description of the artificial Sequence: chymotryptic peptide

<400> 60

Ser Ser Ala Pro Val Val Met Trp
1               5


<210> 61
<211> 8
<212> PRT
<213> Artificial Sequence

<220>
<223> Description of the artificial Sequence: chymotryptic peptide

<400> 61

Glu Ser Ile Cys Glu Glu Thr Leu
1               5


<210> 62
<211> 10
<212> PRT
<213> Artificial Sequence

<220>
<223> Description of the artificial Sequence: chymotryptic peptide

<400> 62

Glu Asn Met Asp Glu Glu Lys His Lys Tyr
1               5                   10

<210>  63
<211>  8
<212>  PRT
<213>  Artificial Sequence

<220>
<223>  Description of the artificial Sequence: elastatic peptide

<400>  63

Asp Trp Ser Pro Lys Cys Thr Leu
1               5


<210>  64
<211>  14
<212>  PRT
<213>  Artificial Sequence

<220>
<223>  Description of the artificial Sequence: elastatic peptide

<400>  64

Glu Asn Met Asp Glu Glu Lys His Lys Tyr Cys Phe Met Leu
1               5                   10


<210>  65
<211>  7
<212>  PRT
<213>  Artificial Sequence

<220>
<223>  Description of the artificial Sequence: peptide resulting from co
       mbined tryptic/chymotryptic and elastatic digestion

<400>  65

Glu Asn Met Asp Glu Glu Lys
1               5


<210>  66
<211>  10
<212>  PRT
<213>  Artificial Sequence

<220>
<223>  Description of the artificial Sequence: tryptic peptide

<400>  66

Leu Val Val Leu Pro Gly Val Asp Trp Lys
1               5                   10

<210> 67
<211> 8
<212> PRT
<213> Artificial Sequence

<220>
<223> Description of the artificial Sequence: tryptic peptide

<400> 67

Thr Asp Gly Asn Pro Leu Gly Lys
1               5


<210> 68
<211> 10
<212> PRT
<213> Artificial Sequence

<220>
<223> Description of the artificial Sequence: tryptic peptide

<400> 68

Gly Gly Ala Cys Ser Leu Gln Leu Leu Trp
1               5                   10


<210> 69
<211> 8
<212> PRT
<213> Artificial Sequence

<220>
<223> Description of the artificial Sequence: chymotryptic peptide

<400> 69

Val Val Leu Pro Gly Val Asp Trp
1               5


<210> 70
<211> 14
<212> PRT
<213> Artificial Sequence

<220>
<223> Description of the artificial Sequence: chymotryptic peptide

<400> 70

Lys Gln Ser Ser Ser Thr Cys Arg Thr Asp Gly Asn Pro Leu
1               5                   10


<210> 71

<211>  10
<212>  PRT
<213>  Artificial Sequence

<220>
<223>  Description of the artificial Sequence: chymotryptic peptide

<400>  71

Thr Cys Asp Glu Gly Asn Phe Pro Gly Leu
1               5                   10


<210>  72
<211>  8
<212>  PRT
<213>  Artificial Sequence

<220>
<223>  Description of the artificial Sequence: chymotryptic peptide

<400>  72

Thr Gly Ala Gln Asn Ala His Trp
1               5


<210>  73
<211>  9
<212>  PRT
<213>  Artificial Sequence

<220>
<223>  Description of the artificial Sequence: chymotryptic peptide

<400>  73

Met Glu Pro Arg Gly Cys His Ala Leu
1               5


<210>  74
<211>  8
<212>  PRT
<213>  Artificial Sequence

<220>
<223>  Description of the artificial Sequence: chymotryptic peptide

<400>  74

Cys Arg Gly Gly Ala Cys Ser Leu
1               5


<210>  75
<211>  17

<212>  PRT
<213>  Artificial Sequence

<220>
<223>  Description of the artificial Sequence: elastatic peptide

<400>  75

Leu Pro Phe Lys Gln Ser Ser Ser Thr Cys Arg Thr Asp Gly Asn Pro
1               5                   10                  15

Leu

<210>  76
<211>  8
<212>  PRT
<213>  Artificial Sequence

<220>
<223>  Description of the artificial Sequence: elastatic peptide

<400>  76

His Ser Pro His Thr Gly Phe Leu
1               5

<210>  77
<211>  10
<212>  PRT
<213>  Artificial Sequence

<220>
<223>  Description of the artificial Sequence: elastatic peptide

<400>  77

Thr Cys Asp Glu Gly Asn Phe Pro Gly Leu
1               5                   10

<210>  78
<211>  13
<212>  PRT
<213>  Artificial Sequence

<220>
<223>  Description of the artificial Sequence: elastatic peptide

<400>  78

His Trp Arg Lys Trp Met Glu Pro Arg Gly Cys His Ala
1               5                   10

<210>  79
<211>  7
<212>  PRT
<213>  Artificial Sequence

<220>
<223>  Description of the artificial Sequence: peptide resulting from co
       mbined tryptic/chymotryptic and elastatic digestion

<400>  79

Gln Ser Ser Ser Thr Cys Arg
1               5


<210>  80
<211>  7
<212>  PRT
<213>  Artificial Sequence

<220>
<223>  Description of the artificial Sequence: peptide resulting from co
       mbined tryptic/chymotryptic and elastatic digestion

<400>  80

His Ser Pro His Thr Gly Phe
1               5


<210>  81
<211>  10
<212>  PRT
<213>  Artificial Sequence

<220>
<223>  Description of the artificial Sequence: peptide resulting from co
       mbined tryptic/chymotryptic and elastatic digestion

<400>  81

Thr Cys Asp Glu Gly Asn Phe Pro Gly Leu
1               5                   10


<210>  82
<211>  8
<212>  PRT
<213>  Artificial Sequence

<220>
<223>  Description of the artificial Sequence: chymotryptic peptide

<400>  82

Met His Thr Gly Gly Ser Gly Trp

1                   5


<210>  83
<211>  8
<212>  PRT
<213>  Artificial Sequence

<220>
<223>  Description of the artificial Sequence: chymotryptic peptide

<400>  83

Ser His Val Asp Val Met Pro Tyr
1               5


<210>  84
<211>  10
<212>  PRT
<213>  Artificial Sequence

<220>
<223>  Description of the artificial Sequence: chymotryptic peptide

<400>  84

Ser Ser Cys Gly Asn Val Gly Ile Asp Leu
1               5                   10


<210>  85
<211>  11
<212>  PRT
<213>  Artificial Sequence

<220>
<223>  Description of the artificial Sequence: elastatic peptide

<400>  85

Met His Thr Gly Gly Ser Gly Trp Ser His Val
1               5                   10


<210>  86
<211>  8
<212>  PRT
<213>  Artificial Sequence

<220>
<223>  Description of the artificial Sequence: peptide resulting from co
       mbined tryptic/chymotryptic and elastatic digestion

<400>  86

Met His Thr Gly Gly Ser Gly Trp

1               5


<210>  87
<211>  11
<212>  PRT
<213>  Artificial Sequence

<220>
<223>  Description of the artificial Sequence: tryptic peptide

<400>  87

Leu Val Ile Tyr Ile Phe Ser Asn Ser Pro Gly
1               5                   10


<210>  88
<211>  9
<212>  PRT
<213>  Artificial Sequence

<220>
<223>  Description of the artificial Sequence: HLA restricted Peptide

<400>  88

Arg Thr Asp Gly Asn Pro Leu Gly Lys
1               5


<210>  89
<211>  10
<212>  PRT
<213>  Artificial Sequence

<220>
<223>  Description of the artificial Sequence: HLA restricted Peptide

<400>  89

Arg Thr Asp Gly Asn Pro Leu Gly Lys Leu
1               5                   10


<210>  90
<211>  10
<212>  PRT
<213>  Artificial Sequence

<220>
<223>  Description of the artificial Sequence: HLA restricted Peptide

<400>  90

Gln Leu Glu Lys Leu Val Val Leu Pro Gly
1               5                   10

<210> 91
<211> 9
<212> PRT
<213> Artificial Sequence

<220>
<223> Description of the artificial Sequence: HLA restricted Peptide

<400> 91

Asn Met Asp Glu Glu Lys His Lys Tyr
1               5


<210> 92
<211> 9
<212> PRT
<213> Artificial Sequence

<220>
<223> Description of the artificial Sequence: HLA restricted Peptide

<400> 92

Leu Ser Glu Asn Val Arg Thr Leu Glu
1               5


<210> 93
<211> 10
<212> PRT
<213> Artificial Sequence

<220>
<223> Description of the artificial Sequence: HLA restricted Peptide

<400> 93

Leu Ser Glu Asn Val Arg Thr Leu Glu Asn
1               5                   10


<210> 94
<211> 10
<212> PRT
<213> Artificial Sequence

<220>
<223> Description of the artificial Sequence: HLA restricted Peptide

<400> 94

Glu Asn Met Asp Glu Glu Lys His Lys Tyr
1               5                   10

<210>  95
<211>  9
<212>  PRT
<213>  Artificial Sequence

<220>
<223>  Description of the artificial Sequence: HLA restricted Peptide

<400>  95

Arg Thr Asp Gly Asn Pro Leu Gly Lys
1               5


<210>  96
<211>  10
<212>  PRT
<213>  Artificial Sequence

<220>
<223>  Description of the artificial Sequence: HLA restricted Peptide

<400>  96

Arg Thr Asp Gly Asn Pro Leu Gly Lys Leu
1               5                   10


<210>  97
<211>  10
<212>  PRT
<213>  Artificial Sequence

<220>
<223>  Description of the artificial Sequence: HLA restricted Peptide

<400>  97

Gln Leu Glu Lys Leu Val Val Leu Pro Gly
1               5                   10


<210>  98
<211>  9
<212>  PRT
<213>  Artificial Sequence

<220>
<223>  Description of the artificial Sequence: HLA restricted Peptide

<400>  98

Trp Ser His Val Asp Val Met Pro Tyr
1               5

<210>  99
<211>  9
<212>  PRT
<213>  Artificial Sequence

<220>
<223>  Description of the artificial Sequence: HLA restricted Peptide

<400>  99

Ala Gly Glu Glu Pro Ala Leu Phe Ser
1               5


<210>  100
<211>  9
<212>  PRT
<213>  Artificial Sequence

<220>
<223>  Description of the artificial Sequence: HLA restricted Peptide

<400>  100

Met Asp Glu Glu Lys His Lys Tyr Cys
1               5


<210>  101
<211>  9
<212>  PRT
<213>  Artificial Sequence

<220>
<223>  Description of the artificial Sequence: HLA restricted Peptide

<400>  101

Met Glu Asn Ser Lys Leu Val Ile Tyr
1               5


<210>  102
<211>  9
<212>  PRT
<213>  Artificial Sequence

<220>
<223>  Description of the artificial Sequence: HLA restricted Peptide

<400>  102

Val Val Leu Pro Gly Val Asp Trp Lys
1               5


<210>  103

<211>  9
<212>  PRT
<213>  Artificial Sequence

<220>
<223>  Description of the artificial Sequence: HLA restricted Peptide

<400>  103

Arg Thr Asp Gly Asn Pro Leu Gly Lys
1               5


<210>  104
<211>  9
<212>  PRT
<213>  Artificial Sequence

<220>
<223>  Description of the artificial Sequence: HLA restricted Peptide

<400>  104

Lys Leu Val Val Leu Pro Cys Val Asp
1               5


<210>  105
<211>  9
<212>  PRT
<213>  Artificial Sequence

<220>
<223>  Description of the artificial Sequence: HLA restricted Peptide

<400>  105

Leu Val Val Leu Pro Gly Val Asp Trp
1               5


<210>  106
<211>  9
<212>  PRT
<213>  Artificial Sequence

<220>
<223>  Description of the artificial Sequence: HLA restricted Peptide

<400>  106

Lys Leu Ala Ser Asn Val Cys Leu His
1               5


<210>  107
<211>  10

<212>  PRT
<213>  Artificial Sequence

<220>
<223>  Description of the artificial Sequence: HLA restricted Peptide

<400>  107

Leu Val Val Leu Pro Gly Val Asp Trp Lys
1               5                   10


<210>  108
<211>  10
<212>  PRT
<213>  Artificial Sequence

<220>
<223>  Description of the artificial Sequence: HLA restricted Peptide

<400>  108

Lys Leu Val Val Leu Pro Gly Val Asp Trp
1               5                   10


<210>  109
<211>  10
<212>  PRT
<213>  Artificial Sequence

<220>
<223>  Description of the artificial Sequence: HLA restricted Peptide

<400>  109

Cys Val Leu Pro Phe Lys Gln Ser Ser Ser
1               5                   10


<210>  110
<211>  9
<212>  PRT
<213>  Artificial Sequence

<220>
<223>  Description of the artificial Sequence: HLA restricted Peptide

<400>  110

Thr Leu Glu Asn Met Asp Glu Glu Lys
1               5


<210>  111
<211>  9
<212>  PRT

<213>  Artificial Sequence

<220>
<223>  Description of the artificial Sequence: HLA restricted Peptide

<400>  111

Ser Leu Leu Ser Ser Ala Pro Val Val
1               5


<210>  112
<211>  10
<212>  PRT
<213>  Artificial Sequence

<220>
<223>  Description of the artificial Sequence: HLA restricted Peptide

<400>  112

Ser Leu Leu Ser Ser Ala Pro Val Val Met
1               5                   10


<210>  113
<211>  10
<212>  PRT
<213>  Artificial Sequence

<220>
<223>  Description of the artificial Sequence: HLA restricted Peptide

<400>  113

Thr Leu Glu Glu Val Asp Gly Ala Thr Trp
1               5                   10


<210>  114
<211>  9
<212>  PRT
<213>  Artificial Sequence

<220>
<223>  Description of the artificial Sequence: HLA restricted Peptide

<400>  114

Val Val Leu Pro Gly Val Asp Trp Lys
1               5


<210>  115
<211>  9
<212>  PRT
<213>  Artificial Sequence

<220>
<223>  Description of the artificial Sequence: HLA restricted Peptide

<400>  115

Ala Leu Cys Arg Gly Gly Ala Cys Ser
1               5


<210>  116
<211>  9
<212>  PRT
<213>  Artificial Sequence

<220>
<223>  Description of the artificial Sequence: HLA restricted Peptide

<400>  116

Arg Thr Asp Gly Asn Pro Leu Gly Lys
1               5


<210>  117
<211>  9
<212>  PRT
<213>  Artificial Sequence

<220>
<223>  Description of the artificial Sequence: HLA restricted Peptide

<400>  117

Lys Leu Val Val Leu Pro Gly Val Asp
1               5


<210>  118
<211>  9
<212>  PRT
<213>  Artificial Sequence

<220>
<223>  Description of the artificial Sequence: HLA restricted Peptide

<400>  118

Leu Val Val Leu Pro Gly Val Asp Trp
1               5


<210>  119
<211>  9
<212>  PRT
<213>  Artificial Sequence

<220>
<223>  Description of the artificial Sequence: HLA restricted Peptide

<400>  119

Lys Leu Ala Ser Asn Val Cys Leu His
1               5


<210>  120
<211>  10
<212>  PRT
<213>  Artificial Sequence

<220>
<223>  Description of the artificial Sequence: HLA restricted Peptide

<400>  120

Leu Val Val Leu Pro Gly Val Asp Trp Lys
1               5                   10


<210>  121
<211>  10
<212>  PRT
<213>  Artificial Sequence

<220>
<223>  Description of the artificial Sequence: HLA restricted Peptide

<400>  121

Ala Leu Cys Arg Gly Gly Ala Cys Ser Leu
1               5                   10


<210>  122
<211>  10
<212>  PRT
<213>  Artificial Sequence

<220>
<223>  Description of the artificial Sequence: HLA restricted Peptide

<400>  122

Lys Leu Val Val Leu Pro Gly Val Asp Trp
1               5                   10


<210>  123
<211>  10
<212>  PRT
<213>  Artificial Sequence

<220>

<223>  Description of the artificial Sequence: HLA restricted Peptide

<400>  123

Cys Val Leu Pro Phe Lys Gln Ser Ser Ser
1               5                   10


<210>  124
<211>  10
<212>  PRT
<213>  Artificial Sequence

<220>
<223>  Description of the artificial Sequence: HLA restricted Peptide

<400>  124

Ala Leu Phe Ser Ser Cys Gly Asn Val Gly
1               5                   10


<210>  125
<211>  9
<212>  PRT
<213>  Artificial Sequence

<220>
<223>  Description of the artificial Sequence: HLA restricted Peptide

<400>  125

Lys His Lys Tyr Cys Phe Met Leu Phe
1               5


<210>  126
<211>  9
<212>  PRT
<213>  Artificial Sequence

<220>
<223>  Description of the artificial Sequence: HLA restricted Peptide

<400>  126

Lys Leu Lys Gly Leu Lys Cys Ser Arg
1               5


<210>  127
<211>  9
<212>  PRT
<213>  Artificial Sequence

<220>
<223>  Description of the artificial Sequence: HLA restricted Peptide

<400>  127

Lys Leu Val Ile Tyr Ile Phe Ser Asn
1               5


<210>  128
<211>  9
<212>  PRT
<213>  Artificial Sequence

<220>
<223>  Description of the artificial Sequence: HLA restricted Peptide

<400>  128

Leu Val Ile Tyr Ile Phe Ser Asn Ser
1               5

<210>  129
<211>  9
<212>  PRT
<213>  Artificial Sequence

<220>
<223>  Description of the artificial Sequence: HLA restricted Peptide

<400>  129

Val Ile Tyr Ile Phe Ser Asn Ser Pro
1               5


<210>  130
<211>  9
<212>  PRT
<213>  Artificial Sequence

<220>
<223>  Description of the artificial Sequence: HLA restricted Peptide

<400>  130

Met Gln Leu Glu Lys Leu Val Val Leu
1               5


<210>  131
<211>  9
<212>  PRT
<213>  Artificial Sequence

<220>
<223>  Description of the artificial Sequence: HLA restricted Peptide

<400>  131

Pro Leu Gly Lys Leu Ala Ser Asn Val
1               5


<210>  132
<211>  10
<212>  PRT
<213>  Artificial Sequence

<220>
<223>  Description of the artificial Sequence: HLA restricted Peptide

<400>  132

Val Leu Pro Gly Val Asp Trp Lys Cys Val
1               5                   10


<210>  133
<211>  10
<212>  PRT
<213>  Artificial Sequence

<220>
<223>  Description of the artificial Sequence: HLA restricted Peptide

<400>  133

Arg Thr Asp Gly Asn Pro Leu Gly Lys Leu
1               5                   10


<210>  134
<211>  10
<212>  PRT
<213>  Artificial Sequence

<220>
<223>  Description of the artificial Sequence: HLA restricted Peptide

<400>  134

Cys Leu His Ser Pro His Thr Gly Phe Leu
1               5                   10


<210>  135
<211>  9
<212>  PRT
<213>  Artificial Sequence

<220>
<223>  Description of the artificial Sequence: HLA restricted Peptide

<400>  135

Thr Leu Ser Glu Asn Val Arg Thr Leu
1               5


<210>  136
<211>  9
<212>  PRT
<213>  Artificial Sequence

<220>
<223>  Description of the artificial Sequence: HLA restricted Peptide

<400>  136

Ser Leu Leu Ser Ser Ala Pro Val Val
1               5


<210>  137
<211>  9
<212>  PRT
<213>  Artificial Sequence

<220>
<223>  Description of the artificial Sequence: HLA restricted Peptide

<400>  137

Cys Leu Met Gln Gly Arg Ser Leu Leu
1               5


<210>  138
<211>  9
<212>  PRT
<213>  Artificial Sequence

<220>
<223>  Description of the artificial Sequence: HLA restricted Peptide

<400>  138

Leu Leu Ser Ser Ala Pro Val Val Met
1               5


<210>  139
<211>  8
<212>  PRT
<213>  Artificial Sequence

<220>
<223>  Description of the artificial Sequence: HLA restricted Peptide

<400>  139

Val Met Trp Ser Ile Cys Glu Glu
1               5


<210>  140
<211>  10
<212>  PRT
<213>  Artificial Sequence

<220>
<223>  Description of the artificial Sequence: HLA restricted Peptide

<400>  140

Ser Leu Leu Ser Ser Ala Pro Val Val Met
1               5                   10


<210>  141
<211>  10
<212>  PRT
<213>  Artificial Sequence

<220>
<223>  Description of the artificial Sequence: HLA restricted Peptide

<400>  141

Leu Met Gln Gly Arg Ser Leu Leu Ser Ser
1               5                   10


<210>  142
<211>  10
<212>  PRT
<213>  Artificial Sequence

<220>
<223>  Description of the artificial Sequence: HLA restricted Peptide

<400>  142

Glu Thr Leu Ser Glu Asn Val Arg Thr Leu
1               5                   10


<210>  143
<211>  10
<212>  PRT
<213>  Artificial Sequence

<220>
<223>  Description of the artificial Sequence: HLA restricted Peptide

<400>  143

Leu Leu Ser Ser Ala Pro Val Val Met Trp

1                   5                   10

<210>  144
<211>  9
<212>  PRT
<213>  Artificial Sequence

<220>
<223>  Description of the artificial Sequence: HLA restricted Peptide

<400>  144

Gly Asn Phe Pro Gly Leu Val Trp Val
1               5

<210>  145
<211>  9
<212>  PRT
<213>  Artificial Sequence

<220>
<223>  Description of the artificial Sequence: HLA restricted Peptide

<400>  145

Met Gln Leu Glu Lys Leu Val Val Leu
1               5

<210>  146
<211>  9
<212>  PRT
<213>  Artificial Sequence

<220>
<223>  Description of the artificial Sequence: HLA restricted Peptide

<400>  146

Pro Leu Gly Lys Leu Ala Ser Asn Val
1               5

<210>  147
<211>  9
<212>  PRT
<213>  Artificial Sequence

<220>
<223>  Description of the artificial Sequence: HLA restricted Peptide

<400>  147

Leu Cys Arg Gly Gly Ala Cys Ser Leu
1               5

<210>  148
<211>  10
<212>  PRT
<213>  Artificial Sequence

<220>
<223>  Description of the artificial Sequence: HLA restricted Peptide

<400>  148

Ala Leu Cys Arg Gly Gly Ala Cys Ser Leu
1               5                   10


<210>  149
<211>  10
<212>  PRT
<213>  Artificial Sequence

<220>
<223>  Description of the artificial Sequence: HLA restricted Peptide

<400>  149

Val Leu Pro Gly Val Asp Trp Lys Cys Val
1               5                   10


<210>  150
<211>  10
<212>  PRT
<213>  Artificial Sequence

<220>
<223>  Description of the artificial Sequence: HLA restricted Peptide

<400>  150

Arg Thr Asp Gly Asn Pro Leu Gly Lys Leu
1               5                   10


<210>  151
<211>  10
<212>  PRT
<213>  Artificial Sequence

<220>
<223>  Description of the artificial Sequence: HLA restricted Peptide

<400>  151

Trp Met Glu Pro Arg Gly Cys His Ala Leu
1               5                   10

<210>  152
<211>  10
<212>  PRT
<213>  Artificial Sequence

<220>
<223>  Description of the artificial Sequence: HLA restricted Peptide

<400>  152

Cys Leu His Ser Pro His Thr Gly Phe Leu
1               5                   10


<210>  153
<211>  9
<212>  PRT
<213>  Artificial Sequence

<220>
<223>  Description of the artificial Sequence: HLA restricted Peptide

<400>  153

Ala Leu Phe Ser Ser Cys Gly Asn Val
1               5


<210>  154
<211>  10
<212>  PRT
<213>  Artificial Sequence

<220>
<223>  Description of the artificial Sequence: HLA restricted Peptide

<400>  154

His Thr Gly Gly Ser Gly Trp Ser His Val
1               5                   10


<210>  155
<211>  10
<212>  PRT
<213>  Artificial Sequence

<220>
<223>  Description of the artificial Sequence: HLA restricted Peptide

<400>  155

Gly Gly Ser Gly Trp Ser His Val Asp Val
1               5                   10

<210> 156
<211> 9
<212> PRT
<213> Artificial Sequence

<220>
<223> Description of the artificial Sequence: HLA restricted Peptide

<400> 156

His Lys Tyr Cys Phe Met Leu Phe Leu
1               5


<210> 157
<211> 10
<212> PRT
<213> Artificial Sequence

<220>
<223> Description of the artificial Sequence: HLA restricted Peptide

<400> 157

Lys His Lys Tyr Cys Phe Met Leu Phe Leu
1               5                   10


<210> 158
<211> 9
<212> PRT
<213> Artificial Sequence

<220>
<223> Description of the artificial Sequence: HLA restricted Peptide

<400> 158

Lys Leu Lys Gly Leu Lys Cys Ser Arg
1               5


<210> 159
<211> 9
<212> PRT
<213> Artificial Sequence

<220>
<223> Description of the artificial Sequence: HLA restricted Peptide

<400> 159

Lys Leu Val Ile Tyr Ile Phe Ser Asn
1               5


<210> 160

<211>  9
<212>  PRT
<213>  Artificial Sequence

<220>
<223>  Description of the artificial Sequence: HLA restricted Peptide

<400>  160

Val Ile Tyr Ile Phe Ser Asn Ser Pro
1               5


<210>  161
<211>  10
<212>  PRT
<213>  Artificial Sequence

<220>
<223>  Description of the artificial Sequence: HLA restricted Peptide

<400>  161

Met Glu Asn Ser Lys Leu Val Ile Tyr Ile
1               5                   10

## Description

The present invention relates to colorectal lesion associated (CLA) nucleic acids and polypeptides useful for the detection and treatment of disorders characterized by abnormal cell proliferation. The invention is more specifically related to a nucleic acid and the polypeptides transcribed thereof, the expression of which is significantly altered in association with adenomas and adenocarcinomas of the colon and other cancers. Another aspect of the nucleic acids disclosed herein are transcripts of the gene disclosed herein, that are differentially produced in various tumours, such as e.g. those of the gastrointestinal, the respiratory or the anogenital tracts. Furthermore the present invention provides methods for early diagnosis, prognosis and monitoring of the disease course and for therapy and vaccination of tumours associated with the expression of the inventive compounds.

In colorectal lesions as in most other tumours there is a strong correlation between the patients outcome following initial therapy and the stage at which the disease has been diagnosed. So the earlier the cancer could be detected the better are the chances for the patient to survive. Thus sensitive testing methods are required for detecting the tumours in early stages or even in preliminary stages of the cancer such as pre-cancerous stages or the precursors of malignant cancerous stages.

The most promising methods for early diagnosis of tumours are those involving molecular markers characteristic for tumours or characteristic for precursory stages of tumours.

Colorectal cancer is a quite heterogeneous disease. Multiple regulators of the cell growth can be involved in the genesis of cancer. These regulatory elements of the cell cycle can be either positive regulators, named oncogenes when mutated, so that a transformed state is reached, or negative regulators, named tumour suppressor genes. The number of factors known to be involved in the regulation of the cell cycle and potentially being candidates for the development of cancer exceeds 100 up to know and is still increasing.

The molecules being involved in the emergence of the cancerous state of a cell can be used to discriminate between cancer cells and normal tissue. Thus cancerous tissue can be detected by detecting molecules characteristic for the cancer cells. This turns out to be sophisticated due to the large number of molecules potentially being involved in causing cancer.

For improved diagnosis of tumours there is a need for new marker molecules for use in diagnosis of cancers and especially of colorectal cancer, that enable for specific early detection and give the opportunity to treat the disorders at an early stage.

The present invention provides nucleic acids and polypeptides associated with colorectal cancer. According to the present invention these molecules may be used as molecular markers that allow for comprehensive detection of cell proliferative disorders such as e.g. colorectal lesions even at early stages. Furthermore the inventive polypeptides and polynucleotides may be used for the detection and therapy of disorders characterized by abnormal proliferation of cells.

During the experiments leading to the present invention a CLA2 gene as given in SEQ NO 1, 2, 4, 6, 8, 10, 12, 14 and 16 was identified, the expression of which is associated with colorectal lesions. The inventors found, that the CLA2 gene is differentially expressed on a nucleic acid level as well as on a polypeptide level in adenomas and adenocarcinomas of the colon compared to the respective matched normal tissue. The inventors subsequently proved, that the CLA2 gene is also differentially expressed in other cancers such as lung cancer, cervical cancer, gastric cancer, breast cancer and pancreatic cancer.

The inventors found, that the identified CLA2 gene as well as the CLA2 nucleic acid and polypeptide expression products transcribed thereof are not published up to date. There is only restricted data concerning EST clones with stretches of identical sequence in databases. In total five stretches could be found. The inventors identified a CLA2 gene of total 35586 nucleic acids exhibiting an open reading frame. The gene is encoding several differentially spliced CLA2 transcripts (designated CLA2.1 - CLA2.3). Antibody based as well as mass spectrometric analysis could reveal the expression of the protein products in tumour cells.

Investigations on the function of the identified polypeptides and nucleic acids revealed, that the sequences are derived from medium reiteration frequency elements (MER61) which may represent endogenous viral sequences integrated into the human genome, that are expressed e.g. in tumour tissues.

Investigation on the expression of the gene in different stages of tumours elucidated its use for diagnostic and prognostic purposes. Thus the present invention is furthermore based on the inventors findings shown in the examples given below, that the level of expression of nucleic acids as well as of polypeptides transcribed from the CLA2 marker gene presented herein in SEQ NO 3, 5, 7, 9, 11, 13, 15 and 17 in samples allows to diagnose and grade disorders such as e.g. colorectal lesions associated with abnormal cell proliferation, to predict the course of the disease and to follow up the disease after initial therapy.

The present invention provides CLA2 polypeptides and nucleic acids, the expression of which is significantly altered associated with colorectal lesions, that allow for enhanced prognosis and diagnosis of diseases associated with abnormal cell proliferation such as tumours and their preliminary stages. Furthermore the CLA2 nucleic acids disclosed herein comprise transcripts arising from alternative splicing of genes, that do not occur in normal tissue in the extent they may be found in tumourous tissue. The present invention thus provides novel CLA2 nucleic acids and the polypeptides encoded thereby, which are associated with colorectal lesions such as adenomas and adenocarcinomas of the colon.

The present invention provides tumour associated nucleic acids and polypeptides associated with colorectal lesions characterized by the sequences given in SEQ NO 1 - 17. Furthermore the present invention provides variants of the nucleic acids and polypeptides, which arise from tumour associated alternative splicing events.

In another aspect of the invention the CLA2 nucleic acids and/or polypeptides disclosed herein alone or in combination with other molecules may be used for therapy and/or vaccination of disorders associated with abnormal cell proliferation such as tumours.

Yet another aspect of the present invention are pharmaceutical compositions containing CLA2 polypeptides and/or CLA2 polynucleotides disclosed herein alone or combination with one or more other therapeutic or diagnostic agents and/or carrier or adjuvant substances.

The present invention also provides kits such as diagnostic kits or research kits for the detection of the CLA2 polynucleotides or CLA2 polypeptides disclosed herein or comprising the CLA2 polynucleotides or CLA2 polypeptides disclosed herein or combinations thereof.

Another aspect of the present invention is a method for diagnosis of disorders such as e.g. tumours comprising the detection of the CLA2 nucleic acids and/ or the CLA2 polypeptides disclosed herein or of immunological entities directed against these CLA2 molecules alone or combination with one or more other marker molecules in samples. In this aspect the detection of the level of the nucleic acids and polypeptides presented herein may be used as a marker indicative of the presence or absence of cell proliferative disorders.

In one aspect the method according to the present invention is especially useful for early detection of disorders associated with abnormal cell proliferation such as e.g. colorectal lesions and for detection of disseminated tumour cells in the course of diagnosis of minimal residual disease. The method for detection of said disorders comprises the detection of the presence or absence and/or the level of CLA2 molecules in biological samples. This methods may e.g. employ minimally invasive or non-invasive methods for obtaining the sample.

In another aspect of the present invention the detection of CLA2 nucleic acids or CLA2 polypeptides disclosed herein may be used in the course of diagnosis of disorders associated with abnormal proliferation of cells in samples such as tumour resections, biopsies or the like. In this aspect the invention provides a method, that allows to build a strategy for the therapy of diseases according to their molecular properties. According to the present invention the level of said CLA2 polypeptides and/or CLA2 nucleic acids can be used as a molecular marker for prognosis, monitoring and the design of a strategy of tumour therapeutics.

It is yet another aspect of the present invention to provide methods for identification of molecules binding to the nucleic acids and polypeptides of the present invention as well as of activators and inhibitors of the expression of the genes of the present invention. Also a method for the identification of drug candidates for the therapy of proliferative disorders is provided.

One further aspect of the present invention is a method for therapy of disorders associated with abnormal cell proliferation. In this aspect the inventive CLA2 polypeptides and/or nucleotides may be administered to individuals suffering from said disorders in the course of immuno-therapy or gene-therapy.

Yet another aspect of the present invention is the use of CLA2 for vaccination therapy of individuals being at risk of contracting a disease associated with abnormal cell proliferation or suffering from such a disease.

The present invention provides marker molecules such as tumour associated nucleic acids and polypeptides characterized by the sequences given in SEQ NO 1 - 17.

Marker molecules as used in the present invention may comprise nucleic acids and polynucleotides. On the level of nucleic acids the marker molecules may be DNA or RNA comprising genomic DNA, cDNA, and RNA such as mRNA or hnRNA. In one preferred embodiment of the invention nucleic acids arising from particular differential splicing events may be marker molecules.

Expression as used according to the present invention may comprise for example expression of proteins. The transcription to RNA and thus the level of mRNA may also be understood to be expression according to the present invention.

The expression of a compound is said to be significantly altered according to the present invention, if the level of expression differs by more than 30%. The alteration of the expression may comprise for example elevated expression or reduced expression of said compound. Another aspect of the altered expression may be an alteration in a way, that the compound is expressed under non wild-type circumstances. This may comprise, that the compound is for example expressed in situations, that naturally suppress the expression, or is not expressed in situations, that naturally induce the expression of the compound.

Alteration of the expression as used herein may also comprise an alteration in the transcription pattern of a gene. E.g. the alteration of the transcription pattern may comprise alternative splicing of the gene. The alterations in the transcription pattern may influence the polypeptides translated from the altered transcripts or may be restricted to untranslated regions. The alteration in the transcription pattern of a gene may comprise use of novel exons in the transcripts, deletions of exons in the transcripts or the variation in the ratios of different splicing variants in cells. Thus alterations in transcriptional patterns of genes as used herein may comprise the production of nucleic acids such as e.g. mRNA, cDNA etc. containing additional stretches of nucleic acid sequences compared to wild type nucleic acids occurring in control tissues. Alternatively the nucleic acids produced by alternative splicing patterns may produce nucleic acids missing stretches of nucleic acid sequences present in wild type polynucleotides. The presence of additional stretches may occur simultaneously with the absence of original sequence-stretches in single transcripts. Alterations in the expression of genes as used in the context of the present invention may also comprise an alteration in the level of expression of splicing variants of genes. This may include increased or decreased expression of particular splicing variants as well as expression of variants not present in wild type tissue or the absence of expression of splicing variants present in wild type tissue. In one embodiment the alteration of the expression of the splicing variants may comprise the alteration of the ratios of different splicing variants in said tissue.

Nucleic acids as used in the context of the present invention are preferably polynucleotides or fragments thereof. Preferred polynucleotides comprise at least 20 consecutive nucleotides, preferably at least 30 consecutive nucleotides and more preferably at least 45 consecutive nucleotides, that are identical, share sequence homology or encode for identical, or homologous polypeptides, compared to the polypeptides associated with the proliferative disorders disclosed herein. The nucleic acids according to the present invention may also be complementary to any of said polynucleotides. Polynucleotides may for example include single-stranded (sense or antisense) or double-stranded molecules, and may be DNA (genomic, cDNA or synthetic) or RNA. RNA molecules comprise as well hnRNA (containing introns) as mRNA (not containing introns). According to the present invention the polynucleotides may also be linked to any other molecules, such as support materials or detection marker molecules, and may, but need not, contain additional coding or non-coding sequences.

The CLA2 polynucleotides according to the present invention may be native sequences or variants thereof. The variants may contain one or more substitutions, additions, deletions and/or insertions such that the immunogenicity of the encoded polypeptide is not diminished, relative to the respective native CLA2 proteins. The variants show preferably 65 -70%, more preferably at least 80% and most preferably at least 90% of sequence identity to the native nucleic acid molecules used in the methods according to the present invention. In one embodiment of the invention the variants show sequence identity of at least 65% to 99% or any value in between to the native CLA2 nucleic acids as disclosed herein in SEQ-N°1, 2, 4, 6, 8, 10, 12, 14, and 16. In another embodiment of the invention the variants show sequence homologies of about 60, 65, 70, 75, 80, 85, 90, 95 or even 100%. Methods for determination of sequence similarity are known to those of skill in the art.

One example for detecting the similarity of sequences can be carried out using the FastA and/or BlastN bioinformatics software accessible on the HUSAR server of the DKFZ Heidelberg.

Furthermore CLA2 nucleic acids according to the present invention are all polynucleotides, which hybridise to probes specific for the sequences disclosed herein under stringent conditions. Stringent conditions applied for the hybridisation reaction are known to those of ordinary skill in the art and may be applied as described in Sambrook et al. Molecular cloning: A Laboratory Manual, 2^{nd} Edition, 1989.

The present invention also provides polynucleotides, that due to the degeneracy of the genetic code encode the CLA2 polypeptides natively encoded by the disclosed CLA2 nucleic acids while not showing the percentage of sequence homology as described above within the nucleic acid sequence. Such nucleic acids might arise by changing the codons present in the disclosed sequences by degenerate codons and so preparing a synthetic nucleic acid.

The nucleotide sequences according to the present invention may be joined to a variety of other nucleic acid sequences using the known recombinant DNA techniques. The sequences may for example be cloned into any of a variety of cloning vectors, such as plasmids, phagemids, lambda phage derivatives and cosmids. Furthermore vectors such as expression vectors, replication vectors, probe generation vectors and sequencing vectors may be joined with the sequences disclosed herein. Sequences of special interest, that could be cloned to the nucleic acids according to the present invention are for example non coding sequences and regulatory sequences including promoters, enhancers and terminators.

In certain embodiments of the present invention one or more of the nucleic acid sequences encoding CLA2 polypeptides may be joined. This may be especially useful for therapeutic purposes or for the expression of recombinant proteins. In these embodiments 2, 3, 4, 5, 6, 7, 8, 10 or even more different or even identical CLA2 nucleic acids may be joined together in one nucleic acid molecule.

In a preferred embodiment CLA2 polynucleotides may be formulated such, that they are able to enter mammalian cells and to be expressed in said cells. Such formulations are especially useful for therapeutic purposes. The expression of nucleic acid sequences in target cells may be achieved by any method known to those skilled in the art. The nucleic acids may for example be joined to elements that are apt to enable their expression in a host cell. Such elements may comprise promoters or enhancers, such as CMV-, SV40-, RSV-, metallothionein I- or polyhedrin-promoters respectively CMV- or SV40-enhancers. Possible methods for the expression are for example incorporation of the polynucleotides into a viral vector including adenovirus, adeno-associated virus, retrovirus, vaccinia virus or pox virus. Viral vectors for the purpose of expression of nucleic acids in mammalian host cells may comprise pcDNA3, pMSX, pKCR, pEFBOS, cDM8, pCEV4 etc.. These techniques are known to those skilled in the art.

Other formulations for administration in therapeutic purposes include colloidal dispersion systems such as for example macromolecule complexes, microspheres, beads, micelles and liposomes.

Generally, by means of conventional molecular biological processes it is possible (see, e.g., Sambrook et al., supra) to introduce different mutations into the nucleic acid molecules of the invention. As a result the inventive tumour associated CLA2 polypeptides or polypeptides related thereto with possibly modified biological properties are synthesized. One possibility is the production of deletion mutants in which nucleic acid molecules are produced by continuous deletions from the 5'- or 3'-terminal of the coding DNA sequence and that lead to the synthesis of CLA2 polypeptides that are shortened accordingly. Another possibility is the introduction of single-point mutation at positions where a modification of the amino aid sequence influences, e.g., the proliferation specific properties. By this method muteins can be produced, for example, that possess a modified Km-value or that are no longer subject to the regulation mechanisms that normally exist in the cell, e.g. with regard to allosteric regulation or covalent modification. Such muteins might also be valuable as therapeutically useful antagonists of the inventive tumour associated marker.

For the manipulation in prokaryotic cells by means of genetic engineering the CLA2 nucleic acid molecules of the invention or parts of these molecules can be introduced into plasmids allowing a mutagenesis or a modification of a sequence by recombination of DNA sequences. By means of conventional methods (cf. Sambrook et al., supra) bases can be exchanged and natural or synthetic sequences can be added. In order to link the DNA fragments with each other adapters or linkers can be added to the fragments. Furthermore, manipulations can be performed that provide suitable cleavage sites or that remove superfluous DNA or cleavage sites. If insertions, deletions or substitutions are possible, *in vitro* mutagenesis, primer repair, restriction or ligation can be performed. As analysis method usually sequence analysis, restriction analysis and other biochemical or molecular biological methods are used.

The CLA2 polypeptides encoded by the various variants of the CLA2 nucleic acid molecules of the invention show certain common characteristics, such as molecular weight, immunological reactivity or conformation or physical properties like the electrophoretical mobility, chromatographic behaviour, sedimentation coefficients, solubility, spectroscopic properties, stability, pH optimum, temperature optimum.

The invention furthermore relates to vectors containing the inventive tumour associated CLA2 nucleic acid molecules. Preferably, they are plasmids, cosmids, viruses, bacteriophages and other vectors usually used in the field of genetic engineering. Vectors suitable for use in the present invention include, but are not limited to the T7-based dual expression vectors (expression in prokaryotes and in eucaryotes) for expression in mammalian cells and baculovirus-derived vectors for expression in insect cells. Preferably, the CLA2 nucleic acid molecule of the invention is operatively linked to the regulatory elements in the recombinant vector of the invention that guarantee the transcription and synthesis of an mRNA in prokaryotic and/or eucaryotic cells that can be translated. The nucleotide sequence to be transcribed can be operably linked to a promoter like a T7, metallothionein I or polyhedrin promoter.

In a further embodiment, the present invention relates to recombinant host cells transiently or stably containing the CLA2 nucleic acid molecules or vectors of the invention. A host cell is understood to be an organism that is capable to take up *in vitro* recombinant DNA and, if the case may be, to synthesize the polypeptides encoded by the nucleic acid molecules of the invention. Preferably, these cells are prokaryotic or eucaryotic cells, for example mammalian cells, bacterial cells, plant cells, insect cells or yeast cells. The host cells of the invention are preferably characterized by the fact that the introduced CLA2 nucleic acid molecule of the invention either is heterologous with regard to the transformed cell, i.e. that it does not naturally occur in these cells, or is localized at a place in the genome different from that of the corresponding naturally occurring CLA2 sequence.

A further embodiment of the invention relates to a polypeptide exhibiting a biological property of the inventive tumour associated marker CLA2 and being encoded by the CLA2 nucleic acid molecules of the invention, as well as to methods for their production, whereby, e.g., a host cell of the invention is cultivated under conditions allowing the synthesis of the CLA2 polypeptide and the CLA2 polypeptide is subsequently isolated from the cultivated cells and/or the culture medium. Isolation and purification of the recombinantly produced polypeptide may be carried out by conventional means including preparative chromatography and affinity and immunological separations using, e.g., an antibody directed against the inventive tumour associated marker proteins, or, e.g., can be substantially purified by the one-step method described in Smith and Johnson, Gene 67; 31-40 (1988). These polypeptides, however, not only comprise recombinantly produced CLA2 polypeptides but include isolated naturally occurring CLA2 polypeptides, synthetically CLA2 produced polypeptides, or polypeptides produced by a combination of these methods. Means for preparing such polypeptides or related polypeptides are well understood in the art. These polypeptides are preferably in a substantially purified form.

The production of a CLA2 polypeptide according to the present invention may for example be carried out in a cell free *in vitro* transcription and/or translation system. Such systems are known to those of ordinary skill in the art. One example may comprise an intro translation system as provided by Roche molecular Biochemicals' Rapid translation System.

CLA2 (poly)peptides as used according to the present invention may comprise amino acid chains of any length, including full length proteins, wherein the amino acid residues are linked by covalent peptide bonds.

Tumour associated CLA2 peptides as disclosed in the context of the present invention shall comprise polypeptides of lengths of at least 4 amino acids. The CLA2 peptides may for example comprise 4 to 50 amino acids or any number of amino acids in between, In another embodiment of the present invention the peptides may comprise polypeptides with more than 50 amino acids. These CLA2 polypeptides for use in the context of the present invention may for example comprise 50,100, 500, 750, 1000 amino acids or any number of amino acids in between and may comprise proteins, or fragments thereof, and/or fusion- or chimeric proteins comprising on or more additional heterologous sequences. The additional sequences may be derived from the native CLA2 protein or may be heterologous, and such sequences may (but need not) be immuno-reactive and/or antigenic. As detailed below, such polypeptides may be isolated from tumour tissue or prepared by synthetic or recombinant means.

As used herein, a polypeptide exhibiting biological properties of the tumour associated CLA2 peptides is understood to be a polypeptide having at least substantially the same immunogenic properties, i.e. is still capable of binding an antibody directed against said CLA2 polypeptide, e.g. comprises an immunogenic portion of said CLA2 polypeptide.

Peptides disclosed herein are immunogenic polypeptides. This requires, that the polypeptides may stimulate immune responses in host organisms either in the form the polypeptides adopt in their natural environment and/or especially in the form the polypeptides adopt after processing by the cellular antigen processing and presenting machinery.

Immunogenic portion as used above is a portion of a protein, that is recognized by a B-cell and/or T-cell surface antigen receptor. The immunogenic portions comprise at least 4 amino acid residues, at least 10 amino acid residues or at least 15 amino acid residues of the protein disclosed herein. In one embodiment of the present invention, particular domains of the protein, such as for example transmembrane domains or N-terminal leader sequences have been deleted.

The immunogenic portions according to the present invention react with antisera or specific antibodies in the same or nearly same intensity as the native full length proteins. The immunogenic portions are generally identified using the techniques well known in the art. Possible techniques are for example screening of the polypeptides for the ability to react with antigen-specific antibodies, antisera and/or T-cell lines or clones.

In one embodiment of the present invention the immunogenic peptides that may be derived from a protein may be predicted as described by Rammensee et al, "MHC ligands and peptide motifs: 1st listing", Immunogenetics 41, 178-228, 1995 and Rammensee, et al., "MHC ligands and peptide motifs" Bioscience 1997, which methods shall be included herein by reference. The methods are exemplified below in the examples.

Peptides derived from the CLA2 tumour associated proteins as used in the context of the present invention may comprise the respective full length proteins, fragments thereof, or peptides of any length. In certain embodiments of the present invention the peptides derived from cell cycle regulatory proteins showing altered expression in association with cancers may be e.g. at least one or more peptides selected from the peptides given below.

In certain embodiment of the present invention the peptides derived from the CLA2 expression products may be e.g. 15-mer peptides.

In other embodiment the peptides are preferably presented by MHC class II molecules.

In yet another embodiment the peptides may be HLA-A1 restricted nonamer or decamer polypeptides selected from a group consisting of the following:

| | | | |
|---|---|---|---|
| RTDGNPLGK | RTDGNPLGKL | QLEKLVVLPG | NMDEEKHKY |
| LSENVRTLE | LSENVRTLEN | ENMDEEKHKY | RTDGNPLGK |
| RTDGNPLGKL | QLEKLVVLPG | WSHVDVMPY | AGEEPALFS |
| MDEEKHKYC | MENSKLVIY | | |

In yet another embodiment the peptides are HLA-A3 restricted nonamers or decamers selected from a group consisting of:

| | | | |
|---|---|---|---|
| WLPGVDWK | RTDGNPLGK | KLVVLPCVD | LWLPGVDW |
| KLASNVCLH | LVVLPGVDWK | KLWLPGVDW | CVLPFKQSSS |
| TLENMDEEK | SLLSSAPVV | SLLSSAPVVM | TLEEVDGATW |
| WLPGVDWK | ALCRGGACS | RTDGNPLGK | KLVVLPGVD |
| LVVLPGVDW | KLASNVCLH | LWLPGVDWK | ALCRGGACS |
| KLVVLPGVDW | CVLPFKQSSS | ALFSSCGNVG | KHKYCFMLF |
| KLKGLKCSR | KLVIYIFSN | LVIYIFSNS | VIYIFSNSP |

In one further embodiment the peptides may be HLA A*0201 restricted nonamers or decamers selected from a group comprising the following peptides:

| | | | |
|---|---|---|---|
| MQLEKLVVL | PLGKLASNV | VLPGVDWKCV | RTDGNPLGKL |
| CLHSPHTGFL | TLSENVRTL | SLLSSAPVV | CLMQGRSLL |
| LLSSAPVVM | VMWSICEE | SLLSSAPWM | LMQGRSLLSS |
| ETLSENVRTL | LLSSAPWMW | GNFPGLVWV | MQLEKLVVL |
| PLGKLASNV | LCRGGACSL | ALCRGGACSL | VLPGVDWKCV |
| RTDGNPLGKL | WMEPRGCHAL | CLHSPHTGFL | ALFSSCGNV |
| HTGGSGWSHV | GGSGWSHVDV | HKYCFMLFL | KHKYCFMLFL |
| KLKGLKCSR | KLVIYIFSN | VIYIFSNSP | MENSKLVIYI |

In certain embodiments peptides being characteristic for one of the various polypeptides encoded by the CLA2 gene may be desirable. Peptides suitable for differential binding of the various polypeptides identified so far are e.g. for distinguishing CLA2.1-CLA2.8, may be selected from the following:

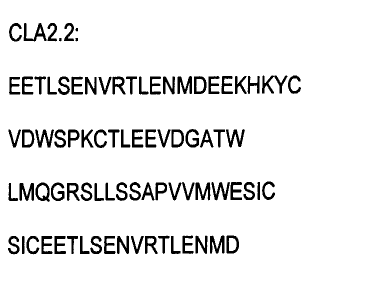

EEKHKYCFMLFL

CLA2.1 and CLA2.3:
RTDGNPLGKLASNVC
MQLEKLVVLPGVDWKC
VLPFKQSSSTCRTDG
NPLGKLASNVCLHSPHTG
FLTCDEGNFPGLVWV

Unique in CLA2.3:
AQNAHWRKWMEPRGC
CHALCRGGACSLQLLW

CLA2.4:
MHTGGSGWSHVDVMPYA
GEEPALFSSCGNVGIDL

CLA2.5:
MDEEKHKYCFMLFL

CLA2.6:
MKLKGLKCSR

CLA2.7:
MHSSLRFG

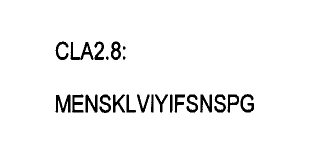

It must be understood, that due to the similarity of the sequences CLA2.1 and CLA2.3 the peptides useful for detection of CLA2.3 may also be used for the detection of CLA2.1. The peptide given for detection of CLA2.3 may be used to detect CLA2.3 and not CLA2.1 and thus is suited to distinguish these proteins. Besides the given peptide sequences other sequences derived from the proteins may be used as immunogenic epitopes (antigens) in immuno-chemical detection reaction for the detection of immunological entities directed against the CLA2 molecules or for the purpose of generation of binding agents. Also fragments or variants comprising substitutions in the amino acid sequences may be used in the context of the present invention.

In certain embodiments of the present invention CLA2 polypeptides may comprise fusion or chimeric polypeptides containing sequences disclosed herein. Fusion proteins comprise the polypeptide according to the present invention together with any second and further polypeptides, such as e.g. one or more polypeptides of the same sequence or of another sequence. Heterologous polypeptides may comprise e.g. enzymes, receptor molecules, antigens, antigenic or immunogenic epitopes or fragments, antibodies or fragments thereof, signalling polypeptides or signal transducing polypeptides, labelled polypeptides etc.. The immunogenic protein may for example be capable of eliciting a recall response. Examples of such proteins include tetanus, tuberculosis and hepatitis proteins (see, for example, Stoute et al. New Engl. J. Med., 336:86-91 (1997)). For use in pharmaceutical compositions fusion proteins comprising serum albumin or fragments thereof may be useful in certain embodiments of the present invention.

In one embodiment of the invention the fusion peptides may be constructed for enhanced detection or purification of the polypeptides, or of complexes of the CLA2 polypeptides with the respective immunological entities according to the present invention. For the purpose of purification tags, such as e.g. his-tags, myc-tags etc. may be added to the polypeptides. For the purpose of detection antigenic portions, enzymes, chromogenic sequences etc. may be fused to the polypeptides. The fusion proteins of the present invention may (but need not) include a linker peptide between the first and second polypeptides.

A peptide linker sequence may be employed to separate the first and the second polypeptides by a distance sufficient to ensure, that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes.

Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8262, 1986; U.S. Pat. No. 4,935,233 and U.S. Pat. No. 4,751,180. The linker sequence may be from 1 to about 50 amino acids in length. Peptide sequences are not required when the first and second polypeptides have non-essential N-terminal amino acid regions that can be used to separate the functional domains and prevent steric interference.

The CLA2 polypeptides associated with tumours according to the present invention comprise also variants of the native CLA2 proteins. These variants may differ from the native protein in one or more alterations such as substitutions, deletions, additions and/or insertions. The immuno-reactivity of the variants according to the present invention is not substantially diminished compared to the native CLA2 proteins. In a preferred embodiment of the invention the immuno-reactivity is diminished less than 50% in a more preferred embodiment the immuno-reactivity is diminished less than 20 % compared to the native polypeptides. In one embodiment the immuno-reactivity is diminished less than 5%, 10%, 15%, 20%, 25%, 30%, 35%, 40%, 45%, 50% or any value in between. In certain embodiments the immuno-reactivity of the variants is reduced by even more than 50%.

In one embodiment CLA2 variants may be deficient in one or more portions, such as for example N-terminal leader sequences, transmembrane domains or small N- and/or C-terminal sequences. The variants exhibit 60%, 65% or 70%, more preferably at least 75%, 80%, 85% or 90% and most preferably at least 92.5% 95%. 97.5%, 98%, 98.5%, 99% or 99.5% identity to the CLA2 polypeptides disclosed according to the present invention.

The variants of the present invention are preferably conservative substitutions, so that the amino acids changed are substituted for amino acids with similar properties. The properties concerned may include polarity, charge, solubility, hydrophobicity, hydrophilicity and/or amphipathic nature of the amino acid residues. The variants disclosed herein may also comprise additional terminal leader sequences, linkers or sequences, which enable synthesis, purification or stability of the polypeptides in an easier or more comfortable way.

The CLA2 polypeptides and CLA2 polynucleotides according to the present invention are isolated. This means that the molecules are removed from their original environment. Naturally occurring CLA2 proteins are isolated if they are separated from some or all of the materials, which coexist in the natural environment. CLA2 polynucleotides are isolated for example if they are cloned into vectors.

The CLA2 (poly)peptides according to the present invention may be produced by any method known to those of skill in the art. E.g. the polypeptides may be isolated from cells or organisms expressing the polypeptides, may be produced recombinantly in recombinant host cells or may be synthesized chemically by the methods commonly applied for synthesis of polypeptides.

Furthermore the present invention provides binding agents such as antibodies and antigen-binding fragments, that specifically bind to the CLA2 proteins associated with tumours disclosed herein.

The term binding agent comprises a variety of substances such as oligopeptides, antibodies, peptdiomimetic molecules comprising antigen binding oligopeptides, nucleic acids, carbohydrates, organic compounds, etc.. Antibody according to the present invention preferably relates to antibodies which consist essentially of pooled monoclonal antibodies with different epitopic specificities, as well as distinct monoclonal antibody preparations. Monoclonal antibodies are made from an antigen containing fragments of the polypeptides of the invention by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab') 2 fragments) which are capable of specifically binding to protein. Fab and f(ab')2 fragments lack the Fc fragment of intact antibody, clear more rapidly from the circulation, and may have less non-specific tissue binding than an intact antibody. (Wahl et al., J. Nucl. Med. 24: 316-325 (1983)). Thus, these fragments are preferred, as well as the products of a FAB or other immuno-globulin expression library. Moreover, antibodies of the present invention include chimerical, single chain, and humanized antibodies.

Binding agents according to the present invention may for example be employed for the inhibition of the activity of the inventive CLA2 polypeptides disclosed herein. In this respect the term "binding agents" relates to agents specifically binding to the CLA2 polypeptides transcribed from the novel tumour associated nucleic acids and thus inhibiting the activity of said polypeptide. Such binding agents may for example comprise nucleic acids (DNA, RNA, PNA etc.), polypeptides (antibodies, receptors, antigenic fragments, oligopeptides), carbohydrates, lipids, organic or inorganic compounds (metal-ions, sulphur compounds, boranes, silicates, reducing agents, oxidizing agents). The binding agents may preferably interact with the polypeptide by binding to epitopes, that are essential for the biological activity. The interaction may be reversible or irreversibly. The binding may be non-covalent or even covalent binding to the polypeptide. Furthermore the binding agents may introduce alterations to the CLA2 polypeptide, that alter or diminish the biological activity of the inventive CLA2 polypeptide.

For certain purposes, e.g. diagnostic methods, the antibody or binding agent of the present invention may be detectably labelled, for example, with a radioisotope, a bioluminescent compound, a chemiluminescent compound, a fluorescent compound, a metal chelate, a biologically relevant binding structure such as biotin or digoxygenin or an enzyme. Furthermore any method suitable for the detection of the intermolecular interaction may be employed.

The antibody or antigen-binding agent is said to react specifically, if it reacts at a detectable level with a CLA2 protein disclosed herein, and does not significantly react with other proteins. The antibodies according to the present invention may be monoclonal or polyclonal antibodies. Other molecules capable of binding specifically may be for example antigen-binding fragments of antibodies such as Fab fragments, RNA molecules or polypeptides. According to the present invention binding agents may be used isolated or in combination. By means of combination it is possible to achieve a higher degree of sensitivity.

In certain embodiments binding agents may exhibit selective specificities for the various polypeptide products encoded by the CLA2 gene. These binding agents may e.g. be defined epitopic specificity. The specificity may e.g. chosen in a way to ensure that only one polypeptide product of the CLA2 gene is recognized by the respective binding agent.

The antibodies or binding agents useful for the methods according to the present invention may comprise further binding sites for either therapeutic agents or other polypeptides or may be coupled to said therapeutic agents or polypeptides. Therapeutic agents may comprise drugs, toxins, radionuclides and derivatives thereof. The agents may be coupled to the binding agents either directly or indirectly for example by a linker or carrier group. The linker group may for example function in order to enable the coupling reaction between binding agent and therapeutic or other agent or the linker may act as a spacer between the distinct parts of the fusion molecule. The linker may also be cleavable under certain circumstances, so as to release the bound agent under said conditions. The therapeutic agents may be covalently coupled to carrier groups directly or via a linker group. The agent may also be non-covalently coupled to the carrier. Carriers that can be used according to the present invention are for example albumins, polypeptides, polysaccharides or liposomes.

The antibody used according to the present invention may be coupled to one or more agents. The multiple agents coupled to one antibody may be all of the same species or may be several different agents bound to one antibody.

The invention also relates to a transgenic non-human animal such as transgenic mouse, rats, hamsters, dogs, monkeys, rabbits, pigs, C. elegans and fish such as torpedo fish comprising a CLA2 nucleic acid molecule or vector of the invention, preferably wherein said CLA2 nucleic acid molecule or vector may be stably integrated into the genome of said non-human animal, preferably such that the presence of said CLA2 nucleic acid molecule or vector leads to the expression of the inventive marker CLA2 polypeptide (or related polypeptide) of the invention, or may otherwise be transiently expressed within the non-human animal. Said animal may have one or several copies of the same or different nucleic acid molecules encoding one or several forms of the inventive CLA2 polypeptide or mutant forms thereof. This animal has numerous utilities, including as a research model for the regulation of cell proliferation and differentiation and therefore, presents a novel and valuable animal in the development of therapies, treatment, etc. for diseases caused by deficiency or failure of the inventive CLA2 protein involved in the development of cell proliferative disorders, e.g., tumours. Accordingly, in this instance, the non-human mammal is preferably a laboratory animal such as a mouse or rat.

In certain embodiments, the transgenic non-human animal of the invention further comprises at least one inactivated wild type allele of the corresponding gene encoding the inventive CLA2 polypeptide. This embodiment allows for example the study of the interaction of various mutant forms of the inventive CLA2 polypeptides on the onset of the clinical symptoms of disease associated with the regulation of cell proliferation and differentiation. All the applications that have been herein before discussed with regard to a transgenic animal also apply to animals carrying two, three or more transgenes. It might be also desirable to inactivate the inventive CLA2 protein expression or function at a certain stage of development and/or life of the transgenic animal. This can be achieved by using, for example, tissue specific, developmental and/or cell regulated and/or inducible promoters which drive the expression of, e.g., an antisense or ribozyme directed against the RNA transcript encoding the inventive CLA2 encoding mRNA; see also supra. A suitable inducible system is for example tetracycline-regulated gene expression as described, e.g., by Gossen and Bujard (Proc. Natl. Acad. Sci. 89 USA (1992), 5547-5551) and Gossen et al. (Trends Biotech. 12 (1994), 58-62). Similar, the expression of the mutant inventive tumour associated protein may be controlled by such regulatory elements.

Furthermore, the invention also relates to a transgenic mammalian cell which contains (preferably stably integrated into its genome or transiently introduced) a CLA2 nucleic acid molecule according to the invention or part thereof, wherein the transcription and/or expression of the nucleic acid molecule or part thereof leads to reduction of the synthesis of an inventive tumour associated marker protein. In a preferred embodiment, the reduction is achieved by an anti-sense, sense, ribozyme, co-suppression and/or dominant mutant effect. "Antisense" and "antisense nucleotides" means DNA or RNA constructs which block the expression of the naturally occurring gene product. In another preferred embodiment the native nucleic acid sequence coding for the inventive CLA2 marker polypeptide may be altered or substituted by a variant of said nucleic acid sequence, e.g. by means of recombination, thus rendering the inventive tumour associated marker gene non functional. Thus an organism lacking the inventive tumour associated marker polypeptide activity may be produced according to knock out experiments.

The provision of the CLA2 nucleic acid molecules according to the invention opens up the possibility to produce transgenic non-human animals with a reduced level of the inventive tumour associated marker protein as described above and, thus, with a defect in the regulation of cell proliferation and differentiation. Techniques how to achieve this are well known to the person skilled in the art. These include, for example, the expression of antisense-RNA, ribozymes, of molecules which combine antisense and ribozyme functions and/or of molecules which provide for a co-suppression effect. When using the antisense approach for reduction of the amount of the inventive tumour associated marker proteins in cells, the nucleic acid molecule encoding the antisense-RNA is preferably of homologous origin with respect to the animal species used for transformation. However, it is also possible to use nucleic acid molecules which display a high degree of homology to endogenously occurring nucleic acid molecules encoding a inventive CLA2 protein. In this case the homology is preferably higher than 75%, 80% or 85%, particularly higher than 90%, 91%, 92%, 93% or 94% and still more preferably higher than 95%, 95,5%, 96%, 96.5%, 97%, 97.5%, 98%, 98.5%, 99% or 99.5%. The reduction of the synthesis of a polypeptide according to the invention in the transgenic mammalian cells can result in an alteration in, e.g., degradation of endogenous proteins. In transgenic animals comprising such cells this can lead to various physiological, developmental and/or morphological changes.

Thus, the present invention also relates to transgenic non-human animals comprising the above-described transgenic cells. These may show, for example, a deficiency in regulation of cell proliferation and/or differentiation compared to wild type animals due to the stable or transient presence of a foreign DNA resulting in at least one of the following features:
(a) disruption of (an) endogenous gene(s) encoding the inventive CLA2 marker;
(b) expression of at least one antisense RNA and/or ribozyme against a transcript comprising a CLA2 nucleic acid molecule of the invention;
(c) expression of a sense and/or non-translatable mRNA of the CLA2 nucleic acid molecule of the invention;
(d) expression of an antibody directed against the CLA2 polypeptides of the invention;
(e) incorporation of a functional or non-functional copy of the regulatory sequence of the invention; or
(f) incorporation of a recombinant DNA molecule or vector of the invention.

Methods for the production of a transgenic non-human animal of the present invention, preferably transgenic mouse, are well known to the person skilled in the art. Such methods, e.g., comprise the introduction of a nucleic acid molecule or vector of the invention into a germ cell, an embryonic cell, stem cell or an egg or a cell derived thereof. The non-human animal can be used in accordance with a screening method of the invention described herein and may be a non-transgenic healthy animal, or may have a disorder, preferably a disorder caused by at least one mutation in the inventive CLA2 protein and/or gene. Such transgenic animals are well suited for, e.g., pharmacological studies of drugs in connection with mutant forms of the above described inventive tumour associated marker polypeptide. Production of transgenic embryos and screening of those can be performed, e.g., as described by A. L. Joyner Ed., Gene Targeting, A Practical Approach (1993), Oxford University Press. The DNA of the embryonal membranes of embryos can be analysed using, e.g., Southern blots with an appropriate probe, amplification techniques based on nucleic acids (e.g. PCR) etc.; see supra.

Another aspect of the present invention is a pharmaceutical composition for use in the treatment of disorders associated with abnormal cell proliferation. The CLA2 polypeptides, CLA2 polynucleotides and CLA2 binding agents (esp. antibodies) according to the present invention may be incorporated into pharmaceutical or immunogenic compositions.

The pharmaceutical compositions may be administered by any suitable way known to those of skill in the art. The administration may for example comprise injection, such as e.g., intracutaneous, intramuscular, intravenous or subcutaneous injection, intranasal administration for example by aspiration or oral administration. A suitable dosage to ensure the pharmaceutical benefit of the treatment should be chosen according the parameters, such as age, sex, body weight etc. of the patient, known to those of skill in the art.

The pharmaceutical compositions comprise said compounds and a physiologically acceptable carrier. The type of carrier to be employed in the pharmaceutical compositions of this invention, will vary depending on the mode of administration. For parenteral administration, such as subcutaneous injection, the carrier preferably comprises water, saline, alcohol, a lipid, a wax and/or a buffer. For oral administration, any of the above carriers or a solid carrier, such as mannitol, lactose, starch, magnesium stearate, sodium saccharine, talcum, cellulose, glucose, sucrose, and/or magnesium carbonate, may be employed. Biodegradable microspheres (e.g., polylactic glycolide) may also be employed as carriers for the pharmaceutical compositions of this invention. Suitable biodegradable microspheres are disclosed, for example, in U.S. Pat. Nos. 4,897,268 and 5,075,109.

A pharmaceutical composition or vaccine may for example contain DNA, that codes for one or more CLA2 polypeptides according to the present invention. The DNA may be administered in a way that allows the polypeptides to be generated *in situ*. Suitable expression systems are known to those skilled in the art. In another embodiment of the invention the CLA2 nucleic acids may be for example anti-sense constructs. Pharmaceutical compositions may also comprise CLA2 nucleic acid molecules expressible in a mammalian or human host system comprising a viral or other expression system for example an adenoviral vector system.

The CLA2 nucleic acid may also be administered as a naked nucleic acid. In this case appropriate physical delivery systems, which enhance the uptake of nucleic acid may be employed, such as coating the nucleic acid onto biodegradable beads, which are efficiently transported into the cells. Administration of naked nucleic acids may for example be useful for the purpose of transient expression within a host or host cell.

Alternatively the pharmaceutical compositions may comprise one or more polypeptides. The polypeptides incorporated into pharmaceutical compositions may be the inventive CLA2 polypeptide. Optionally the CLA2 polypeptide may be administered in combination with one or more other known polypeptides such as for example enzymes, antibodies, regulatory factors, such as cyclins, cyclin-dependent kinases or CKIs, or toxins.

CLA2 polypeptides of the present invention or fragments thereof, that comprise an immunogenic portion of a inventive tumour associated protein, may be used in immunogenic compositions, wherein the polypeptide e.g. stimulates the patient's own immune response to tumour cells. A patient may be afflicted with disease, or may be free of detectable disease. Accordingly, the CLA2 compounds disclosed herein may be used to treat cancer or to inhibit the development of cancer. The compounds may be administered either prior to or following a conventional treatment of tumours such as surgical removal of primary tumours, treatment by administration of radiotherapy, conventional chemotherapeutic methods or any other mode of treatment of the respective cancer or its precursors.

Immunogenic compositions such as vaccines may comprise one or more polypeptides and a non-specific immune-response enhancer, wherein the non-specific immune response enhancer is capable of eliciting or enhancing an immune response to an exogenous antigen. Any suitable immune-response enhancer may be employed in the vaccines of this invention. For example, an adjuvant may be included. Most adjuvants contain a substance designed to protect the antigen from rapid catabolism, such as aluminium hydroxide or mineral oil, and a non-specific stimulator of immune response, such as lipid A, Bordetella pertussis or Mycobacterium tuberculosis. Such adjuvants are commercially available as, for example, Freund's Incomplete Adjuvant and Complete Adjuvant (Difco Laboratories, Detroit, Mich.) and Merck Adjuvant 65 (Merck and Company, Inc., Rahway, N.J.).

Pharmaceutical compositions and vaccines may also contain other epitopes of tumour antigens, either incorporated into a fusion protein as described above (i.e., a single polypeptide that contains multiple epitopes) or present within a separate polypeptide.

The present invention further provides kits for use in e.g. research or diagnostic methods. Such kits may contain two or more components for performing a scientific or diagnostic assay. Components may be compounds, reagents, containers and/or equipment. One component may be an antibody or fragment thereof that specifically binds to a CLA2 polypeptide. Additionally the kit may contain reagents, buffers or others known in the art as necessary for performing the diagnostic assay. Alternatively the research kit or diagnostic kit may contain nucleotide probes or primers for the detection of CLA2 DNA or RNA. Such a kit should contain appropriate additional reagents and buffers known in the art.

A kit according to present invention comprises:
a) reagents for the detection of the CLA2 molecular marker molecules
b) the reagents and buffers commonly used for carrying out the detection reaction, such as buffers, detection-markers, carrier substances and others
d) a CLA2 marker sample for carrying out a positive control reaction.

The reagent for the detection of the CLA2 marker includes any agent capable of binding to the marker molecule. Such reagents may include proteins, polypeptides, nucleic acids, glycoproteins, proteoglycans, polysaccharides or lipids.

The sample for carrying out a positive control may comprise for example CLA2 nucleic acids in applicable form, such as solution or salt, CLA2 peptides in applicable form, tissue section samples or positive cells expressing the CLA2 molecules.

In a preferred embodiment of the invention the detection of the marker molecules is carried out on the level of polypeptides. In this embodiment the binding agents may be for example antibodies specific for CLA2 or fragments thereof.

In another embodiment of the test kit the detection of CLA2 is carried out on the nucleic acid level. In this embodiment of the invention the reagents for the detection may be for example nucleic acid probes or primers complementary to said CLA2 nucleic acids.

Cell proliferative disorders (herein also referred to as "disorders characterized by abnormal cell proliferation") according to the present invention are disorders characterized by abnormal growth properties of cells or tissues compared to the growth properties of normal control cells or tissues. The growth of the cells or tissues may be for example abnormally accelerated or may be regulated abnormally. Abnormal regulation as used above may comprise any form of presence or absence of non wild-type responses of the cells or tissues to naturally occurring growth regulating influences. The abnormalities in growth of the cells or tissues may be for example neoplastic or hyperplastic. In one preferred embodiment of the invention the tumours are cancers or pre-cancerours conditions of the respiratory tract.

Disorders characterized by abnormal cell proliferation, as used in the context of the present invention, may comprise for example neoplasms such as benign and malignant tumours, carcinomas, sarcomas, leukemias, lymhomas or dysplasias. Tumours may comprise tumours of the head and the neck, tumours of the respiratory tract, tumours of the gastrointestinal tract, tumours of the urinary system, tumours of the reproductive system, tumours of the endocrine system, tumours of the central and peripheral nervous system, tumours of the skin and its appendages, tumours of the soft tissues and bones, tumours of the lymphopoietic and hematopoietic system, breast cancer, prostate cancer, gastrointestinal cancer, colorectal cancer, anogenital cancer etc..

In certain embodiments the disorders are for example adenomas or adenocarcinomas of the colon, disorders of the respiratory tract such as Squamous Cell Lung Carcinoma, Small Cell Lung Carcinoma, Adenocarcinoma of the Lung, Large Cell Lung Carcinoma, Adeno-Squamous Lung Carcinoma, Carcinoid Tumour of the Lung, Broncheal Gland Tumour or (malignant) Mesothelioma, anogenital cancer such as, cervical cancer, vulval cancer, vaginal cancer, cancer of the rectum, cancer of the anus and cancer of the penis.

A sample according to the method of the present invention is any sample, that may contain cells, tissues or body fluid. Furthermore any sample potentially containing the marker molecules to be detected may be a sample according to the present invention. Such samples are e.g. blood, plasma, serum, liquor, bone marrow, swabs, washes, secretions, transsudates, exudates, sputum, stool, urine, semen, cell- and tissue-samples, punctuates or biopsies.

Biopsies as used in the context of the present invention may comprise e.g. resection samples of tumours, tissue samples prepared by endoscopic means or needle biopsies. Furthermore any sample potentially containing the marker molecules to be detected may be a sample according to the present invention.

In one embodiment of the present invention samples comprise cells of the anogenital tract, of the respiratory tract, the gastrointestinal tract or of the skin and its appendages. In certain embodiments the cells may be cells of the uterine cervix, the vagina, the vulva, the penis, the anus, the rectum, the bronchic tree, the lung, the peritoneum, the peritoneal space, the naso-pharyngeal space, the oral cavity, the colon ascendens, the colon transversum, the colon descendens, the colon sigmoidum, the pancreas, the small intestine, the duodenum, the jejunum, the ileum, the caecum, the esophagus, the stomach, the bile tree, the liver or the skin.

In certain embodiments of the present invention the sample may be a histological sample, a biopsy, or a cytological sample such as e.g. a smear, a swab, a wash, a body fluid containing cells (sputum, a secretion, saliva, etc.). In certain embodiments of the present invention the samples may comprise cells infected by papilloma virus. The samples may in certain embodiments comprise cervical smears, bronchioalveolar lavages, stool, samples obtained by endoscopic means such as e.g. gastroscopy, colonoscopy, bronchioscopy etc.

Preparation of a sample may comprise e.g. obtaining a sample of a tissue, of a body fluid, of cells from a patient. According to the present invention preparation of the sample may also comprise several steps of further preparations of the sample, such as preparation of dissections, preparation of cell suspensions, spreading or applying the cells to be examined onto microscopic slides, preparation of tissue arrays, isolation of polypeptides or nucleic acids, preparation of solid phase fixed peptides or nucleic acids or preparation of beads, membranes or slides to which the molecules to be determined are coupled covalently or non-covalently.

A sample for use in the method of the present invention may be obtained and prepared by any suitable procedure. The samples e.g. may comprise any samples of the content of the gastrointestinal tract (e.g. the stomach, the oesophagus or the bowel) of the respiratory tract (e.g. of the naso-pharyngeal space, the bronchus or the bronchioles), the anogenital tract (e.g. the vagina), the urogenital tract (e.g. the bladder or the urethra) the vascular system etc. The sample may be obtained by active excretion of the material by an individual, or may be may obtained in the course of a surgical, invasive or minimally invasive medical procedure. For example a stool sample, as used in the context of the present invention may be a sample of material contained in the lumen of the colon, which has been obtained by clysters, by colonoscopy, digitally from the rectum or may be obtained from a stool voided by a patient. A stool sample according to the present invention may, but need not contain cells or cell debris of colorectal origin. In one embodiment of the present invention the polypeptides used for the detection of the colorectal lesions are secreted proteins that may be detected in stool samples independent of the presence of cells or cell debris originating from a colorectal lesion within the sample. In one embodiment of the present invention the sample may comprise blood, lymph, lymph nodes, bone marrow etc. In another embodiment of the present invention the sample may comprise breast tissue, breast cells, nipple aspirates, ductal lavages or any sample containing cells or cell debris originating from the breast.

In certain embodiments of the present invention sample may refer to the respective material within the body of an individual such as the urine, the stool, the sputum etc. A sample in this context may be e.g. the content of the intestine of an individual *in vivo*. In this embodiment the (stool, urine, sputum, exudates, semen, secretion) sample need not to be separated from the patient to be subjected to the methods disclosed herein.

The method for preparation of the sample may comprise any method suitable for assuring accurate detection of the presence or absence of lesions associated with abnormal growth properties. In certain embodiments of the present invention the preparation of the samples may comprise e.g. picking any portion of a total samples (such as e.g. voided stool, excreted urine, obtained smear, wash, sputum) with any suitable means such as a spatula, a brush, a spoon, a tip, a cloth, a membrane, a capillary, a syringe, a needle or pin or the like. For example the sample preparation may comprise blotting of a portion of the sample (such as e.g. the surface of a stool) to a membrane, a foil, a plastic film or a cloth, picking a portion of the stool by means of a needle, syringe, capillary, spatula, spoon or the like. Sample preparation may in certain embodiments comprise swabs from the surface of solid or viscous samples (such as e.g. voided stool, certain secretions, etc) obtained by suitable means like a spatula, a brush, a tampon, a cloth, a porous or textile (cotton, cellulose, derivatized cellulose etc.) device or tip or any other suitable means.

In certain embodiments of the invention any random fraction of a sample may be suitable for performing the detection method disclosed herein. In certain other embodiments of the invention a sample may be prepared in a way to assure the presence of a representative portion of the total sample. Such representative portions may be e.g. obtained by the methods described in US6303304, which shall be incorporated herein by reference, for stool samples.

In certain special embodiments of the present invention the sample may be prepared as a monolayer or thin layer preparation of a cytological specimen. The respective methods for preparation of monolayer or thin-layer preparation in cytology are known to those of skill in the art. In one embodiment the preparation may e.g. comprise the ThinPrep technology. Other methods comprise conventional smears, or method employing suspensions of cells for preparation of the cytological specimens.

In certain embodiments of the present invention the a procedure for enriching or purifying the polypeptides and/or polynucleotides of interest may be employed. Where the case may be the identification of nucleic acids, proteins or peptides in complex samples, which comprise several nucleic acids, proteins or peptides, may be enhanced by a separation procedure of particular molecule species present in the sample. These purification processes may involve purification in the meaning of separating all nucleic acids, protein or peptide components of the sample from other components such as lipids, nucleic acids etc.. In certain embodiments of the invention the purification may also involve the separation of nucleic acids and/or proteins or peptides of particular properties from other protein or peptide components within the mixture.

Generally the methods for purification of nucleic acids and polypeptides mentioned herein may be applied in the course of any detection procedure suitable for the detection of the CLA2 molecules of the present invention. Thus as the case may be any detection procedure for the detection of the marker molecules disclosed herein alone or combination with other marker molecules may comprise purification methods for nucleic acids and/or polypeptides as mentioned below. The purification process may be performed at any stage in the course of the total procedure e.g. before a detection or amplification reaction, subsequently to a detection or amplification reaction, in a single step reaction simultaneously to a detection or amplification reaction etc.

Separation of proteins and/or nucleic acids may be carried out by use of their physical, chemical or biological properties. Physical parameters used for separation may comprise charge, hydrophobicity, mass, volume, shape or any other physical parameter suitable for separating different protein or peptide species. Chemical parameters applicable in separation of proteins comprise the use of reactive groups such as hydroxyl-, sulfhydryl or any other reactive or non reactive structure suitable for the separation of proteins/peptides. Biological parameters which may be used for separating proteins may include enzymatic activity, molecular interactions such as e.g. binding of biological binding moieties, such as e.g. ligands or receptors, immunogenicity or any other biological property suitable for separating different proteins or peptides. With respect to nucleic acids biological parameter may especially pertain to hybridisation properties.

All parameters mentioned above may be used independently or in any combination suitable for separating and purifying nucleic acids, proteins and/or peptides. In one embodiment a complex sample may be separated by electrophoretic methods such as agarose gel electrophoresis, PAGE, SDS-PAGE, free flow electrophoresis, capillary electrophoresis, 2D-electrophoresis or any other electrophoretic method suitable for separating nucleic acids, proteins or peptides. In certain embodiments 2D-electrophoresis may be used such that the separation in the first dimension is based on charge (e.g. in a polyacrylamide gel under high voltage conditions) and the resulting separated proteins or peptides are separated by their mass (e.g. in a sodium dodecylsulfate polyacrylamide gel in a direction perpendicular to the first dimension). Alternatively the fist dimension of separation of proteins or peptides may be achieved by isoelectric focussing of the molecules in a pH gradient under high voltage. In certain embodiments a pulsed field electrophoresis may be applied for the separation of the CLA2 molecules according to the present invention.

In certain further embodiments capillary electrophoresis may be used for the separation of complex mixtures. For example a capillary may be filled with a suitable separation medium such as e.g. polyacrylamide and the sample is put on one end (depending on the positioning of the capillary e.g. the top) of the capillary. Depending on buffer and gel conditions the proteins and or peptides in the sample may be separated by mass or charge, respectively.

Furthermore liquid chromatography may be applied for separating nucleic acids, proteins and/or peptides. Macromolecules such as nucleic acids, peptides and/or proteins may be separated according to their physical and or chemical behaviour and or biological behaviour and or combinations of these depending on the chromatographic media and or solvents used for chromatography. In one embodiment the complex mixture of proteins and/or peptides may be bound to a solid phase according to their charge and eluted separately by increasing salt concentrations. In one embodiment the complex mixture is separated by a solid phase according to their mass by using a solid phase with a defined pore size distribution where proteins and or peptides enlarge their flow rate by diffusion into the pores according to their mass. In one embodiment the complex mixture of proteins and or peptides is separated by a solid phase according to their shape by using a solid phase with a defined pore shape and or pore size distribution where proteins and or peptides enlarge their flow rate by diffusion into the pores according to their shape. In one embodiment the complex mixture of proteins and or peptides is bound to a solid phase according to their hydrophobicity and eluted separately by applying a gradient of hydrophobic solvents. In one embodiment one or all chromatographic methods mentioned above combined in a manner which is suitable for separating complex mixtures of proteins and or peptides.

In certain embodiments two dimensional HPLC may be used for separation of nucleic acids, proteins and/or peptides. For example a separation by means of ion exchange columns may be applied in combination with a reversed phase column. The ion exchange column may for example be an anion or cation exchange column of a suitable strength for use in the method disclosed herein. Materials for use in these HPLC methods are known to those of ordinary skill in the art. In certain embodiments the eluates from the first column (eluted e.g. by means of increasing salt steps) may be loaded onto the reversed phase column. The reversed phase column may e.g. be eluted by an ascending gradient of an appropriate solvent (e.g. acetonitril).

In certain embodiments of the present invention a pre-treatment of the separated macromolecules such as e.g. proteins or polypeptides may be applied prior to the detection reaction. Such procedures may for example employ reduction or oxidation of proteins or peptides, proteolytic cleavage, modification of the proteins or peptides, derivatization or application of protecting moieties to prevent reactive parts of the peptides from unwanted reactions. For example in certain embodiments sulfhydryl-groups may be prevented from oxidation. Generally a protein extract for use in the methods according to the present invention may but need not be digested with suitable enzymes such as trypsin or any other protease to prepare peptides suitable for detection.

By treatment with proteolytic enzymes the following peptides may be obtained from the CLA2 proteins as main products:

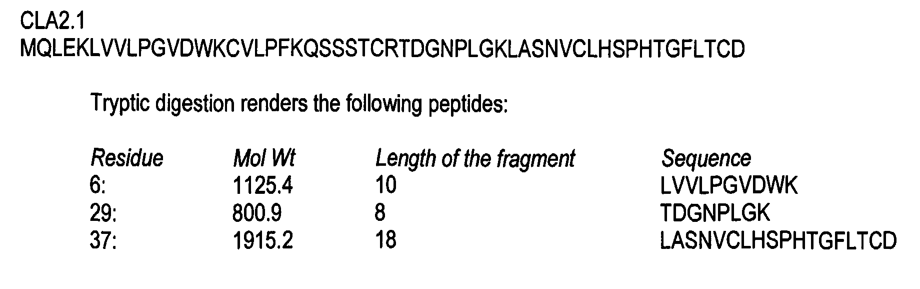

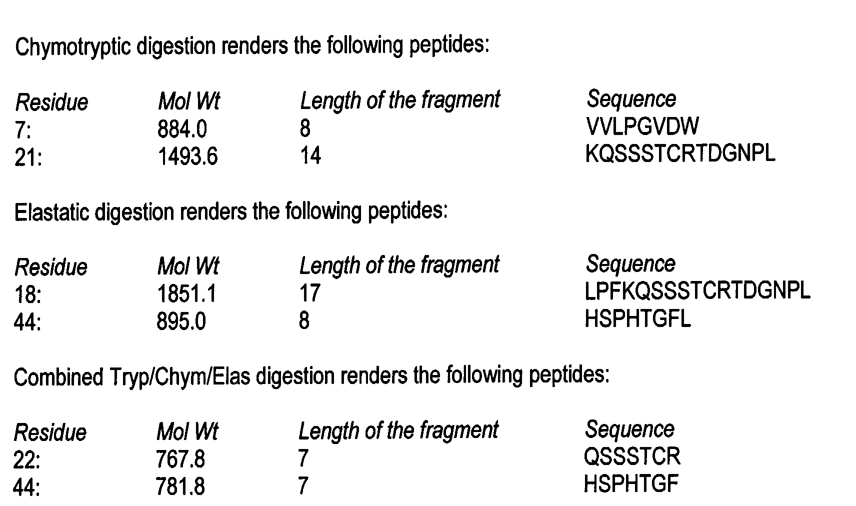

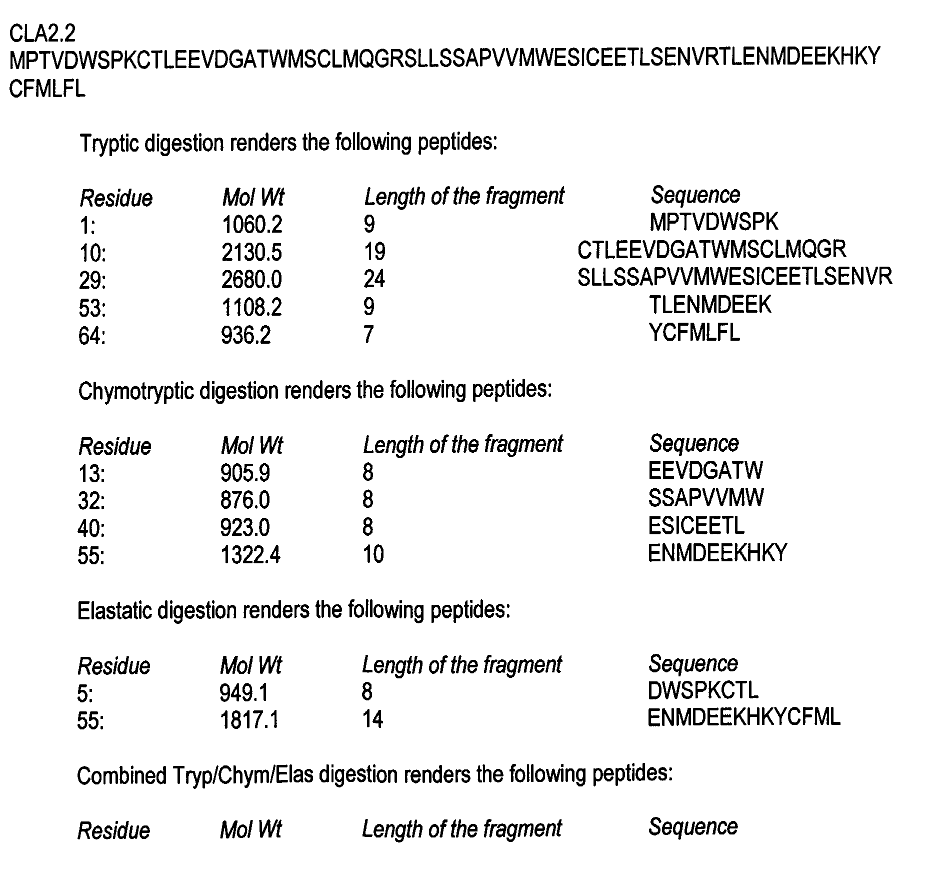

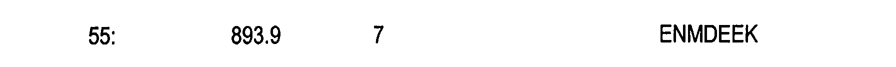

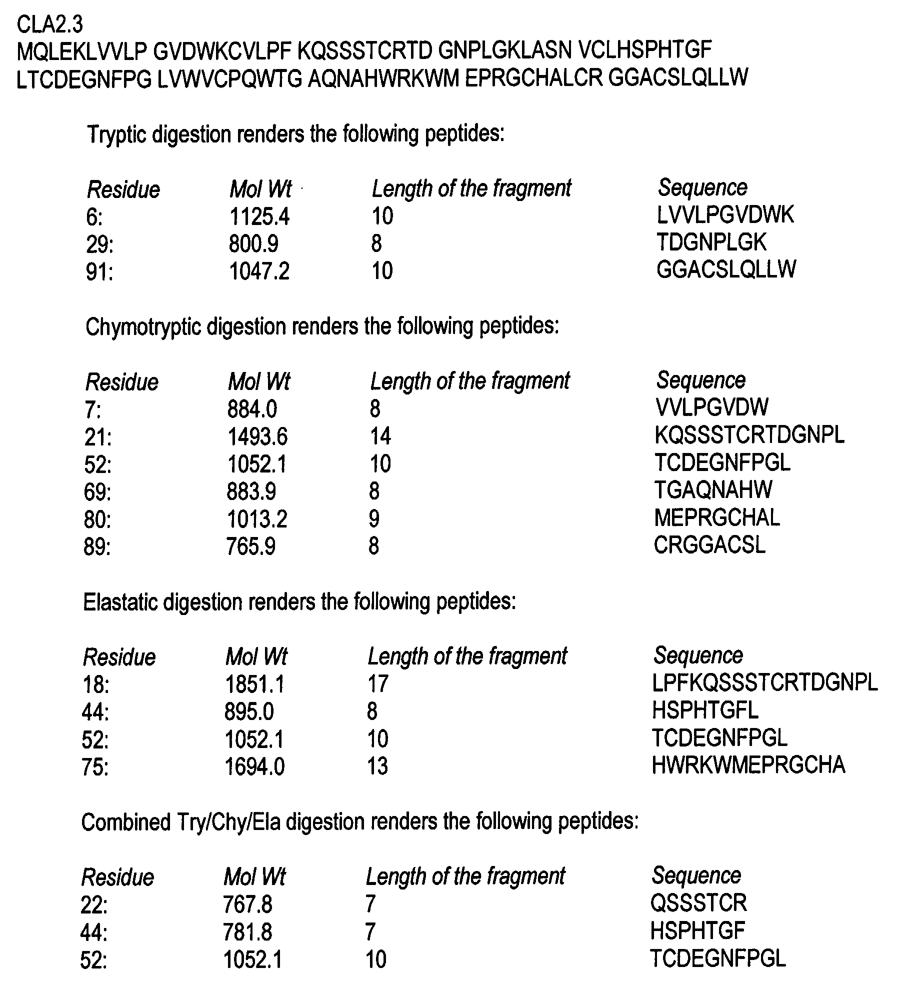

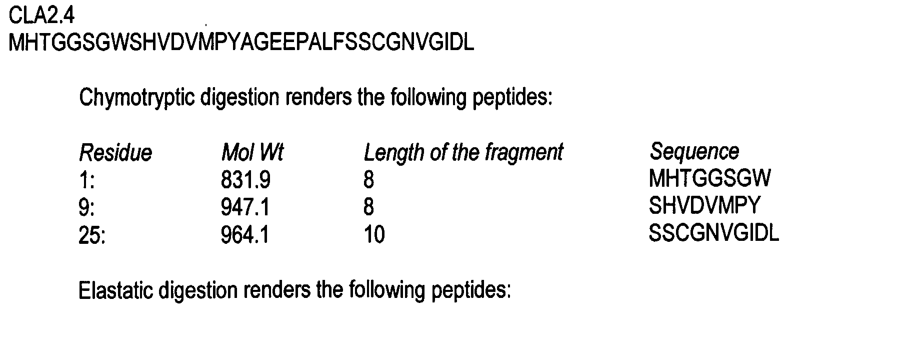

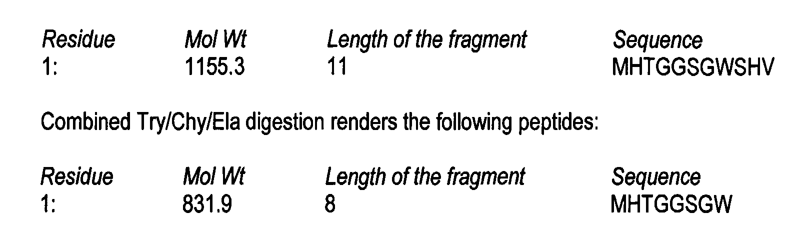

Peptides CLA2.5 - CLA2.7 do not render fragments upon digestion with the mentioned proteolytic enzymes. The peptides themselves are detectable after subjection of a sample to proteolytic conditions.

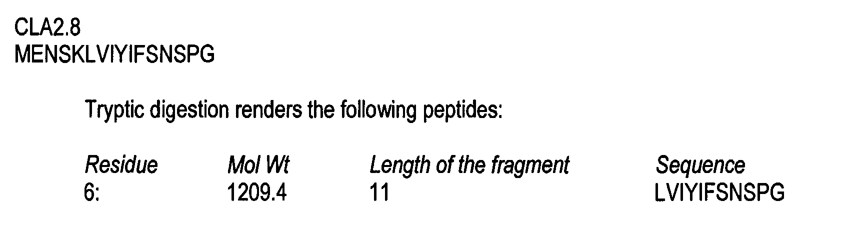

In certain embodiments of the present invention one or more of the named fragments may be present without subjecting the sample to any steps of sample preparation. This may be due to activity of (digestive) proteolytic enzymes within the sample such as e.g. in stool, in fluids of the gastrointestinal tract or in gastrointestinal secretions. The named fragments may be detected by any means describes herein. In one embodiment the fragments may be detected in the course of a mass spectrometric analysis. Detection of the respective fragment peaks corresponding to the named peptides derived from CLA2 may be especially useful for the detection of the presence or absence and/or the level of CLA2 Proteins in samples.

In one embodiment of the present invention nucleic acids may but not need to be subjected to a purification process prior to the a subsequent detection or amplification reaction. This may be desirable e.g. to further enhanced signal to noise ratios. Furthermore purification of nucleic acids may be also applied subsequently to amplification reaction as the case may be.

Purification techniques for the purpose of purification of nucleic acids are known to those of ordinary skill in the art and comprise for example gel electrophoresis, chromatography, precipitation, ultra centrifugation etc.. E.g. the nucleic acids may be purified using electrophoresis in a suitable solid, viscous or liquid medium, such as a gel made from substances known to those of skill in the art (agarose, polyacrylamid, starch etc.).

Alternatively nucleic acids may be purified using hybridisation of the nucleic acids to complementary or reverse-complementary nucleic acid probes (e.g. fixed to a solid phase such as beads, membranes, slides etc.) in the procedure of an affinity chromatography or other suitable capture formats. Additionally precipitation methods for precipitation of nucleic acids (e.g. using ethanol, isopropanol or other alcohol in appropriate concentration, trichloroacetic acid, or other suitable acids or any other agent suitable for precipitation of nucleic acids from solutions) may be applied for the purification as well as chromatographic methods, such as ion exchange chromatography, affinity chromatography etc. Acc0ording to the present invention nucleic acids may be purified using ultra centrifugation techniques, such as density gradient centrifugation in e.g. an isokinetic or isopyknic manner or other suitable centrifugation techniques.

Generally a method for detection of the level of the marker molecules for use in the methods according to the present invention is any method, which is suited to detect and identify biological macromolecules such as nucleic acids, peptides and protein molecules in samples. In certain embodiments of the invention these methods may be methods exhibiting high sensitivity, so that even small amounts of molecules may be detected. In further embodiments of the present invention standard detection methods exhibiting suitable sensitivities may be employed. Any method may be employed such as e.g. those including detection reactions in solution, methods employing solid phase adsorbed or coupled agents, etc. The method may be *in vitro* methods or may be methods to be applied *in vivo* e.g. in the course of *in vivo* imaging procedures.

In certain embodiments more than one peptide derived from CLA2 marker polypeptides and/or polynucleotides will be determined in a detection procedure. The detection of the level of marker polypeptides or fragments thereof according to the present invention may be the detection of the level of single marker molecules in separated reaction mixtures as well as the detection of a combination of markers simultaneously.

Furthermore the detection of the marker CLA2 nucleic acids, polypeptides and/or polynucleotides as disclosed herein may be carried out in combination to one or more further detection reactions. These detection reaction may for example be reaction for determination of the presence of further suitable marker nucleic acids and/or polypeptides or of one or more nucleic acids marker molecules in the samples. Further marker molecules, that may be suitable for the detection proliferative disorders in the course of a method as disclosed herein may comprise e.g. cyclins (Cyclin A, Cyclin, B, Cyclin E), cyclin-dependent kinase inhibitors (p13.5, p14, p15, p16, p18, p19, p21, p27 etc.), cyclin-dependent kinases (cdk2, cdk4, cdk6 etc.) cell cycle regulatory proteins (p14ARF, pRb, mdm2, p53), proliferation marker molecuoles (mcm2, mcm3, mcm4, mcm5, mcm6, mcm7, cdc2, cdc6, Ki67, Ki-S2, PCNA, DNA polymerase delta, rF Kappa B, etc.), marker for viral infection (such as hBV, hPV (especially high risk HPV: 16, 18, 31, 33, 38, 44, 45, 58, 68, etc.), hIV etc.) or other tumour marker proteins or nucleic acids (e.g. her2neu, CEA, PSA etc.).

The detction of one or more molecular markers may be performed in a single reaction mixture or in two or separate reaction mixtures. The detection reactions for several marker molecules may for example be performed simultaneously in multi-well reaction vessels. The CLA2 nucleic acids and/or polypeptides disclosed herein may be detected using methods and/or reagents that specifically detect these molecules. Simultaneously one or more further markers may be detected using methods and/or reagents that specifically detect them. The detection procedure for each single marker may comprise one or more steps. In certain embodiments the detection procedure may comprise detection of the marker molecules by a primary detecting step followed by a further secondary detecting step making the result of the procedure available for quantitative and/or qualitative analysis. Examples of detection procedures involving multiple steps may e.g. comprise the use of primary and secondary and further binding agents.

In certain embodiments the detection procedure further may comprise a reporter reaction indicating the level of the inventive CLA2 polypeptides and/or nucleic acids. The reporter reaction may be for example a reaction producing a coloured compound, a bioluminescence or chemiluminescence reaction, a fluorescence reaction, generally a radiation emitting reaction, or a reaction involving a chemical binding reaction such as biotin binding or metal chelate binding.

In certain embodiments of the present invention procedures for the detection reaction according to the present invention may employ for example any immunological methods for detection of molecules, such as for example Western blot, dot blot, immuno-precipitation or immunological assays, such as ELISA, RIA, lateral flow assays etc..

In these embodiments determination of the (CLA2) marker nucleic acids and/or polypeptides may for example be carried out in a reaction comprising a binding agent specific for the detection of the marker molecules. These binding agents may comprise for example nucleic acid probes, antibodies and antigen-binding fragments, bifunctional hybrid antibodies, peptidomimetics containing minimal antigen-binding epitopes etc. The binding agents may be used in many different detection techniques for example in southern-, northern-, western-blot, ELISA, lateral flow assay, (hybrid) capture assay, latex-agglutination, immuno-chromatographic strips or immuno-precipitation. Generally binding agent based detection may be carried out as well *in vitro* as directly *in situ* for example in the course of an immuno-cytochemical staining reaction. Any other method suitable for determining the amount of particular polypeptides in solutions of biological samples, such as biochemical, chemical, physical or physico-chemical methods, may be used according to the present invention.

Methods for detection of methylation of nucleic acids are known to those of skill in the art and may comprise for example methods employing chemical pre-treatment of nucleic acids with e.g. sodium bisulphite, permanganate or hydrazine, and subsequent detection of the modification by means of specific restriction endonucleases or by means of specific probes e.g. in the course of an amplification reaction. The detection of methylation may furthermore be performed using methylation specific restriction endonucleases.

In one embodiment of the invention the detection of the level of marker molecules is carried out by detection of the level of nucleic acids coding for the marker molecules or fragments thereof present in the sample. The means for detection of nucleic acid molecules are known to those skilled in the art. The procedure for the detection of nucleic acids can for example be carried out by a binding reaction of the molecule to be detected to complementary nucleic acid probes, proteins with binding specificity for the nucleic acids or any other entities specifically recognising and binding to said nucleic acids. This method can be performed as well *in vitro* as directly *in situ* for example in the course of a detecting staining reaction. Another way of detecting the marker molecules in a sample on the level of nucleic acids performed in the method according to the present invention is an amplification reaction of nucleic acids, which can be carried out in a quantitative manner such as for example PCR, LCR or NASBA.

In certain embodiments of the present invention amplification of ribonucleic acids or of deoxyribonucleic acids may be applied to detect small amounts of CLA2 marker molecules or of small amounts of cells expressing CLA2 marker molecules in samples. This may be especially useful for the detection of dispersed tumour cells in samples, or for detection of CLA2 molecules, that have been dispersed to body fluids from tumour cells, that expressed these CLA2 molecules. Generally the detection of metastases, minimal residual disease or disseminated tumour cells in body samples may comprise a nucleic acid amplification reaction as mentioned above.

In the course of the detection of minimal residual disease generally detection of CLA2 molecules such as peptides, proteins, DNA or mRNA in blood samples or the detection of disseminated cells may be suitable. In the course of the detection of CLA2 molecules an amplification reaction (e.g. PCR, LCR, NASBA) may be employed. In the course of the detection of disseminated tumour cells may be separated from a body liquid and after lysing of the cells CLA2 molecules may be detected in the lysate. In certain embodiments of the present invention the detection of the dispersed tumour cells may be carried out in lymph node samples or in bone marrow samples to detect metastases or disseminated tumour cells, that have spread to the respective samples. It must be understood, that in the course of a detection of disseminated tumour cells any sample obtainable from an individual may be useful.

Alternatively for the purpose of detection of disseminated tumour cells, metastases or minimal residual disease mass spectrometric detection of nucleic acids may be applied subsequently to amplification, or may be applied without amplification reaction.

In another embodiment of the invention the detection of the level of marker molecules is carried out by determining the level of expression of a protein. The determination of the marker molecules on the protein level may for example be carried out in a reaction comprising a binding agent specific for the detection of the marker molecules. These binding agents may comprise for example antibodies and antigen-binding fragments, bifunctional hybrid antibodies, peptidomimetics containing minimal antigen-binding epitopes etc. The binding agents may be used in many different detection techniques for example in western-blot, ELISA, lateral flow assay, latex-agglutination, immuno-chromatographic strips or immuno-precipitation. Generally binding agent based detection may be carried out as well *in vitro* as directly *in situ* for example in the course of an immuno-cytochemical staining reaction. Any other method suitable for determining the amount of particular polypeptides in solutions of biological samples, such as biochemical, chemical, physical or physico-chemical methods, can be used according to the present invention.

In certain embodiments of the present invention mass spectrometry may be used for detection of the CLA2 marker nucleic acids and/or polypeptides. Generally any type of mass spectrometry may be used in the method according to the present invention. The molecules to be analysed may be ionised by any suitable method. In one embodiment the ionisation method in the course of mass spectrometry may be a matrix assisted laser desorption ionisation, fast atom bombardment ionisation, electron spray ionisation or any other suitable method. Any technique known in the art for mass spectrometric resolution and detection of the generated ions may be used in the method according to the present invention. The mass spectrometric analysis may for example be performed by means of a time of flight analyser, may employ an ion trap, a quadrupole, a sector field analyser, a cyclotron etc..

The complete analysis including 2D-HPLC and mass spectrometric identification may for example be carried out on the ProteomeX-Workstation (ThermoFinnigan, San Jose, CA, USA). The system includes a HPLC system comprising two HPLC pumps and an auto sampler which are connected to independently provide solvent to a strong cation exchange column and a reversed column. In the first step the tryptic digest is loaded onto the strong ion exchange column and washed using a appropriate solvent to remove any contaminations not suitable for further analysis. In increasing salt steps beginning with 1mM ammonium chloride and ending at 900mM ammonium chloride fractions of the proteolytic peptides are eluted from the strong cation exchange column and loaded onto the reversed phase column. Using the second HPLC pump the peptides on the reversed phase column are eluted by an ascending acetonitril gradient from 5% to 80% acetonitril in water after washing the bound peptides to remove access salt and conditioning the peptides for the subsequent mass spectrometric analysis. The peptides eluting from the reversed phase column are measured on line by use of a electrospray ionisation ion trap mass spectrometer (DECA LCQ, ThermoFinnigan, San Jose, CA, USA) which enables the direct analysis and fragmentation of eluted peptides. Each reversed phase run is monitored continually by the ESI-MS and ESI-MS/MS spectra and saved for subsequent protein identification with the SEQUEST software package. SEQUEST uses peptide fragmentation mass spectra retrieved during the data dependent MS/MS process which generated fragment mass spectra of eluted peptides. The SEQUEST algorithm links experimentally derived fragment spectra with *in silico* generated fragment spectra from databases and enables correlation of the experimentally derived spectrum to the appropriate database record which identifies the peptide matching to this record.

The fragments detected during the MS analysis may be identified by comparison of these fragments to the data obtained from a database. Using appropriate algorithms proteins may be identified according to the fragment data obtained from the MS analysis.

In one embodiment the peptide fragments obtainable by proteolytic cleavage of the CLA2 proteins as depicted in SEQ NO 45 - 87 may be especially useful for the detection of CLA2 proteins in samples. Detection of the presence or the level of CLA2 proteins in samples may in one embodiment of the present invention comprise the detection of the presence or absence and/or the level of one or more of the named proteolytic fragment peptides derived from CLA2 proteins in a sample. In one embodiment the detection may comprise the detection of the respective fragment peaks in a mass spectrum or in a complex pattern of different peptide fragment signals obtainable by a suitable analytical method.

In certain embodiments the separation of the proteins, subsequent analysis of the proteins and peptides and final identification of the proteins according to the detected mass spectra may be carried out in an assembled process.

Another technique suitable for peptide identification in complex samples uses 2D-Electrophoresis instead of 2D liquid chromatography. Gel spots stained with coomassie brilliant blue can be cut from the gel and digested using trypsin. The subsequent identification of both single peptides as well as the "mass fingerprint" of the digested protein can be performed using matrix assisted laser desorption and ionisation mass spectrometry or electrospray ionisation mass spectrometry.

For detection in mass spectrometry of nucleic acids purified nucleic acids may be subjected to amplification reactions. Suitable amplification reactions are known to those of ordinary skill in the art and may comprise DNA based amplification as well as RNA based amplification. Amplification reactions according to the present invention may comprise PCR, LCR, NASBA, etc.. The amplification reaction may be performed using one or more specific primers. In one embodiment of the invention an amplification reaction comprises the amplification of a single nucleic acid. In another embodiment of the invention the amplification is carried out as a multiplex amplification reaction simultaneously amplifying a set of several nucleic acids.

In one embodiment of the present invention the amplified nucleic acids may be used for a subsequent primer extension reaction, with or without prior purification which gives rise to nucleic acid fragments of 10 to about 50 bp length.

In certain embodiments of the present invention immunological entities directed against CLA2 may be detected. This detection reaction may be carried out in the course of detection of disorders associated with the expression of CLA2 molecules or in the course of a immuno-therapy for determination of the immuno-status of an individual or for monitoring of the effect of an immunization or vaccination therapy.

In one embodiment of the invention the detection of the level of immunological entities specific for CLA2 peptides is carried out on the level of antibodies. The method for detection of disorders according to the present invention thus may employ the detection of immunological entities directed against one single peptide or the detection of a set of immunological entities. The use of a multiplicity of potential peptides raises the probability to detect the presence of a particular disorder, and may furthermore give additional information useful in stratification of a disorder, in monitoring the disease course or in assessment of prognosis concerning the disease course.

Immunological entities as used in the context of the present invention shall comprise any components of the mammalian immune system, that are able to specifically react with an antigenic epitope. Such immunological entities may comprise for example antibodies, all immuno-globulins, such as e.g. IgG, IgM, IgA, IgE, IgD, specific CD8+ T-cells or specific T-helper cells.

In this embodiment the detection may be e.g. performed using the specific interaction between the respective CLA2 peptides with the antibodies. The determination of the presence or absence and/or the level of antibodies directed against CLA2 peptides in an individual may for example be carried out with recombinantly produced CLA2 peptides. The peptides can be used in many different detection techniques for example in western-blot, ELISA or immuno-precipitation. In one embodiment the detection of antibodies is carried out as antibody capture assay (Antibodies A laboratory Manual, Harlow, Ed. et al., Cold Spring Harbor Laboratory 1988).

In another embodiment of the invention the detection of the specific antibodies is carried out using monoclonal or polyclonal antibodies specifically recognizing the antigen binding epitope of the first antibodies. For this purpose the above mentioned immunological detection procedures may be applied. In a further embodiment chimeric antigens may be employed in the detection reaction. Such chimeric antigens may for example comprise fusion proteins combining the antigenic epitope of a tumour associated polypeptide, recognized by the antibody in question, fused to another antigen, that may be recognized by a detection antibody. The particular antigens within the chimeric polypeptide may be separated by a linker or spacer region.

Any other method for determining the amount of particular antibodies or immuno-globulins in biological samples can be used according to the present invention.

Generally the detection of the antibodies according to the present invention may be performed as well *in vitro* as directly *in situ* for example in the course of an immuno-histochemical or immuno-cytochemical staining reaction.

In one embodiment of the present invention the immunological entities directed against CLA2 molecules may be detected in a skin test. In this testing format peptides of CLA2 may be introduced intradermally into the skin of individuals *in vivo*. The testing format is known to those of skill in the art from the so called TINE test or the SERO test stamp by Sero-Mérieux. In this test the presence of immunological entities directed against CLA2 molecules is diagnosed from a reaction of the individual visible e.g. as inflammation of the skin at the respective point of injection of the peptide. The evaluation thus relies on reddening of the skin and e.g. formation of reddish papules etc. on the skin. The test result may be dependent e.g. on the diameter of the reddening or the papules detectable after application of the antigens. The test result may be recorded e.g. by photo-documentation.

The peptides (also called in this context "antigens") applicable for the detection of immunological entities in individuals may be produced by any method known to those of ordinary skill in the art and may comprise for example chemical synthesis of the polypeptides (fmoc-synthesis or equivalent) or may be produced recombinantly in any suitable host. However it must be obeyed, that the peptides are free of immunogenic components other than the respective CLA2 derived peptides.

The amounts of peptides that must be applied in this testing format in order to render visible immuno-reaction ranges between 0,1µg and 10µg of the purified peptide. In certain embodiments 0.05µg, 0.1µg, 0.5µg, 1µg or 5µg of the antigen or any value in between may be used per application. The antigens (peptides) are preferably applied as solution (whereas any other suitable format of application such as powder, aerosol or the like may be used according to the present invention as well). The solvent may be any medically acceptable solution being sterile and free of pyrogens, that does not cause inflammatory or immunogenic reaction, when applied in a testing format as used in the presented skin test.

Cells exhibiting specificity for a CLA2 antigen may be detected by any methods suitable for that purpose known to those of ordinary skill in the art. Methods may for example comprise proliferation-assays, cytokine-ELISAs, ELISpot assays, intracellular FACS-staining, PCR-mediated identification of peptide-specific cytokine (or similar) -expressing cells, tetramer-staining, cytotoxicity assays and DTH-(delayed type hypersensitivity) reactions.

In case of proliferation-assays induction of peptide-specific T-cell proliferation may be measured by methods known to those of skill in the art. This can be achieved by simple counting of cells, by measuring incorporation of labelled nucleotides into cellular DNA or by measuring level and/or activity of cellular protein(s). Cytokine-ELISA may comprise identification of peptide-specific cytokine-secreting cells by measuring cytokine levels in supernatant. In the course of an ELISpot assay the number of peptide-specific cytokine (i.e. IFN-g)- secreting cells in a sample is determined. Similarly the intracellular FACS-staining identifies cytokine-expressing cells on the protein level. In contrast (real-time) PCR may be used for identification of peptide-specific cytokine (or similar) - expressing cells on the transcript level. In the course of a tetramer-staining assay the label is a tetramer-molecule of recombinant MHC-class I molecules, loaded with specific peptide and coupled to a dye. The tetramer binds to the T-cell receptor. Cytotoxicity assays are a method for identification of cells, that can recognize and kill target cells in a peptide-specific manner. DTH-(delayed type hypersensitivity) reaction is based on the measuring of skin-reaction of vaccinated persons after intradermal (or similar) application of peptide(s).

The method for the detection of immunological entities as disclosed herein may be performed for the purpose of monitoring in the course of immuno-therapeutic treatments of individuals. In this respect the presence or absence and or the level of antibodies directed against an inventive peptide in an individual is determined. The determination of the level may be performed using the methods as se forth above. The detection may be performed at several consecutive points in time as to monitor the timely alteration of the level of the immunological entities. The determination may for example be performed daily, weekly, monthly, once a year, or in once in a decade or at any interval in between.

In one preferred embodiment of the invention the level of markers is significantly elevated compared to a non tumourous test sample. In this case the marker is over expressed in the sample. In another preferred embodiment of the present invention the level of the marker is lowered compared to a non tumourous test sample. In a third embodiment there is no detectable expression of the marker at all in the test sample unlike in a control sample. In yet another embodiment there is detectable level of non wild-type marker molecules. Non wild-type marker molecules may comprise any marker molecules that deviate in sequence or structure from the structure or sequence, that is functional in wild type tissue not affected by a cell proliferative disease. Wild type sequences or structures are the sequences or structures predominantly present in normal cells or tissues. In one preferred embodiment of the invention the level of particular splicing variants of the marker gene is altered in the test samples compared to the wild type tissue. This may lead to altered levels of splicing variants, new splicing variants, neo-peptides, altered ratios of different splicing variants of genes.

The detection procedure according to the present invention may furthermore comprise a cytochemical staining procedure rendering a chromogenic or fluorescent staining of cells or cell compartments. Such staining procedures are known to those of skill in the art and may for example comprise e.g. staining for acidophilic or basophilic structures, of sub cellular regions (e.g. the nucleus, the mitochondria, the Golgi, the cytoplasm etc.), of specific molecules (the chromosomes, of lipids, of glycoproteins, of polysaccharides etc.) in the cytological specimens. Fluorescence dyes such as DAPI, Quinacrin, Chromomycin, etc. may be employed. Furthermore chromogenic dyes such as Azan, Acridin-orange, Hematoxylin, Eosin, Sudan-red, Thiazin―stains (Toluidin-blue, Thionin) may be applied. In other embodiments staining procedures such as Pap-staining, Giemsa-staining, Hematoxylin-Eosin staining, van-Gieson staining, Schiff-staining (using Schiff reagent), Feulgen staining, staining procedures employing precipitation of metals (such as e.g. of silver in staining procedures employing Silver Nitrate) or insoluble stains such as e.g. of Turnbulls-blue (or other insoluble metal cyanides), etc. may be used in the course of a method as disclosed herein. It must be understood, that the named dyes and staining methods shall be examples for the applicable methods and that any other method known in the art may be applied to a method as disclosed herein.

The staining procedures may produce chromogenic stains for light microscopic inspection or fluorescent stains for inspection under fluorescence microscopic conditions. In another embodiment of the present invention radiation emitting procedures, procedures employing substances impairing the transmission of radiation or other contrast media for imaging of the cytological conditions in a sample (e.g. the generation of optical impression by means such as (micro-)autoradiographic or (micro-)radiographic picture generation) may be of use for a method according to the present invention.

All the staining and imaging procedures may be used for analysis not only in microscopic procedures but also in automated analysis procedures such flow cytometry, automated microscopic (computerized or computer aided) analysis or any other method for analysis of stained cytological specimens.

The analysis of the staining or imaging results of the different procedures may be performed in a single analysis step or in different subsequent steps. E.g. the light microscopic inspection of a specimen may be performed before or after fluorescence microscopic inspection of the specimen. In Fluorescence microscopy the analysis of different stains with different excitation wavelengths may be analyses simultaneous or subsequently. Other imaging methods may be employed simultaneously or subsequently to the named procedures.

There may be various circumstances, under which combinations of different staining methods will be suitable. E.g. in cases, where no satisfying cytological staining results may be achieved by immuno-chemical staining the additional application of general cytological staining techniques may be suitable.

In certain embodiments of the present invention the method for detection of the marker molecules in samples may be performed in an automated manner. The automation of the method may be achieved by automated staining and analysis of histological or cytological specimens on a solid surface by microscopic means. In another embodiment the automation may comprise a flow-cytometric analysis of the staining of cells in solution.

In one preferred embodiment the detection of tissues expressing CLA2 gene products is carried out in form of molecular imaging procedures. The respective procedures are known to those of ordinary skill in the art. Imaging methods for use in the context of the present invention may for example comprise MRI, SPECT, PET and other methods suitable for *in vivo* imaging.

In one embodiment the method may be based on the enzymatic conversion of inert or labelled compounds to molecules detectable in the course of molecular imaging methods by the marker molecules. In another embodiment the molecular imaging method may be based on the use of compounds carrying a suitable label for *in vivo* molecular imaging, such as radio isotopes, metal ions etc., specifically binding to marker molecules *in vivo*.

In a preferred embodiment of the invention these compounds are non-toxic compounds and may be eliminated from the circulation of organisms, such as humans, in a time span, that allows for performing the detection of label accumulated in tumour tissue over expressing the CLA2 marker gene. In another preferred embodiment of the invention compounds are used for molecular imaging, for which clearance from the circulation is not relevant for performing the molecular imaging reaction. This may be for example due to low background produced by the circulating molecules etc, The compounds for use in molecular imaging methods are administered in pharmaceutical acceptable form in compositions that may additionally comprise any other suitable substances, such as e.g. other diagnostically useful substances, therapeutically useful substances, carrier substances or the like.

The CLA2 molecules disclosed according to the present invention may be used for diagnosis, monitoring of the disease course and prognosis in cell proliferative disorders such as e.g. tumours.

Any methods for the detection of CLA2 molecules or of immunological entities directed against CLA2 molecules according to the present invention may e.g. be useful in the course of diagnosis of disorders associated with altered expression of CLA2 molecules. Furthermore the methods for detection of CLA2 molecules or of immunological entities directed against CLA2 molecules may be used for the determination of an immuno-status of individuals e.g. in the course of immuno-therapy or vaccination procedures.

Diagnosis of disorders associated with the expression of the inventive CLA2 gene as used herein may for example comprise the detection of cells or tissues affected by abnormal growth. In one preferred embodiment diagnosis means the primary detection of a disease in an organism or sample.

According to the present invention the method for diagnosis of disorders such as tumours may be applied in routine screening tests for preventive aspects in order to detect said disease at an early stage of the onset of the disorder. For the purpose of early detection e.g. samples obtained by minimally invasive methods such as e.g. blood samples, stool samples, sputum samples, nipple aspirates, or samples obtained by methods comprising colonoscopy, bronchioscopy, bronchioalveolar-lavage, ductal-lavage etc. may be employed. The methods according to the present invention may be employed in the course of the detection of early stages of tumours and of precursory lesions of tumours or cancers.

In another preferred embodiment the diagnostic method may be used to determine the minimal residual disease of a tumour after primary therapy. In this respect the method of the invention may be applied to determine cells in body samples displaying abnormal expression of marker molecules according to the present invention, characteristic for tumours. Thus a spread of affected cells may be detected in body fluids.

In one embodiment of the invention the methods disclosed herein may be used for the detection and identification of metastases. The method may be applied either for detection of metastases in body tissues or organs by the detection methods described herein, or the metastases may be diagnosed with respect to prognosis and prediction of disease course.

Monitoring of the disease course may comprise determining the levels of marker molecules at different time points, comparing the levels at the different time points and assessing a diagnosis about the progression of the disease over the covered period of time. Thus monitoring may enable for assessment of prognosis and/or for design of an adequate therapy for a particular patient.

Monitoring or diagnosis as used in the context of the present invention may also comprise the detection of an immuno-status of individuals in the course of immuno therapy or vaccination therapy.

Prognosis of the disease course of a cell proliferative disorders such as e.g. tumours according to the present invention may comprise determining the level of expression of one or more marker molecules, comparing the levels with data from subsequent studies in a database and prognosticating the disease course from said comparison. In a preferred embodiment the method may comprise the detection of the levels of a set of marker molecules, the distinct levels of which may characterize distinct stages in the course of the disease. In a further embodiment of the invention the combination of the levels of a combination of markers may be an indicator for the prognosis of the further disease course and may build the basis for design of an adequate therapy.

Another aspect of the present invention is to provide a method for therapy and/or vaccination. According to the present invention a therapy of cell proliferative disorders can be carried out using the inventive CLA2 polypeptides and/or polynucleotides. The therapy may be for example immuno-therapy or somatic gene therapy.

The inventive CLA2 polypeptides and/or polynucleotides may according to the present invention be used for vaccination against cell proliferative disorders. Vaccination according to the present invention may comprise administering an immunogenic compound to an individual for the purpose of stimulating an immune response directed against said immunogenic compound and thus immunizing said individual against said immunogenic compound. Stimulating an immune response may comprise inducing the production of antibodies against said compound as well as stimulating cytotoxic T-cells. For the purpose of vaccination the polypeptides, nucleic acids and binding agents according to the present invention may be administered in a physiological acceptable form. The composition to be administered to individuals may comprise one or more antigenic components, physiologically acceptable carrier substances or buffer solutions, immuno-stimulants and/or adjuvants. Adjuvants may comprise for example Freund's incomplete adjuvant or Freund's complete adjuvant or other adjuvants known to those of skill in the art.

The composition may be administered in any applicable way such as e.g. intravenous, subcutaneous, intramuscular etc.. The dosage of the composition depends on the particular case and purpose of the vaccination. It has to be adapted to parameters by the individual treated such as age, weight, sex etc.. Furthermore the type of the immune response to be elicited has to be taken into account. In general it may be preferable if an individual receives 100 µg - 1 g of a polypeptide according to the present invention or 10⁶ - 10¹² MOI of a recombinant nucleic acid, containing a nucleic acid according to the present invention in a form that may be expressed *in situ*.

Individuals for the purpose of vaccination may be any organisms containing the inventive tumour associated polypeptides and/or polynucleotides and being able to get affected by cell proliferative disorders.

Vaccination of individuals may be favourable e.g. in the case of altered, non wild-type sequences or structure of marker molecules associated with cell proliferative disorders.

Polypeptides disclosed herein may also be employed in adoptive immuno-therapy for the treatment of cancer. Adoptive immuno-therapy may be broadly classified into either active or passive immuno-therapy. In active immuno-therapy, treatment relies on the *in vivo* stimulation of the endogenous host immune system to react against tumours with the administration of immune response-modifying agents (for example, tumour vaccines, bacterial adjuvants, and/or cytokines).

In passive immuno-therapy, treatment involves the delivery of biologic reagents with established tumour-immune reactivity (such as effector cells or antibodies) that can directly or indirectly mediate anti-tumour effects and does not necessarily depend on an intact host immune system. Examples of effector cells include T lymphocytes (for example, CD8+ cytotoxic T-lymphocyte, CD4+ T-helper, tumour-infiltrating lymphocytes), killer cells (such as Natural Killer cells, lymphokine-activated killer cells), B cells, or antigen presenting cells (such as dendritic cells and macrophages) expressing the disclosed antigens. The polypeptides disclosed herein may also be used to generate antibodies or anti-idiotypic antibodies (as in U.S. Pat. No. 4,918,164), for passive immuno-therapy.

The predominant method of procuring adequate numbers of T-cells for adoptive immuno-therapy is to grow immune T-cells *in vitro*. Culture conditions for expanding single antigen-specific T-cells to several billion in number with retention of antigen recognition *in vivo* are well known in the art. These *in vitro* culture conditions typically utilize intermittent stimulation with antigen, often in the presence of cytokines, such as IL-2, and non-dividing feeder cells. As noted above, the immuno-reactive polypeptides described herein may be used to rapidly expand antigen-specific T cell cultures in order to generate sufficient number of cells for immuno-therapy. In particular, antigen-presenting cells, such as dendritic, macrophage or B-cells, may be pulsed with immuno-reactive polypeptides or transfected with a nucleic acid sequence(s), using standard techniques well known in the art. For example, antigen presenting cells may be transfected with a nucleic acid sequence, wherein said sequence contains a promoter region appropriate for increasing expression, and can be expressed as part of a recombinant virus or other expression system. For cultured T-cells to be effective in therapy, the cultured T-cells must be able to grow and distribute widely and to survive long term in *vivo*. Studies have demonstrated that cultured T-cells can be induced to grow *in vivo* and to survive long term in substantial numbers by repeated stimulation with antigen supplemented with IL-2 (see, for example, Cheever, M., et al, "Therapy With Cultured T Cells: Principles Revisited," Immunological Reviews, 157:177,1997).

The CLA2 polypeptides disclosed herein may also be employed to generate and/or isolate tumour-reactive T-cells, which can then be administered to the patient. In one technique, antigen-specific T-cell lines may be generated by *in vivo* immunization with short peptides corresponding to immunogenic portions of the disclosed polypeptides. The resulting antigen specific CD8⁺ CTL clones may be isolated from the patient, expanded using standard tissue culture techniques, and returned to the patient.

Alternatively, peptides corresponding to immunogenic portions of the CLA2 polypeptides of the invention may be employed to generate tumour reactive T-cell subsets by selective *in vitro* stimulation and expansion of autologous T-cells to provide antigen-specific T-cells which may be subsequently transferred to the patient as described, for example, by Chang et al. (Crit. Rev. Oncol. Hematol., 22(3), 213, 1996). Cells of the immune system, such as T-cells, may be isolated from the peripheral blood of a patient, using a commercially available cell separation system, such as CellPro Incorporated's (Bothell, Wash.) CEPRATE.™. system (see U.S. Pat. No. 5,240,856; U.S. Pat. No. 5,215,926; WO 89/06280; WO 91/16116 and WO 92/07243). The separated cells are stimulated with one or more of the immuno-reactive polypeptides contained within a delivery vehicle, such as a microsphere, to provide antigen-specific T-cells. The population of tumour antigen-specific T-cells is then expanded using standard techniques and the cells are administered back to the patient.

In another embodiment, T-cell and/or antibody receptors specific for the polypeptides can be cloned, expanded, and transferred into other vectors or effector cells for use in adoptive immuno-therapy.

In a further embodiment, syngeneic or autologous dendritic cells may be pulsed with peptides corresponding to at least an immunogenic portion of a polypeptide disclosed herein. The resulting antigen-specific dendritic cells may either be transferred into a patient, or employed to stimulate T-cells to provide antigen-specific T-cells, which may, in turn, be administered to a patient. The use of peptide-pulsed dendritic cells to generate antigen-specific T-cells and the subsequent use of such antigen-specific T-cells to eradicate tumours in a murine model has been demonstrated by Cheever et al, Immunological Reviews, 157:177,1997.

These lesions according to the present invention comprise conditions characterized by abnormal growth properties of cells or tissues compared to the growth properties of normal control cells or tissues. The growth of the cells or tissues may be for example abnormally accelerated or may be regulated abnormally. Abnormal regulation as used above may comprise any form of presence or absence of non wild-type responses of the cells or tissues to naturally occurring growth regulating influences. The abnormalities in growth of the cells or tissues may be for example neoplastic or hyperplastic.

Disorders characterized by abnormal cell proliferation, as used in the context of the present invention, may comprise for example neoplasms such as benign and malignant tumours, carcinomas, sarcomas, leukemias, lymhomas or dysplasias. Tumours may comprise tumours of the head and the neck, tumours of the respiratory tract, tumours of the gastrointestinal tract, tumours of the urinary system, tumours of the reproductive system, tumours of the endocrine system, tumours of the central and peripheral nervous system, tumours of the skin and its appendages, tumours of the soft tissues and bones, tumours of the lymphopoietic and hematopoietic system, breast cancer, colorectal cancer, gastrointestinal cancer, anogenital cancer etc..

In certain embodiments the disorders are for example adenomas or adenocarcinomas of the colon, disorders of the respiratory tract such as Squamous Cell Lung Carcinoma, Small Cell Lung Carcinoma, Adenocarcinoma of the Lung, Large Cell Lung Carcinoma, Adeno-Squamous Lung Carcinoma, Carcinoid Tumour of the Lung, Broncheal Gland Tumour or (malignant) Mesothelioma, anogenital cancer such as, cervical cancer, vulval cancer, vaginal cancer, cancer of the rectum, cancer of the anus and cancer of the penis. In one embodiment the disorders may be breast cancer.

Additionally, vectors expressing the disclosed nucleic acids may be introduced into stem cells taken from the patient and clonally propagated *in vitro* for autologous transplant back into the same patient.

Monoclonal antibodies of the present invention may also be used as therapeutic compounds in order to diminish or eliminate tumours. The antibodies may be used on their own (for instance, to inhibit metastases) or coupled to one or more therapeutic agents. Suitable agents in this regard include radio nuclides, differentiation inducers, drugs, toxins, and derivatives thereof. Preferred radio nuclides include 90Y, 123I, 125I, 131I, 186Re, 188Re, 211At, and 212Bi. Preferred drugs include methotrexate, and pyrimidine and purine analogues. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein.

In one embodiment of the invention the therapy of disorders characterized by abnormal cell proliferation may comprise the administration of antisense construct or ribozymes. The methods for administration of ribozymes or antisense constructs are known to those of skill in the art. The administration may take place as administration of naked nucleic acids or as administration of nucleic acids that are suited for expression of the relevant active products *in situ*.

In another embodiment of the invention the treatment of disorders may comprise the administration of binding agents directed against the CLA2 polypeptides. These binding agents may for example be coupled to other compounds such as toxins, enzymes, radio-isotopes etc.

In another embodiment of the invention therapy of disorders associated with abnormal expression of the presented CLA2 polypeptides may comprise the administration of antagonists or agonists of the CLA2 polypeptides, of binding partners of the tumour associated polypeptides of inhibitors or enhancer of the expression of the inventive polypeptides or of drugs identifiable by assays involving the measurement of the activity of the inventive polypeptides. The methods for identifying these substances are known to those of skill in the art.

An example for a method for identifying a binding partner of an inventive CLA2 polypeptide (or related polypeptide) and/or polynucleotide may comprise:
(a) contacting the inventive CLA2 polypeptide of the invention with a compound to be screened; and
(b) determining whether the compound effects an activity of the polypeptide.

The inventive CLA2 polypeptides may be used to screen for proteins or other compounds that bind to the inventive colorectal lesion associated polypeptides or for proteins or other compounds to which the inventive colorectal lesion associated polypeptide binds. The binding of the inventive CLA2 polypeptide and the molecule may activate (agonist), increase, inhibit (antagonist), or decrease activity of the CLA2 polypeptide or the molecule bound. Examples of such molecules include antibodies, oligonucleotides, proteins (e.g., receptors), or small molecules.

Preferably, the molecule is closely related to the natural ligand of the inventive colorectal lesion associated polypeptide, e.g., a fragment of the ligand, or a natural substrate, a ligand, a structural or functional mimetic; see, e.g., Coligan, Current Protocols in Immunology 1(2) (1991); Chapter 5. Similarly, the molecule can be closely related to a natural receptor to which the inventive colorectal lesion associated polypeptide might bind, or at least, a fragment of the receptor capable of being bound by the CLA2 polypeptide (e.g., active site). In either case, the molecule can be rationally designed using known techniques.

Preferably, the screening for these molecules involves producing appropriate cells which express the CLA2 polypeptide, either as a secreted protein or on the cell membrane. Preferred cells include cells from mammals, yeast, Drosophila, or E. coli. Cells expressing the inventive colorectal lesion associated polypeptide (or cell membrane containing the expressed polypeptide) are then preferably contacted with a test compound potentially containing the molecule to observe binding, stimulation, or inhibition of activity of CLA2 polypeptide.

The assay may simply test binding of a candidate compound to the CLA2 polypeptide, wherein binding is detected by a label, or in an assay involving competition with a labelled competitor. Further, the assay may test whether the candidate compound results in a signal generated by binding to the CLA2 polypeptide.

Alternatively, the assay can be carried out using cell-free preparations, polypeptide/molecule affixed to a solid support, chemical libraries, or natural product mixtures. The assay may also simply comprise the steps of mixing a candidate compound with a solution containing the inventive colorectal lesion associated polypeptide, measuring the inventive colorectal lesion associated polypeptide/molecule activity or binding, and comparing the CLA2 polypeptide/molecule activity or binding to a standard.

Preferably, an ELISA assay can measure the CLA2 level or activity in a sample (e.g., biological sample) using a monoclonal or polyclonal antibody. The antibody can measure the CLA2 polypeptide level or activity by either binding, directly or indirectly, to the inventive colorectal lesion associated polypeptide or by competing with the inventive CLA2 polypeptide for a substrate. All of these above assays can be used as diagnostic or prognostic markers. The molecules discovered using these assays can be used to treat disease or to bring about a particular result in a patient (e.g., elimination of a epithelial tumour or stop of progression of tumour growth) by activating or inhibiting the inventive colorectal lesion associated polypeptide molecule. Moreover, the assays can discover agents which may inhibit or enhance the production of the CLA2 polypeptide from suitably manipulated cells or tissues.

Therefore, the invention includes a method of identifying compounds for use in treatment of disorders characterized by abnormal cell proliferation which bind to a the inventive CLA2 polypeptide comprising the steps of: (a) incubating a candidate binding compound with a CLA2 polypeptide of the invention; and (b) determining if binding has occurred.

Moreover, the invention includes a method of identifying activators/agonists or inhibitors/antagonists of the inventive colorectal lesion associated polypeptide for use in treatment of disorders characterized by abnormal cell proliferation comprising the steps of: (a) incubating a candidate compound with a CLA2 polypeptide of the invention; b) assaying a biological activity, and (c) determining if a biological activity of the CLA2 polypeptide of the invention (e.g. receptor activity) has been altered.

In a further embodiment, the present invention relates to method of identifying and obtaining a drug candidate for therapy of a disorder associated with abnormal cell proliferation comprising the steps of 1.contacting a the inventive CLA2 or a cell expressing said CLA2 polypeptide in the presence of components capable of providing a detectable signal in response
to altered regulation of cell proliferation
to altered activity of a polypeptide
to altered cell differentiation, with said drug candidate to be screened under conditions to allow protein degradation, and
(b) detecting presence or absence of a signal or increase of the signal generated from CLA2 polypeptide activity, cell proliferation or differentiation, wherein the presence or increase of the signal is indicative for a putative drug.

Experiments using animals or isolated cells or cell lines may be used to examine the proliferative behaviour of cells or tissues in dependence on the inventive colorectal lesion associated CLA2 polypeptide action. The same procedures may be employed for the study of cell differentiation.

The drug candidate may be a single compound or a plurality of compounds. The term "plurality of compounds" in a method of the invention is to be understood as a plurality of substances which may or may not be identical.

Said compound or plurality of compounds may be chemically synthesized or microbiologically produced and/or comprised in, for example, samples, e.g., cell extracts from, e.g., plants, animals or micro organisms. Furthermore, said compound(s) may be known in the art but hitherto not known to be capable of suppressing or activating the inventive CLA2 polypeptide. The reaction mixture may be a cell free extract or may comprise a cell or tissue culture. Suitable set ups for the method of the invention are known to the person skilled in the art and are, for example, generally described in Alberts et al., Molecular Biology of the Cell, third edition (1994) and in the appended examples. The plurality of compounds may be, e.g., added to the reaction mixture, culture medium, injected into a cell or otherwise applied to the transgenic animal. The cell or tissue that may be employed in the method of the invention preferably is a host cell, mammalian cell or non-human transgenic animal of the invention described in the embodiments hereinbefore.

If a sample containing a compound or a plurality of compounds is identified in the method of the invention, then it is either possible to isolate the compound from the original sample identified as containing the compound capable of suppressing or activating the inventive CLA2 polypeptide, or one can further subdivide the original sample, for example, if it consists of a plurality of different compounds, so as to reduce the number of different substances per sample and repeat the method with the subdivisions of the original sample. Depending on the complexity of the samples, the steps described above can be performed several times, preferably until the sample identified according to the method of the invention only comprises a limited number of or only one substance(s). Preferably said sample comprises substances of similar chemical and/or physical properties, and most preferably said substances are identical.

Several methods are known to the person skilled in the art for producing and screening large libraries to identify compounds having specific affinity for a target. These methods include the phage-display method in which randomised peptides are displayed from phage and screened by affinity chromatography to an immobilized receptor; see, e.g., WO 91/17271, WO 92/01047, US-A-5,223,409. In another approach, combinatorial libraries of polymers immobilized on a chip are synthesized using photolithography; see, e.g., US-A-5,143,854, WO 90/15070 and WO 92/10092. The immobilized polymers are contacted with a labelled receptor and scanned for label to identify polymers binding to the receptor.

The synthesis and screening of peptide libraries on continuous cellulose membrane supports that can be used for identifying binding ligands of the CLA2 polypeptide of the invention and thus possible inhibitors and activators is described, for example, in Kramer, Methods Mol. Biol. 87 (1998), 25-39. This method can also be used, for example, for determining the binding sites and the recognition motifs in the CLA2 polypeptide of the invention. In like manner, the substrate specificity of the DnaK chaperon was determined and the contact sites between human interleukin-6 and its receptor; see Rudiger, EMBO J. 16 (1997), 1501-1507 and Weiergraber, FEBS Lett. 379 (1996), 122-126, respectively.

Furthermore, the above-mentioned methods can be used for the construction of binding super topes derived from the polypeptide of the invention. A similar approach was successfully described for peptide antigens of the anti-p24 (HIV-1) monoclonal antibody; see Kramer, Cell 91 (1997), 799-809. A general route to fingerprint analyses of peptide-antibody interactions using the clustered amino acid peptide library was described in Kramer, Mol. Immunol. 32 (1995), 459-465. In addition, antagonists of the inventive CLA2 can be derived and identified from monoclonal antibodies that specifically react with the polypeptide of the invention in accordance with the methods as described in Doring, Mol. Immunol. 31 (1994), 1059-1067.

More recently, WO 98/25146 described further methods for screening libraries of complexes for compounds having a desired property, especially, the capacity to agonize, bind to, or antagonize a polypeptide or its cellular receptor. The complexes in such libraries comprise a compound under test, a tag recording at least one step in synthesis of the compound, and a tether susceptible to modification by a reporter molecule. Modification of the tether is used to signify that a complex contains a compound having a desired property. The tag can be decoded to reveal at least one step in the synthesis of such a compound. Other methods for identifying compounds which interact with the CLA2 polypeptides according to the invention or CLA2 nucleic acid molecules encoding such molecules are, for example, the *in vitro* screening with the phage display system as well as filter binding assays or "real time" measuring of interaction using, for example, the BIAcore apparatus (Pharmacia).

All these methods can be used in accordance with the present invention to identify activators/agonists and inhibitors/antagonists of the CLA2 polypeptide or related polypeptide of the invention.

Various sources for the basic structure of such an activator or inhibitor can be employed and comprise, for example, mimetic analogues of the polypeptide of the invention. Mimetic analogues of the CLA2 polypeptide of the invention or biologically active fragments thereof can be generated by, for example, substituting the amino acids that are expected to be essential for the biological activity with, e.g., stereo isomers, i.e. D-amino acids; see e.g., Tsukida, J. Med. Chem. 40 (1997), 3534-3541. Furthermore, in case fragments are used for the design of biologically active analogues promimetic components can be incorporated into a peptide to re-establish at least some of the conformational properties that may have been lost upon removal of part of the original polypeptide; see, e.g., Nachman, Regul. Pept. 57 (1995), 359-370.

Furthermore, the inventive colorectal lesion associated polypeptide can be used to identify synthetic chemical peptide mimetics that bind to or can function as a ligand, substrate, binding partner or the receptor of the polypeptide of the invention as effectively as does the natural polypeptide; see, e.g., Engleman, J. Clin. Invest. 99 (1997), 2284-2292. For example, folding simulations and computer redesign of structural motifs of the polypeptide of the invention can be performed using appropriate computer programs (Olszewski, Proteins 25 (1996), 286-299; Hoffman, Comput. Appl. Biosci. 11 (1995), 675-679). Computer modelling of protein folding can be used for the conformational and energetic analysis of detailed peptide and protein models (Monge, J. Mol. Biol. 247 (1995), 995-1012; Renouf, Adv. Exp. Med. Biol. 376 (1995), 37-45). In particular, the appropriate programs can be used for the identification of interactive sites of the inventive CLA2 polypeptide and its possible ligand or other interacting proteins by computer assistant searches for complementary peptide sequences (Fassina, Immunomethods 5 (1994), 114-120. Further appropriate computer systems for the design of protein and peptides are described in the prior art, for example in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991.

The results obtained from the above-described computer analysis can be used for, e.g., the preparation of peptide mimetics of the CLA2 protein of the invention or fragments thereof. Such pseudo-peptide analogues of the natural amino acid sequence of the protein may very efficiently mimic the parent protein (Benkirane, J. Biol. Chem. 271 (1996), 33218-33224). For example, incorporation of easily available achiral-amino acid residues into a protein of the invention or a fragment thereof results in the substitution of amide bonds by polymethylene units of an aliphatic chain, thereby providing a convenient strategy for constructing a peptide mimetic (Banerjee, Biopolymers 39 (1996), 769-777). Superactive peptidomimetic analogues of small peptide hormones in other systems are described in the prior art (Zhang, Biochem. Biophys. Res. Commun. 224 (1996), 327-331). Appropriate peptide mimetics of the protein of the present invention can also be identified by the synthesis of peptide mimetic combinatorial libraries through successive amide alkylation and testing the resulting compounds, e.g., for their binding and immunological properties. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715.

Furthermore, a three-dimensional and/or crystallographic structure of the CLA2 polypeptide of the invention can be used for the design of peptide mimetic inhibitors of the biological activity of the polypeptide of the invention (Rose, Biochemistry 35 (1996), 12933-12944; Rutenber, Bioorg. Med. Chem. 4 (1996), 1545-1558).

The structure-based design and synthesis of low-molecular-weight synthetic molecules that mimic the activity of the native biological polypeptide is further described in, e.g., Dowd, Nature Biotechnol. 16 (1998), 190-195; Kieber-Emmons, Current Opinion Biotechnol. 8 (1997), 435-441; Moore, Proc. West Pharmacol. Soc. 40 (1997), 115-119; Mathews, Proc. West Pharmacol. Soc. 40 (1997), 121-125; Mukhija, European J. Biochem. 254 (1998), 433-438.

It is also well known to the person skilled in the art, that it is possible to design, synthesize and evaluate mimetics of small organic compounds that, for example, can act as a substrate or ligand to the inventive CLA2 polypeptide of the invention or the related polypeptide. For example, it has been described that D-glucose mimetics of hapalosin exhibited similar efficiency as hapalosin in antagonizing multidrug resistance assistance-associated protein in cytotoxicity; see Dinh, J. Med. Chem. 41 (1998), 981-987.

The CLA2 nucleic acid molecule of the invention can also serve as a target for activators and inhibitors. Activators may comprise, for example, proteins that bind to the mRNA of a gene encoding a the inventive CLA2 polypeptide, thereby stabilizing the native conformation of the mRNA and facilitating transcription and/or translation, e.g., in like manner as Tat protein acts on HIV-RNA. Furthermore, methods are described in the literature for identifying nucleic acid molecules such as an RNA fragment that mimics the structure of a defined or undefined target RNA molecule to which a compound binds inside of a cell resulting in retardation of cell growth or cell death; see, e.g., WO 98/18947 and references cited therein. These nucleic acid molecules can be used for identifying unknown compounds of pharmaceutical and/or agricultural interest, and for identifying unknown RNA targets for use in treating a disease. These methods and compositions can be used in screening for novel antibiotics, bacteriostatics, or modifications thereof or for identifying compounds useful to alter expression levels of proteins encoded by a nucleic acid molecule.

Alternatively, for example, the conformational structure of the RNA fragment which mimics the binding site can be employed in rational drug design to modify known antibiotics to make them bind more avidly to the target. One such methodology is nuclear magnetic resonance (NMR), which is useful to identify drug and RNA conformational structures. Still other methods are, for example, the drug design methods as described in WO 95/35367, US-A-5,322,933, where the crystal structure of the RNA fragment can be deduced and computer programs are utilized to design novel binding compounds which can act as antibiotics.

Some genetic changes lead to altered protein conformational states. For example, some mutant the inventive colorectal lesion associated polypeptides may possess a tertiary structure that renders them far less capable of protein degradation. Restoring the normal or regulated conformation of mutated proteins is the most elegant and specific means to correct these molecular defects, although it may be difficult. Of particular interest in this regard is the consensus domain of the inventive colorectal lesion associated polypeptide

The compounds which can be tested and identified according to a method of the invention may be expression libraries, e.g., cDNA expression libraries, peptides, proteins, nucleic acids, antibodies, small organic compounds, hormones, peptidomimetics, PNAs or the like (Milner, Nature Medicine 1 (1995), 879-880; Hupp, Cell 83 (1995), 237-245; Gibbs, Cell 79 (1994), 193-198 and references cited supra). Furthermore, genes encoding a putative regulator of the inventive CLA2 polypeptide and/or which exert their effects up- or downstream the inventive CLA2 polypeptide may be identified using, for example, insertion mutagenesis using, for example, gene targeting vectors known in the art. Said compounds can also be functional derivatives or analogues of known inhibitors or activators. Such useful compounds can be for example transacting factors which bind to the inventive tumour associated polypeptide or regulatory sequences of the gene encoding it. Identification of transacting factors can be carried out using standard methods in the art (see, e.g., Sambrook, supra, and Ausubel, supra).

To determine whether a protein binds to the CLA2 protein itself or regulatory sequences, standard native gel-shift analyses can be carried out. In order to identify a transacting factor which binds to the protein or regulatory sequence, the protein or regulatory sequence can be used as an affinity reagent in standard protein purification methods, or as a probe for screening an expression library.

The identification of nucleic acid molecules which encode polypeptides which interact with the inventive CLA2 described above can also be achieved, for example, as described in Scofield (Science 274 (1996), 2063-2065) by use of the so-called yeast "two-hybrid system". In this system the polypeptide encoded by a nucleic acid molecule according to the invention or a smaller part thereof is linked to the DNA-binding domain of the GAL4 transcription factor. A yeast strain expressing this fusion polypeptide and comprising a lacZ reporter gene driven by an appropriate promoter, which is recognised by the GAL4 transcription factor, is transformed with a library of cDNAs which will express plant proteins or peptides thereof fused to an activation domain. Thus, if a peptide encoded by one of the cDNAs is able to interact with the fusion peptide comprising a peptide of a inventive CLA2 polypeptide, the complex is able to direct expression of the reporter gene. In this way the nucleic acid molecules according to the invention and the encoded peptide can be used to identify peptides and proteins interacting with the CLA2 protein. It is apparent to the person skilled in the art that this and similar systems may then further be exploited for the identification of inhibitors of the binding of CLA2 proteins.

Once the transacting factor is identified, modulation of its binding to or regulation of expression of CLA2 polypeptide can be pursued, beginning with, for example, screening for inhibitors against the binding of the transacting factor to the CLA2 protein of the present invention. Activation or repression of the inventive CLA2 proteins could then be achieved in animals by applying the transacting factor (or its inhibitor) or the gene encoding it, e.g. in an expression vector. In addition, if the active form of the transacting factor is a dimer, dominant-negative mutants of the transacting factor could be made in order to inhibit its activity.

Furthermore, upon identification of the transacting factor, further components in the pathway leading to activation (e.g. signal transduction) or repression of a gene involved in the control of the inventive tumour associated polypeptide then can be identified. Modulation of the activities of these components can then be pursued, in order to develop additional drugs and methods for modulating the metabolism of protein degradation in animals. Thus, the present invention also relates to the use of the two-hybrid system as defined above for the identification of the inventive tumour associated polypeptide or activators or inhibitors of the inventive CLA2 polypeptide.

The compounds isolated by the above methods also serve as lead compounds for the development of analogue compounds. The analogues should have a stabilized electronic configuration and molecular conformation that allows key functional groups to be presented to the inventive CLA2 polypeptide or its possible receptor in substantially the same way as the lead compound. In particular, the analogue compounds have spatial electronic properties which are comparable to the binding region, but can be smaller molecules than the lead compound, frequently having a molecular weight below about 2 kD and preferably below about 1 kD.

Identification of analogue compounds can be performed through use of techniques such as self-consistent field (SCF) analysis, configuration interaction (CI) analysis, and normal mode dynamics analysis. Computer programs for implementing these techniques are available; e.g., Rein, Computer-Assisted Modeling of Receptor-Ligand Interactions (Alan Liss, New York, 1989). Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art; see also supra. Furthermore, peptidomimetics and/or computer aided design of appropriate derivatives and analogues can be used, for example, according to the methods described above.

In a preferred embodiment of the above-described methods of the invention said cell is a cell of or, obtained by a method of the invention or is comprised in the above-described transgenic non-human animal.

Once the described compound has been identified and obtained, it is preferably provided in a therapeutically acceptable form.

It must be understood, that the compounds and methods as disclosed throughout this text are applicable to any mammalian individual. Thus the compounds and methods may be applied to animals as well as to human beings and are such useful in veterinary medical as in medical purposes. Animals, that may be of especial interest with respect to the present invention are companion animals, such as cats, dogs, etc. animals of agricultural interest such as cows, pigs, horses, laboratory animals such as rats, mice, hamsters, rabbits etc. and any other animal, that may be affected by a disorder characterized by abnormal growth of cells.

The present invention provides compounds and methods useful for detection and treatment of disorders characterized by abnormal cell proliferation, such as e.g. cancers. In a first aspect the present invention provides novel CLA2 nucleic acids and CLA2 polypeptides for use as marker molecules indicative of cancer for use in diagnostic purposes. In one aspect the present invention provides a method for the detection of disorders characterized by abnormal cell proliferation, such as e.g. cancers based on the determination of the presence or absence and/or the level of expression of the inventive CLA2 gene in biological samples. This detection method may e.g. be employed in the course of early detection of neoplasias and precursory stages of tumours. In a second aspect the present invention provides a method for treatment of disorders characterized by abnormal cell proliferation, such as e.g. cancers using the inventive tumour associated CLA2 gene products as therapeutically active agents. The invention also provides for therapeutic methods based on the modulation of the activity of the inventive CLA2 polypeptide. It is one aspect of the invention to provide a method for rational tumour management based on the detection of the inventive CLA2 gene products in patient samples and the tailoring of a therapy correlated to the detected over expression of said CLA2 gene products. Furthermore the present invention provides for a research or diagnostic test kit for performing the reactions involved in the detection of the presence or absence and/or the level of over expression of the inventive CLA2 gene. Finally the present invention relates to pharmaceutical compositions applicable in the treatment of disorders according to the present invention comprising the inventive CLA2 compounds.

### Brief description of the drawings:

- Figure 1:: **Overview of splicing variants and open reading frames of the CLA2 gene;** Variant CLA2.1 comprises 5 exons with an open reading frame of 54 amino acids. Variant CLA2.2 comprises the same 5 exons with an alternative open reading frame of 70 amino acids. Variant CLA2.3 comprises 4 exons with an open reading frame of 100 amino acids (exon 3 is missing). Variant CLA2.1 represents the truncated form of CLA2.3. CLA2.4 to CLA2.8 are not shown in the graph. They represent short alternative open reading frames located in exon 4 and exon 5.
- Figure 2:: **Cloned Rsal restriction fragment of the CLA2 gene;** The fragment was spotted in duplicates on nylon membranes and hybridised with the normal colon subtracted probe (N) and the adenoma subtracted probe (Ad), respectively. For experimental details see Example 1.
- Figure 3:: **Results of RT PCR experiments;** RT PCR experiments were performed for determination of the level of expression of the inventive CLA2 gene in three adenoma samples (Ad) and the corresponding normal colon tissues (N). For experimental details see Example 2.
- Figure 4:: **Gel electrophoretic analysis of the PCR products obtainable with primers specific for the inventive CLA2 marker gene;** In this experiment the various splicing variants (a-b) of the gene are displayed. For experimental details see Example 3.

The following examples are given for the purpose of illustration only and are not intended to limit the scope of the invention disclosed herein.

### Example 1: Identification of differential expression of CLA2 gene in human colon tissue versus human colon adenoma tissue

### (i) Isolation of total RNA:

Total RNA was extracted from human colon tissue by using the RNeasy kit (Qiagen) according to the manufacturer's protocol. The quality of the prepared RNA was determined by agarose gel electrophoresis according to the standard procedures (Sambrook J, et al., 1989). The mRNA fraction was amplified using the SMART™ PCR cDNA synthesis kit (Clontech).

### (ii) Identification of differentially expressed genes by suppressive subtractive hybridisation:

This technique compares two populations of mRNA and provides clones of genes that are expressed in one population but not in the other. The applied technique was described in detail in Diatchenko et al., 1996. In the present invention, mRNA populations from human colon adenoma tissue and normal colon tissue were compared. Specifically, mRNA of the normal colon biopsy was subtracted from mRNA of the colon adenoma tissue. The necessary reagents were taken from the PCR-Select™ cDNA subtraction kit (Clontech) and all steps were performed as described in the manufacturer's protocol.

In detail, 2µg SMART™-amplified double-stranded cDNA was used. After Rsal-digestion and adaptor ligation hybridisation of tester and driver were performed for 8 h (first hybridisation) and 15 h (second hybridisation) at 68°C. Two PCR steps were performed to amplify differentially expressed genes (first PCR: 27 cycles of 94°C 30 s, 66°C 30 s and 72°C 1.5 min; nested PCR: 12 cycles of 94°C 30 s, 66°C 30 s and 72°C 1.5 min) using adaptor specific primers (included in the subtraction kit) and 50x Advantage Polymerase Mix (Clontech). Efficiencies of Rsal-digestions, adaptor ligations and subtractive hybridisations were checked as recommended in the kit.

Subtracted cDNAs were inserted into the pCR2.1 vector and transformed into INVF' cells (Invitrogen).

To isolate individual cDNAs of the subtracted library single bacterial transformants were incubated in 100 µl LB amp (50 µg/ml) at 37°C for at least 4 hours. Inserts were PCR amplified (95°C 30 sec, 68°C 3 min for 30 cycles) in 20 µl containing 10 mM Tris-HCl pH 9.0, 1.5 mM MgCl2, 50 mM KCI, 200 µM dNTP, 0.5 µM adaptor specific primers (included in the subtraction kit), 1.5 Units Taq polymerase (Pharmacia Biotech), and 1µl of bacterial culture.

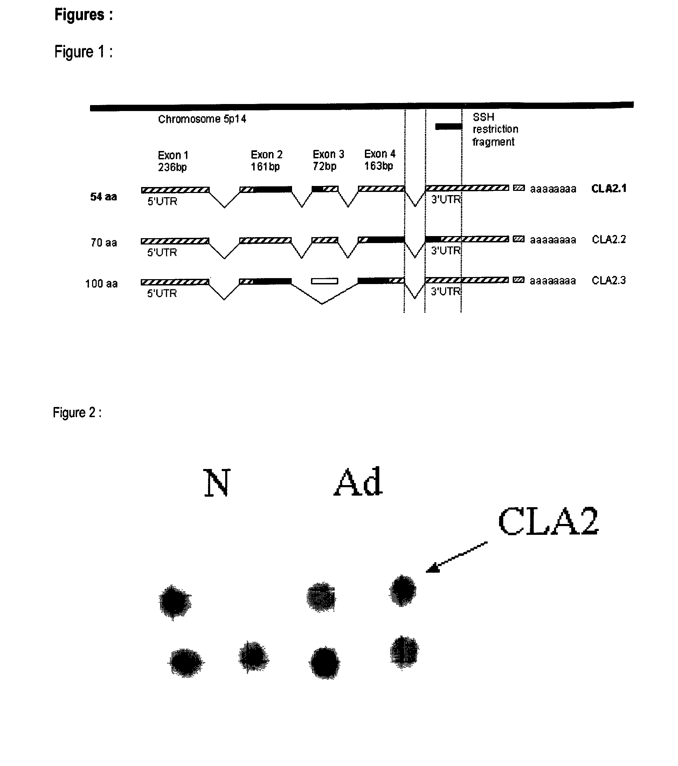

1.5 µl of a mixture containing 3 µl PCR amplified inserts and 2 µl 0.3 N NaOH/15% Ficoll were spotted onto a positively charged nylon membrane (Roche). In this way, hundreds of spots were arrayed on duplicate filters for subsequent hybridisation. The differential screening step consists of hybridisations of the subtracted library with itself to minimize background (Wang et al., 1991). The probes were made of the nested PCR product of the subtraction following the instructions of the Clontech subtraction kit. Labelling with Digoxygenin was performed with the DIG DNA Labelling Kit (Roche). Hybridisations were carried out overnight in DIG Easy HYB (Roche) at 43°C. The filters were washed twice in 2 x SSC / 0.5% SDS at 68°C for 15 min and twice in 0,1 x SSC / 0.5% SDS at 68°C for 15 min, and subjected to detection using anti-DIG-AP conjugates and CDP-Star™ as chemiluminescent substrate according to the instructions of the DIG DNA Detection Kit (Roche). Blots were exposed to Kodak Biomax MR chemiluminescent film at room temperature for several minutes (Figure 2).

The novel CLA2 gene given in SEQ NO. 1 has been identified to be differentially expressed in colorectal lesions compared to normal tissue. The gene is located on chromosome 5p14.

### Example 2: Detection of the level of expression of the inventive CLA2 nucleic acid associated with colorectal disorders by RT-PCR in colorectal samples and samples of lung tissue

Samples of adenocarcinomas and adenomas of the colon and samples of lung carcinomas are used to determine the level of mRNA of the inventive nucleic acids using quantitative RT PCR. Each 3 tumour biopsies (of the colon and of the lung) and samples of 3 adenomas are used in this study.

Carcinomas and adenomas are collected, snap frozen, and stored at -80°C. They are verified to be composed predominantly of neoplastic cells by histopathological analysis. mRNA is isolated from tumours and patient-matched normal tissue using Qiagen reagents (Qiagen, Hilden, Germany), and single-stranded cDNA is synthesized using Omniscript (Qiagen). PCR is performed using the ABI

Prism 7700 Sequence Detector (PE Applied Biosystems) and the QuantiTect™ SybrGreen PCR mix (Qiagen), as described in the manufacturers manual.

PCR reactions are performed in 25 µl volumes with a final concentration of 100nM for each primer (5'primer: 5'-CCACAGGAGCTGAAGAGAGC-3'; 3'primer: 5'-AGGGCTTGTCTGGGTATGC-3'), with 95°C for 15 sec and 60°C for 70 sec for 45 cycles.

The specificity of the PCR products is verified by gel electrophoresis.

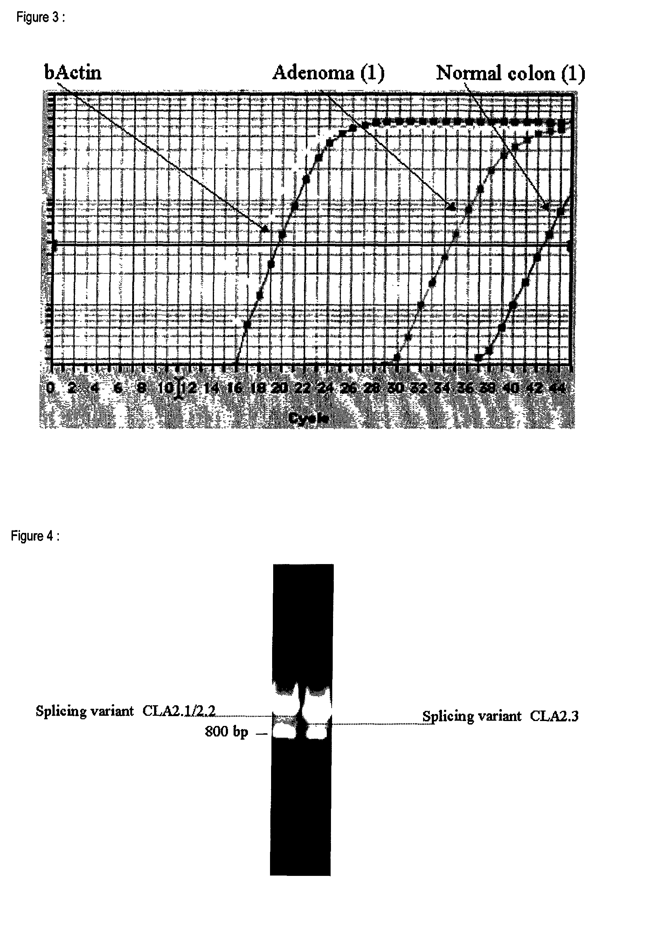

The results show, that the CLA2 gene identifiable by the method described in Example 1 is over expressed in tumour tissue in comparison to normal colon/lung mucosa tissue. Figure 3 shows the results of RT-PCR experiments for a colon adenoma.

The example illustrates, that the CLA2 gene identified herein is suitable for the detection of disorders associated with abnormal proliferation of cells such as e.g. colorectal lesions or lung tumours by determining the expression level of the marker gene given in SEQ NO. 1.

The results of the present experiment are given in Figure 3. The present example illustrates that the comparison of the expression level of the particular marker molecules in test tissue to the expression level in normal control tissue may be used to state whether the test tissue is affected by a disorder or not.

### Example 3: Detection of the splicing variants of the inventive CLA2 marker molecule

The marker molecule transcript was found to be over expressed in colon adenoma biopsies when compared with normal colon by subtractive hybridisation as described in Example 1. The cloned Rsal restriction fragment of the marker molecule is located in the 3' untranslated region of the marker CLA2 gene. Full-length 5'RACE-cloning was performed using two different gene specific primers (5'-GAGGCCAGTTTTCCCAAGGGGTTCCCATC-3', 5'-CACGGGCACATTGAAGGGTCAGTAGAGC-3') and the SMART™ RACE cDNA Amplification kit as described in the manufacturers manual (Clontech Laboratories, Palo Alto, CA).

Detection of splicing variants were performed by PCR using primer in the 5'UTR and 3'UTR of the transcript (5'-CCATCCCCCAATTTCAGTC-3', 5'-GTGTGAGGTGGTGCTATGTAGC-3'). PCR is performed using the Eppendorf Mastercycler (Eppendorf) and the Advantage2 PCR kit, as described in the manufacturers manual (Clontech Laboratories, Palo Alto, CA) and the reactions were performed in 25 µl volumes with a final concentration of 400nM for each primer (primer as described above), with 95°C for 30 sec, 60°C for 60 sec, and 68°C for 1.5 min for 35 cycles.

The PCR products were cloned into TA-pCR2.1-vector (Invitrogen) and individual clones were used for standard colony PCR to check for the inserts and analysed by sequencing.

The PCR products are verified by gel electrophoresis (Figure 4). The results show that two different PCR products are generated (CLA2.1/CLA2.2 and CLA2.3, compare Figure 1 and Figure 4). This indicates the presence of at least two different splicing variants in the test sample.

The results revealed that two of the colon adenoma specific splicing variants (CLA2.1 and CLA2.3) share one common start codon, but vary in splicing. Thus two polypeptides of different length and identical N-terminal sequence are rendered.

One third polypeptide (CLA2.2) encoded by the novel CLA2 gene uses a different start codon and thus varies from the other two CLA2 proteins in sequence.

The sequences of the three different protein products encoded by the novel CLA2 gene are given in SEQ NO. 3, 5, 7. The organization of the CLA2 gene and the positioning of the respective transcripts and polypeptides is presented in Figure 1.

Moreover, 5 additional short open reading frames were identified (CLA2.4 to CLA2.8) that lead to the translation of 5 different tumour specific neo-peptides with different lengths (34, 14, 10, 8, and 16 amino acids, respectively). The phenomenon that even very short open reading frames are translated to immunogenic peptides in tumours has already been described by Schiavetti et al. (Cancer Research 62, (2002) 5510-5516), which shall be incorporated herein by reference.

### Example 4: Determination of tissue specific expression profile of CLA2

To determine the presence or absence of the CLA2 transcript in different human tissues RT-PCRs (10sec at 95°C, 30sec at 60°C, 2min at 72°C for 40 cycles) were performed using specific CLA2-primer (5'- CCATCCCCCAATTTCAGTC-3' and 5'- GTGTGAGGTGGTGCTATGTAGC-3') and cDNA obtained from different human tissues (Clontech Human MTC Panel II and Human Digestive System MTC Panel) as well as cDNA from different tumour samples. The reactions were performed using 0.4µM primer, 0.4mM dNTP and Advantage2 DNA Polymerase (Clontech).

CLA2 is expressed in various cancer tissues including such of gastrointestinal origin, those originating from the respiratory tract and others. Particularly over expression has been detected in the majority of 20 tested colon carcinomas, as well as in adenomas of the colon where 25 out of 29 tested adenomas showed strong over expression. Over expression could also be detected in 7 out of 10 gastric carcinomas and in 2 out of 5 lung carcinomas. Similar results were obtained for tested cervical carcinoma samples, and samples of ovarial carcinoma.

In contrast in normal tissue CLA2 in all tested tissues except in testis showed very weak to no expression. Tested tissues include colon, small intestine, spleen, thymus, prostate, ovary, peripheral blood leukocytes, esophagus, duodenum, liver and stomach.

The results of the expression profiling for CLA2 in normal and tumour tissues of various origins indicate that CLA2 is a tumour associated gene product. Thus the gene products may be used to identify tumour tissues. Due to the absence of expression in normal tissue CLA2 may also represent a valuable tool for minimal residual disease (MRD) diagnostics.

### Example 5: Generation of polyclonal antibodies directed against the inventive Polypeptides

Recombinantly produced CLA2 polypeptides of all open reading frames (CLA2.1, CLA2.2, CLA2.3) of the herein presented nucleic acids were used to produce polyclonal antibodies directed against these polypeptides. The polyclonal antibodies were purified by affinity chromatography and afterwards used for the detection of the respective polypeptides in patient samples. The procedures were performed as follows:

### Production of the recombinant proteins:

CLA2 specific primers at the 5' and 3' end of the three different open reading frames were designed to amplify the ORFs by PCR:

CLA2.1:	5'-ATGCAATTGGAGAAACTGGTTGTT-3'
	5'-GTCGCAGGTTAAGAACCCTGTATG-3'

CLA2.2:	5'-ATGCCCACAGTGGACTGGAGCCCAA-3'
	5'-TAAGAAAAGCATAAAGCAGTACTTATGC-3'

CLA2.3:	5'-ATGCAATTGGAGAAACTGGTTGTT-3'
5'-CCACAGGAGCTGAAGAGAGCA-3'

The different open reading frames were PCR-amplified (20sec at 94°C, 30sec at 60°C, 1.5min at 68°C for 32 cycles) using TitaniumTaq DNA Polymerase (Clontech Laboratories, Palo Alto, CA), 200µM dNTP, 20ng adenoma cDNA and 0.2µM of the above mentioned primer. The products were ligated into pTrcHis2-TOPO expression vector (Invitrogen) as described in the manufacturers manual (1µl PCR product was incubated for 5 min at room temperature together with 1µl vector and 3µl H2O). The ligation reaction was transformed into E. coli TOP10 cells (Invitrogen). To verify the correct size and orientation of the insert single bacterial transformants were incubated in 100 µl LB amp (50 µg/ml) at 37°C for at least 4 hours. Inserts were PCR amplified (94°C for 60 sec, 55°C for 60 sec, 68°C for 1.5min for 30 cycles) using a vector specific forward primer (5'-GGCACTCGACCGGAATTATCG-3') and an ORF-specific reverse primer (see above). The products were analysed on agarose gel and positive clones containing inserts of the correct size and orientation were inoculated in 5ml LB-Amp and incubated overnight at 37°C at 225rpm. A 1:20 dilution of the culture was incubated for 1h at 37°C at 225rpm. Induction of protein translation with 6µl 1M IPTG (end conc. 1 mM) was performed when OD at 600nm of the bacterial culture was in the range of 0.4 to 0.6. After incubation at 37°C at 225rpm for 4h the bacteria were centrifuged and stored without supernatant at -80°C for subsequent protein analysis and purification.

NZW (New Zealand White) Rabbits are immunized with 200 mg recombinant CLA2 (ground immunization) in Complete Freunds Adjuvans and boosted 4 times with the same amount of protein in incomplete Freunds Adjuvans at intervals of 2 weeks.

Blood is taken one week after the 2nd boost and subjected to ELISA on the immobilized Antigen. One week after the final boost animals are subjected exsanguination. After coagulation the final blood is centrifuged at 4000 x g for 10 minutes. The supernatant from this step is centrifuged again at 16600 x g for 15min. The supernatant represents the raw CLA2 antiserum.

The antiserum is purified in 2 steps. Step 1 represents a conventional Protein A Chromatography. In step 2 the Ig fraction of step 1 is purified by Affinity chromatography on the Antigen immobilized onto Sepharose. The eluate of step 2 represents the purified Cla2 antiserum.

The purified antiserum is tested in several dilutions 1/1000 - 1/1000 000 on the immobilized antigen by peptide ELISA. Hereby specific antibodies are detected by anti Rabbit secondary reagents coupled to horseradish peroxidase (HRP) with a subsequent colorimetric reaction (e.g. TMB).

In a second approach the purified Antiserum is evaluated by Western Blot with immobilized Antigen subsequently to SDS-PAGE and Transfer onto Nitrocellulose.

In a last evaluation step the antiserum is tested on tissue arrays by Immuno-histochemistry (IHC). In Western Blot Analysis as well as with IHC bound antibodies are visualized with anti Rabbit secondary Reagents conjugated to HRP catalysing a colorimetric reaction. Due to the Sequence homology between CLA2.1 and CLA2.3, no antibody preparation distinguishing CLA2.3 from CLA2.1 could be generated. In order to obtain antibodies with selective specificities for these two variant polypeptide products encoded by the CLA2 gene monoclonal antibodies with defined epitopic specificity need to be generated.

An antibody directed against the sequence AQNAHWRKWMEPRGC might be especially valuable for this purpose, as this sequence is uniquely present in CLA2.3 and absent in CLA2.1. For certain purposes differentiation of the expression of CLA2.1 and CLA2.3 might be desirable.

### Example 6: Western Blot analysis of the novel polypeptides in samples of lung tumour

In order to evaluate, whether any one of the protein molecules encoded by the inventive CLA2 nucleic acids may be detected in tumour samples of patients with lung cancer, clinical samples with known diagnosis are subjected to an immuno-chemical analysis on the basis of the polyclonal antibodies generated according to Example 5.

As a negative control a cell line is used, that has been tested to be negative for reaction with the respective antibodies in a Western blot assay. As a positive control colon tumour cell line C4.1 cells are used. The positive and negative controls are subjected to the following procedures in the same way as the other samples. The material is analysed by Standard Western Analysis as follows.

In brief the clinical material is in a first step solubilsed by boiling (5min, 95°C) in Lämmli Protein Sample buffer (100 mM Tris pH.6.8, 2% SDS, 200mM DTT, 0.05% BpB) and insoluble components are spun down. In a second step supernatant protein samples are resolved on a SDS-PAGE (12% Acryl amide) and subsequently transferred to a nitrocellulose membrane by tank blotting (Towbin et al., 1979, Proc Natl Acad Sci;76:4350-4354). In a further step the membranes are blocked to prevent unspecific antibody binding (10% non fat dry milk in PBS) and subsequently incubated with the specific rabbit polyclonal antibodies directed against the CLA2.1, CLA2.2 or CLA2.3 polypeptides. The binding of the specific antibodies was visualized by Horseradish Peroxidase conjugated secondary reagents (binding to the marker specific antibody) catalysing photon emitting substrates.

The results show, that in samples of patients with diagnosed lung tumours all three different polypeptides may be detected in the Western blot analysis in different amounts. The predominant protein product detectable in tumour samples is CLA2.1. This shows, that the gene disclosed herein encodes at least three different protein products that may be used for the detection of colorectal carcinomas.

### Example 7: ELISA analysis of the inventive polypeptides in samples of gastric origin of patients and of normal control individuals

In order to evaluate presence of the CLA2 protein in samples of gastric cancer and matched normal tissue ELISA analysis of samples is performed.

In total tumour biopsy samples of each 100 patients and patient matched normal mucosa control samples are examined. As a negative control a negative cell line sample is used, that has been tested to be negative for reaction with the antibodies directed against the inventive polypeptides in a Western blot assay. As a positive control colorectal tumour cell line C4.1 cells are used. The positive and negative controls are subjected to the following procedures in the same way as the other samples.

The samples are analysed by ELISA as follows: Flat bottom 96 well plates (MaxiSorb; Nunc) are coated with affinity purified rabbit polyclonal IgG directed against each of the three CLA proteins (as used in Example 6) as a capture antibody over night at 4°C. Plates are washed 6x with PBS/0.1% Tween-20 and blocked with Superblock buffer (Pierce). Solubilised protein extract from the above described samples are dissolved in incubation buffer (PBS, 3% Superblock, 0.1% Tween20), and added in triplicates to each well. After 1 h incubation at RT, plates are washed 6x with PBS/0.1% Tween-20 and incubated with biotinylated detection antibody (polyclonal rabbit antibodies directed against CLA2 polypeptides) for 1 h at RT. Following 6x washes with PBS/0.1% Tween-20 TMB, 50 µl of Streptavidin-coated Alkaline Phosphatase (1:1000 dilution; Dianova) is added for 30 min. Thereafter, plates are washed 6x with PBS/0.1% Tween-20 and 100 µl of p-nitrophenyl phosphate substrate (PnPP; dissolved in diethanol amine buffer) are added to each well. OD 405 nm (620 nm reference wavelength) was measured with an ELISA reader (Tecan) after 30 min, 1h and 2 hrs.

Samples are identified as being positive for CLA2 proteins, when the measured OD 405 is at least twice that of the negative control sample. It can be seen, that no normal mucosa samples exhibit elevated levels of any of the three CLA2 proteins. In patient samples more than 90% show elevated levels of at least one of the CLA2 polypeptides. CLA2.1 shows the highest prevalence.

The results show, that in samples of normal gastric mucosa no CLA2 may be detected. In contrast in the tumour samples of gastric cancer CLA2 may be detected.

### Example 8: Qualitative analysis of stool samples of patients and of normal control individuals for the presence of colorectal cancer by the means of LC-MS analysis

In order to evaluate presence of the CLA2 proteins in stool samples LC-MS analysis of samples is performed.

In total stool samples of each 50 patients and 45 normal stool samples are examined in this experiment.

The stool samples have been dissolved in solubilisation buffer and proteins are precipitated using acetone. The protein precipitate pellet is re-suspended in 8M urea and cysteine residues within the proteins are reduced by dithiothreitol. Subsequently the reduced extracts are acetylated with iodoacetamid to prevent free sulfhydryl groups from oxidation. The modified protein extracts are digested with trypsin.

Analysis including 2D-HPLC and mass spectrometric identification is carried out on the ProteomeX-Workstation (ThermoFinnigan, San Jose, CA, USA). The system includes a HPLC system comprising two HPLC pumps and an auto sampler which are connected to independently provide solvent to a strong cation exchange column and a reversed-phase column.

In the first step the tryptic digest is loaded onto the strong ion exchange column and washed using a appropriate solvent to remove any contaminations not suitable for further analysis. By means of increasing salt steps beginning with 1mM ammonium chloride and ending at 900mM ammonium chloride fractions of the proteolytic peptides are eluted from the strong cation exchange column and loaded onto the reversed phase column. Using the second HPLC pump the peptides on the reversed phase column are eluted by an ascending acetonitril gradient from 5% to 80% acetonitril in water after washing the bound peptides to remove excess salt and conditioning the peptides for the subsequent mass spectrometric analysis.

The peptides eluting from the reversed phase column are measured on line by use of an electrospray ionisation ion trap mass spectrometer (DECA LCQ, ThermoFinnigan, San Jose, CA, USA) which enables direct analysis and fragmentation of eluted peptides. Each reversed-phase run is monitored continually by the ESI-MS and ESI-MS/MS spectra and saved for subsequent protein identification with the SEQUEST software package. SEQUEST uses peptide fragmentation mass spectra retrieved during the data dependent MS/MS process, which generated fragment mass spectra of eluted peptides. The SEQUEST algorithm links experimentally derived fragment spectra with *in silico* generated fragment spectra from databases and enables correlation of the experimentally derived spectrum to the appropriate database record which identifies the peptide matching to this record.

The results show, that CLA2 proteins may be detected in none of the samples obtained from normal control individuals, whereas in 43 of the 50 patient samples CLA2 proteins could be detected. Especially signals correlated to the peptide fragments ENMDEEK, QSSSTCR and HSPHTGF have been frequently identified in the respective mass spectra. Amongst others these fragments proved to be suited for mass spectrometric identification of CLA2 polypeptides. The peptides CLA2.4 - CLA2.8 proved to be detectable by mass spectrometry in a series of the tested samples.

The above described experimental data indicate, that CLA2 may be used as a marker for the detection of colorectal lesions from stool samples in a method as disclosed herein.

### Example 9: Detection of disseminated tumour cells in blood of individuals

Blood samples of patients with diagnosed adenocarcinomas of the colon were used to determine the level of mRNA of the inventive nucleic acids using quantitative RT PCR. In total samples 7 patients with colon-carcinomas were included. Three of the carcinomas were classified as carcinoma *in situ* and four as Dukes D.

Cells are isolated from the blood samples. Afterwards the cells are lysed. mRNA is isolated from the samples using Qiagen reagents (Qiagen, Hilden, Germany), and single-stranded cDNA is synthesized using Omniscript (Qiagen). PCR is performed using the ABI Prism 7700 Sequence Detector (PE Applied Biosystems) and the QuantiTect™ SybrGreen PCR mix (Qiagen), as described in the manufacturers manual.

PCR reactions are performed in 25 µl volumes with a final concentration of 100nM for each primer (5'primer: 5'-CCACAGGAGCTGAAGAGAGC-3'; 3'primer: 5'-AGGGCTTGTCTGGGTATGC-3'), with 95°C for 15 sec and 60°C for 70 sec for 45 cycles.

The specificity of the PCR products is verified by gel electrophoresis.

The experiment reveals, that no expression products of the CLA2 gene may be detected in blood samples of patients with carcinoma *in situ*. In contrast all patients exhibiting Dukes D classified carcinoma of the colon showed detectable levels of CLA2 mRNA in the blood samples.

The experiment illustrates, that CLA2 may be used for detection of disseminated tumour cells in body fluids of individuals.

### Example 10: Detection of disseminated tumour cells in axillary lymph nodes of individuals with diagnosed invasive breast carcinoma

Axillary lymph nodes of patients who underwent surgical ectomy of invasive carcinomas of the breast were used to determine the level of cells expressing the inventive CLA2 nucleic acids using quantitative RT PCR. In total samples of 20 patients were included. In 7 of the patients a spread of tumour cells to the lymph nodes was identified by conventional pathological methods.

Sections of the lymph nodes may be semi-quantitatively analysed for the mRNA level of CLA2 in an *in situ* staining reaction. The staining reaction is performed as follows:

For rehydration the tissue-sections are incubated in fresh 50% EtOH on a rocking device. Following the sections are rinsed in aqua bidest. The sections are incubated with proteinase K (10µg/ml in PBS) for 10 min at 37°C. Then the slides are transferred to washing buffer (PBS / 0.1% Tween20).

The hybridisation mixture is prepared by mixing 50 µl of ready to use hybridisation buffer (DAKO A/S, Glostrup, Denmark) with about 5 - 10 pmol of the probes. The probes are biotin-and digoxygenin-labelled oligonucleotides of sequences complementary to the respective mRNAs.

The hybridisation mixture is heated to 95°C and afterwards equilibrated to 37°C. After the boiling procedure the sections are incubated with each 50 µl of the hybridisation mixture for 4 hours at 42°C. The samples are washed in excess volumes of the wash buffers two times in 2 x SSC at 37°C for 15 min and once in 1 x SSC at 37 °C for 15 min Then the sections are rinsed two times at room temperature in 2 x SSC. Following this washing procedure the dissections are incubated for 30 min with blocking buffer (NEN, Blockingbuffer) at room temperature. Then follows 1 hour incubation with a 1:100 diluted (in Blocking buffer, see above) Streptavidin-alkaline phosphatase and with monoclonal mouse HRP-labelled anti-Digoxygenin antibodies (Molecular Probes). The sections are then washed 2 times in 1 x PBS/0,1% Triton X-100 for 10 min at room temperature, followed by one wash step with 1 x PBS, 50 mM MgCl2 (pH 9,2) for 10 min at room temperature. Then the staining reaction is performed with ELF 97 phosphate (Molecular Probes) for 10 sec to 7 min at room temperature. Excess substrate is washed 3 times with 1x PBS/0,1% Triton X-100 for 10 min at room temperature. In a second staining step, the section is incubated with Tyramide-Alexa-Fluor 594 for 10 sec to 7 min. Excess substrate is washed 3 times with 1x PBS/0,1% Triton X-100 for 10 min at room temperature. Finally the sections are dipped in H₂O_{dest.} and embedded with Fluorescence mounting medium (DakoCytomation). Then the stained sections can be analysed by fluorescence microscopy.

The microscopic examination of the slides reveals, that cells positive for expression of CLA2 may be found in samples of 13 patients. The staining is strong in the seven samples, which have been already identified as containing disseminated tumour cells by conventional methods. In further 6 samples sporadic staining of few cells may be identified microscopically. This indicates, that disseminated tumour cells, that have not been detected by conventional inspection may be made visible by the *in situ* staining method employing the inventive CLA2 as a molecular marker.

The results show, that the staining of cells with reagents specific for CLA2 enables for an improved more sensitive detection of spread of tumour cells to lymph nodes.

Simultaneously to the histological examination of the sections of the lymph node samples of the lymph node tissue were lysed for analysis by RT-PCR.

mRNA is isolated from the samples using Qiagen reagents (Qiagen, Hilden, Germany), and single-stranded cDNA is synthesized using Omniscript (Qiagen). PCR is performed using the ABI Prism 7700 Sequence Detector (PE Applied Biosystems) and the QuantiTect™ SybrGreen PCR mix (Qiagen), as described in the manufacturers manual.

PCR reactions are performed in 25 µl volumes with a final concentration of 100nM for each primer (5'primer: 5'-CCACAGGAGCTGAAGAGAGC-3'; 3'primer: 5'-AGGGCTTGTCTGGGTATGC-3'), with 95°C for 15 sec and 60°C for 70 sec for 45 cycles.

The specificity of the PCR products is verified by gel electrophoresis.

In the RT-PCR experiment 13 samples showed expression of CLA2. Theses were identical to the samples identified by *in situ* hybridisation.

The experiment reveals, expression of CLA2 mRNA may be used for detection of spread of tumour cells to lymph nodes and is suited to enhance the sensitivity of the conventional detection methods.

### Example 11: Early Diagnosis of ductal carcinoma in situ by detection of CLA2 mRNA in ductal lavage fluid

A collective of 14 individuals was included in this study. 7 patients were identified as having calcifications in breast ducts indicative of early stage ductal carcinoma *in situ* by mammographic examination. 7 individuals did not show any signs indicating a neoplastic lesion of the breast.

Ductal lavages were performed with all 14 individuals and cells were isolated from the lavage fluid.

Afterwards a fraction of the cells is lysed. mRNA is isolated from the samples using Qiagen reagents (Qiagen, Hilden, Germany), and single-stranded cDNA is synthesized using Omniscript (Qiagen). PCR is performed using the ABI Prism 7700 Sequence Detector (PE Applied Biosystems) and the QuantiTect™ SybrGreen PCR mix (Qiagen), as described in the manufacturers manual.

PCR reactions are performed in 25 µl volumes with a final concentration of 100nM for each primer (5'primer: 5'-CCACAGGAGCTGAAGAGAGC-3'; 3'primer: 5'-AGGGCTTGTCTGGGTATGC-3'), with 95°C for 15 sec and 60°C for 70 sec for 45 cycles.

The specificity of the PCR products is verified by gel electrophoresis.

The experiment reveals CLA2 expression in 9 individuals. All patients with a mammographic diagnosis indicating the presence of ductal carcinoma *in situ* could be identified as expressing CLA2 mRNA in the cells isolated from the ductal lavage. Two patients, who did not show any hint for the presence of early stages of breast neoplasias showed expression of CLA2 mRNA in this experiment.

A re-examination of the mammographs of these two patients subsequently to the result of the detection of the CLA2 expression revealed ambiguous patterns, that have not been identified as indication of the presence of a breast lesion without further information.

Subsequently fine needle biopsies of the regions of the breast identified by the re-examined mammographs were performed and analysed pathologically as well as by detection of the CLA2 mRNA level. The presence of early stage of carcinoma of the breast could be verified by this procedure.

The result shows, that the conventional methods for identification of early stages of neoplasias of the breast may be improved by methods based on the detection of the CLA2 expression products presented herein.

## Claims

1. An isolated CLA2 nucleic acid molecule being selected from a group consisting of
a) a nucleic acid molecule encoding a polypeptide that comprises an amino acid sequence as depicted in SEQ NO 3, 5, 7; 9,11,13,15,17;
b) a nucleic acid molecule comprising a nucleotide sequence as depicted in SEQ NO 1, 2, 4, 6; 8,10,12,14 or 16;
c) a nucleic acid molecule encoding a polypeptide the sequence of which shows at least 65% identity to the amino acid sequence of the polypeptide encoded by a nucleic acid molecule specified in (a) or (b);
d) a nucleic acid molecule the sequence of which differs from the sequence of a nucleic acid molecule of (a) to (c) due to the degeneracy of the genetic code;
e) a nucleic acid molecule, which represents a fragment or an allelic variant of a nucleic acid molecule of (a) to (d);
f) an mRNA transcribed from a nucleic acid of (a) - (e) or the corresponding cDNA;
g) a nucleic acid being a cDNA or mRNA arising from an alternative splicing event of the gene presented in SEQ NO 1, 2, 4, 6, 8, 10, 12, 14 or 16;
h) a nucleic acid, which encodes a fragment or a variant of the amino acid sequence depicted in SEQ NO 3, 5, 7,9,11, 13,15 or 17;

2. A recombinant vector containing one or more nucleic acid molecules of Claim 1.

3. The recombinant vector of Claim 2, wherein the nucleic acid molecule is operatively linked to regulatory elements allowing transcription and synthesis of a translatable RNA in prokaryotic and/or eucaryotic host cells.

4. A recombinant host cell which contains the recombinant vector of Claim 2 or 3.

5. The recombinant host cell of Claim 4, which is a mammalian cell, a bacterial cell, an insect cell, a plant cell or a yeast cell.

6. An isolated CLA2 polypeptide molecule being selected from a group consisting of
a) a polypeptide, which is encoded by a nucleic acid molecule of Claim 1;
b) a polypeptide, which comprises an amino acid sequence given in SEQ NO 3, 5, 7, 9, 11, 13,15 or 17;
c) a polypeptide, that is recognized by a binding agent, that has been raised against and is specifically binding the polypeptide of (a) or (b) or against a sequence as given in SEQ NO 18-29;
d) a fragment or a variant of the polypeptides of (a) to (c), that is encoded by a nucleic acid sequence, that hybridises to a nucleic acid according to Claim 1 under stringent conditions; and
e) a fragment or variant of the polypeptides of (a) to (d), which has an increased or decreased biological activity compared to the wild type CLA2 polypeptides.

7. A method of making a polypeptide exhibiting a biological property of the inventive CLA2 polypeptide comprising:
a) culturing the recombinant host cell of Claim 4 or 5 under conditions such that said polypeptide is expressed; and
b) recovering said CLA2 polypeptide.

8. A method for the production of the inventive CLA2 polypeptide in a cell free *in vitro* transcription and/or translation system.

9. A polypeptide produced by the method of Claim 7 or 8.

10. A fusion polypeptide or chimeric nucleic acid comprising at least one CLA2 polypeptide or CLA2 nucleic acid or fragments thereof according to Claims 1 or 2.

11. A nucleic acid being DNA or RNA that is reverse complementary or complementary to a CLA2 nucleic acid of Claim 1 or a fragment thereof.

12. A binding agent specifically recognizing and binding to a polypeptide of Claim 6 or 9 or a binding agent specifically recognizing a sequence as depicted in SEQ NO 18 - 29 being selected from a group consisting of
a) an antibody;
b) an immunogen binding fragment of an antibody;
c) a peptidomimetic compound comprising an immunogen binding epitope; and
d) an oligopeptide capable of specifically binding to antigens.

13. The nucleic acid according to Claim 1, the polypeptide according to Claim 6, 9 or 10 or the binding agent according to Claim 12, which is detectably labelled.

14. The nucleic acid, polypeptide or binding agents according to Claim 13, wherein the label is selected from the group consisting of a radioisotope, a bioluminescent compound, a chemiluminescent compound, a fluorescent compound, a metal chelate, a biologically relevant binding structure such as biotin or digoxygenin or an enzyme.

15. The native or chemically pre-treated genomic nucleic acid sequence of a CLA2 gene given in SEQ NO 1 for use in the detection and therapy of proliferative disorders.

16. A transgenic non-human animal comprising at least one CLA2 nucleic acid of Claim 1 or the recombinant vector of Claim 2 or 3.

17. The transgenic non-human animal of Claim 16 further comprising at least one inactivated wild type allele of the corresponding CLA2 polypeptide encoding gene.

18. The transgenic non-human animal of Claim 16 or 17 which is a mouse or rat.

19. A method for identifying a binding partner to a CLA2 polypeptide of Claim 6, 9 or 10 comprising:
a) contacting a polypeptide of Claim 6, 9 or 10 with a compound to be screened; and
b) determining whether the compound effects an activity of said polypeptide or whether binding of the compound to said polypeptide has occurred.

20. A method for identifying activators/agonists or inhibitors/antagonists of the CLA2 polypeptide comprising the steps of:
a) incubating a candidate compound with a polypeptide of Claim 6, 9 or 10;
b) assaying a biological activity, and
c) determining if a biological activity of said polypeptide has been altered.

21. A method for identifying activators or inhibitors of the expression of the CLA2 polypeptide comprising the steps of:
a) incubating a candidate compound with an *in vivo* or *in vitro* test system for protein expression or administering a compound to a test organism,
b) detecting the level of the polypeptide of Claim 6 or 9 within the test system or within the organism, and
c) determining if the level of said polypeptide has been altered.

22. A method of identifying and obtaining a drug candidate for therapy of cell proliferative disorders comprising the steps of
a) contacting the polypeptide of Claim 6 or 9 or a cell expressing said polypeptide in the presence of components capable of providing a detectable signal in response to protein degradation, cell proliferation or cell differentiation with said drug candidate to be screened under conditions to allow protein degradation, cell proliferation or changes in cell differentiation and
b) detecting presence or absence of a signal or increase of the signal generated from protein degradation, cell proliferation or cell differentiation, wherein the presence or increase of the signal is indicative for a putative drug.

23. The method of Claim 22 wherein said cell is comprised in the transgenic non-human animal of one of the Claims 17 or 18.

24. An activator/agonist or inhibitor/antagonist of the polypeptide of Claim 6 or 9, an activator or inhibitor of the expression of the polypeptide of Claims 6 or 9 or a binding partner of the polypeptide of Claim 6 or 8 obtainable by one of the methods of Claims 19 to 21.

25. Method for diagnosis of disorders associated with abnormal cell proliferation and/or prognosis of disease course comprising
a) obtaining a sample from an individual
b) determining the levels of one or more CLA2 marker molecules being nucleic acid molecules or polypeptides according to Claim 1 or 6;
c) comparing the levels of said CLA2 nucleic acids or CLA2 polypeptides within said sample to the contents within a corresponding control sample, not affected by the disease being tested;
d) wherein the diagnosis or prognosis of disease course is predicted from considering a significant change relative to the wild type level of at least one single CLA2 marker molecule or of a set of CLA2 marker molecules as indicative of said disorder or of the prognosis of the disease course.

26. The method of Claim 25, wherein the sample is selected from a group comprising a liquid containing nucleic acids, polypeptides or fragments thereof, a liquid containing cells or cell debris, a body fluid, a tissue sample and a cell sample.

27. The method of Claim 25 or Claim 26, wherein the sample is blood, plasma, serum, liquor, lymph, bone marrow, swabs, washes, lavages, secretions, transsudates, exsudates, sputum, stool, urine, semen, cell- and tissue-samples, punctuates or biopsies.

28. The method according to any one of the Claims 25 - 27, wherein the disease is a cell proliferative disorder, cancer or a precursory lesion of cancer.

29. The method according to Claim 28, wherein the cancer is cancer of the head and the neck, cancer of the respiratory tract, cancer of the gastrointestinal tract, cancer of the skin and its appendages, cancer of the central and peripheral nervous system, cancer of the urinary system, cancer of the reproductive system, cancer of the endocrine system, cancer of the soft tissues and bone, cancer of the lymphopoietic and hematopoietic system, breast cancer, lung cancer, cervical cancer, colorectal cancer or anogenital cancer.

30. A method according to any one of the Claims 25 - 29, wherein the detection of the expression of the CLA2 molecules is carried out using at least one probe specifically binding to the marker molecules to be detected.

31. A method according to Claim 30, wherein the probe is detectably labelled.

32. The method according to Claim 31, wherein the label is selected from the group consisting of a radioisotope, a bioluminescent compound, a chemiluminescent compound, a fluorescent compound, a metal chelate, a biologically relevant binding structure such as biotin or digoxygenin or an enzyme.

33. The method according to Claims 30 - 32, wherein at least one probe is an antibody, a fragment of an antibody, a peptidomimetic comprising an antigen binding epitope or a mini-antibody.

34. The method according to Claim 33, wherein the detection comprises an immuno-cytochemical detection procedure.

35. The method according to Claims 30 - 32, wherein at least one probe being a nucleic acid hybridising to a marker nucleic acid is used for the detection of the CLA2 marker molecules.

36. The method according to Claim 35, wherein the detection reaction comprises a nucleic acid amplification reaction.

37. The method according to Claims 35 - 36, which is used for *in situ* detection.

38. A method according to any one of the Claims 25 - 37, which is used in the course of an *in vivo* or *in vitro* molecular imaging method.

39. A method according to any one of the Claims 24 - 37, which is carried out in the course of early diagnosis of disorders, of minimal residual disease diagnosis or of screening tests.

40. A test-kit for carrying out the method according to any one of the Claims 24 - 38, comprising at least
a) reagents for the detection of CLA2 nucleic acids and/or CLA2 polypeptides;
b) the reagents and buffers commonly used for carrying out the detection reaction, such as buffers, detection-markers, carrier substances and others,
which is a research kit, a diagnostic kit or a point of care test kit.

41. A test-kit according to Claim 39, wherein the reagent for detection of the CLA2 nucleic acids and/or CLA2 polypeptides is an agent specifically binding to said nucleic acids and/or polypeptides.

42. Use of one or more CLA2 compounds according to any one of the Claims 1 - 15 and/or an activator/agonist or inhibitor/antagonist of the CLA2 polypeptide according to Claim 20, an activator or inhibitor of the expression of the CLA2 polypeptide according to Claim 21, a binding partner of the CLA2 polypeptide according to Claim 19 or a drug candidate identifiable by the method according to Claim 22 or 23 for production of a medicament useful for treating disorders associated with the expression of the inventive CLA2 polypeptide.

43. The use according to Claim 42, wherein the disorder is a benign and malignant tumour, a carcinoma or a dysplasia.

44. The use according to Claim 43, wherein the tumour is cancer of the head and the neck, cancer of the respiratory tract, cancer of the gastrointestinal tract, cancer of the skin and its appendages, cancer of the central and peripheral nervous system, cancer of the urinary system, cancer of the reproductive system, cancer of the endocrine system, cancer of the soft tissues and bone, cancer of the lymphopoietic and hematopoietic system, breast cancer, anogenital cancer or colorectal cancer.

45. A use according to any one of the Claims 42 - 44, wherein the method for treatment is immuno-therapy, vaccination therapy or gene therapy.

46. A pharmaceutical composition comprising at least one CLA2 compound according to Claims 1 - 15 and/or an activator/agonist or inhibitor/antagonist of the CLA2 polypeptide according to Claim 20, an activator or inhibitor of the expression of the CLA2 polypeptide according to Claim 21, a binding partner of the CLA2 polypeptide according to Claim 19 or a drug candidate identifiable by the method according to Claim 22 or 23 for use in treatment of disorders **characterized by** abnormal cell proliferation.
